# EUROPEAN PATENT SPECIFICATION

(11) **EP 4 149 555 B1**
(45) Date of publication and mention of the grant of the patent: **23.09.2026**
(21) Application number: 21727202.0
(22) Date of filing: 14.05.2021
(51) Int. Cl.: A61K 47/54, A61K 35/28, A61P 31/12, C07K 7/64, C12N 5/0789, A61K 47/55, A61K 38/13

(54) **CYCLOSPORINE ANALOGUES**
CYCLOSPORIN-ANALOGA
ANALOGUES DE LA CYCLOSPORINE

(30) Priority: 14.05.2020 GB 202007106
(43) Date of publication of application: 22.03.2023
(73) Proprietor: UCL Ventures Limited, London WC1E 6BT (GB)
(72) Inventor: TOWERS, Greg, London, Greater London W1T 4TP (GB); SELWOOD, David, London, Greater London W1T 4TP (GB); THORNE, Lucy, London, Greater London W1T 4TP (GB); WARNE, Justin, London, Greater London W1T 4TP (GB)
(74) Representative: J A Kemp LLP
(86) International application number: PCT/GB2021/051161
(87) International publication number: WO 2021/229237

(56) References cited:
- WO-A1-2011/144756
- WO-A1-2016/073480
- WO-A1-2017/059207
- WO-A1-2023/247937
- WO-A2-2007/112345
- WO-A2-2014/145686
- US-A1- 2011 206 637
- US-A1- 2018 296 588
- DATABASE CAPLUS [online] 1 January 2014 (2014-01-01), S T GLOBAL: "Preparation of novel cyclosporin derivatives for the treatment and prevention of especially a viral infection - WO 2014145686 A2", XP093267930, Database accession no. 2014:1558703
- VERDINE GREGORY L ;: "Methods and reagents for analyzing protein-protein interfaces", WO2017059207 A1, 1 January 2017 (2017-01-01), pages 1 - 4, XP055824973
- OR Y S: "Preparation of cyclosporin analogs as antiviral agents", US2011206637 A1, 1 January 2011 (2011-01-01), pages 1 - 18, XP055824974

## Description

### FIELD OF THE INVENTION

The present invention relates to novel cyclosporine analogues and their use in medical applications, including as antiviral compounds and in gene therapy.

### BACKGROUND TO THE INVENTION

Haematopoietic Stem Cell (HSC) gene therapy can now treat genetic haematopoietic diseases including immunodeficiencies and metabolic disorders that are often otherwise fatal and have limited long-term drug-based therapeutic options. Gene therapy requires HSC isolation and delivery of a functional copy of the disease gene *ex vivo.* Modified HSC are returned to the patient to replenish the haematopoietic system for long-term therapy.

HSC gene delivery requires vectors based on HIV. A major hurdle is HSC resistance to vector infection. A key protective antiviral protein in HSC, which blocks vector entry and gene delivery, is a known antiviral protein called IFITM3 (see, for instance, Petrillo et al. Cell StemCell 23, 820-832, 2018). As described, for instance, in Petrillo *et al.* (*supra*) and WO 2015/162594, the naturally-occurring cyclosporines CsA and CsH have previously been shown to act as transduction enhancers (TE) by inhibiting IFITM3 to enhance vector infection and gene delivery in these cells.

Unfortunately, CsH has limited availability, high cost and poor purity. Conversely, CsA has undesirable features that reduce its efficacy, particularly its inhibition of the well-characterized HIV cofactor cyclophilin A (CypA). It would be desirable to provide alternative compounds capable of enhancing transduction that do not suffer from these limitations. Particularly desirable would be the provision of easy-to-synthesise, highly potent and selective IFITM3 inhibitors to enhance HIV-vector infection, reduce vector dose required and overcome patient variability.

Meanwhile, the effective treatment of viral infections remains a significant challenge for healthcare systems throughout the world. For instance, there currently exists no known effective treatment for a range of recently emerging coronaviruses, including coronavirus diseases 2019 ("COVID-19"). Cyclosporine compounds have previously been proposed as being potentially useful in the therapy of such conditions (see, for instance, de Wilde et al., J Gen Virol. 2011; 92(Pt 11): 2542-2548). There remains a pressing demand for efficacious antiviral therapies, with a particularly acute current instance being the need for treatments for COVID-19.

WO 2011/144756 A1 is directed to biosynthetic proline/alanine random coil polypeptides.

WO 2017/059207 A1 relates to methods and reagents said to be useful for analyzing protein-protein interfaces such as interfaces between a presenter protein (e.g., a member of the FKBP family, a member of the cyclophilin family, or PIN1) and a target protein.

US 2018/296588 A1 relates to a HBV disease or HIV disease combination therapy involving administering a particular cyclosporine analogue and an NRTT or NtRTT.

WO 2007/112345 A2 relates to particular cyclosporine alkene analogues and their possible use in treating viral-induced disorders.

WO 2016/073480 A1 relates to particular cyclosporine analogues and their possible use in treating HCV.

US 2011/206637 A1 relates to particular cyclosporine analogues and their possible use in treating viral infections.

WO 2014/145686 A2 relates to particular cyclosporine analogues and their possible use in treating various disorders.

### SUMMARY OF THE INVENTION

It has now been found that certain cyclosporine analogues are effective as antiviral compounds, including but not limited to efficacy in treating COVID-19. It has also been found that the cyclosporine analogues are effective as transduction enhancers in HSC gene delivery. The cyclosporine analogues are synthetic analogues of natural cyclosporine compounds, featuring a chemically modified side chain.

Specifically, the present invention provides a cyclosporine analogue that is a compound of formula (III) or a pharmaceutical salt thereof 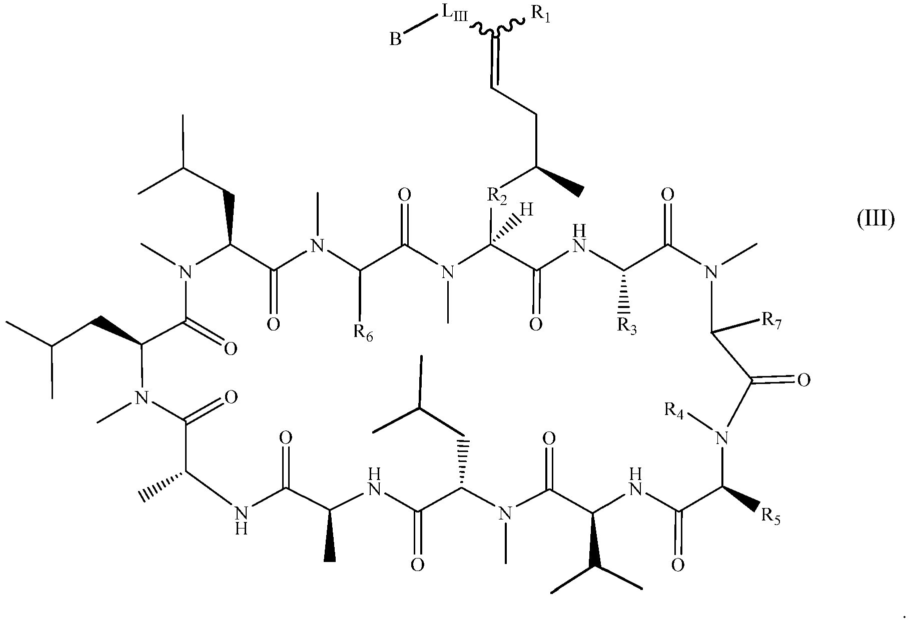 , wherein:
L_{III} is a direct bond or a linker moiety;
B is 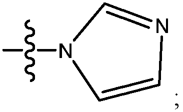
R₁ represents hydrogen;
R₂ represents 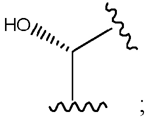
R₃ represents ethyl;
R₄ represents methyl;
R₅ represents -CH₂CH(CH₃)₂;
R₆ represents 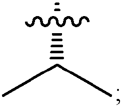
R₇ represents a hydrogen atom; and
the linker moiety is a C₁₋₂₀ alkylene group, a C₂₋₂₀ alkenylene group or a C₂₋₂₀ alkynylene group, which is unsubstituted or substituted by one or more substituents selected from halogen atoms and sulfonic acid groups, and in which
   (a) 0, 1, 2 or 3 carbon atoms are replaced by groups selected from C₆₋₁₀ arylene, 5- to 10-membered heteroarylene, C₃₋₇ carbocyclylene and 5- to 10-membered heterocyclylene groups, and
   (b) up to half of the -CH₂- groups are replaced by groups selected from -O-, -S-, -C(O)- and -N(C₁₋₆ alkyl)- groups, wherein:
      (i) said arylene, heteroarylene, carbocyclylene and heterocyclylene groups are unsubstituted or substituted by one or more substituents selected from: halogen atoms; and C₁₋₆ alkyl; C₁₋₆ haloalkyl; C₁₋₆ alkoxy; -(C₁₋₆ alkyl)ₙC(O)O(C₁₋₆ alkyl) where n=0 or 1; -(C₁₋₆ alkyl)ₙOC(O)(C₁₋₆ alkyl) where n=0 or 1; C₁₋₆ alkylthiol, - N(R_{N})₂ wherein each R_{N} independently represents a hydrogen atom or a C₁₋₆ alkyl group; -CN; -S(O)₂NH₂; nitro; and sulfonic acid groups; and
      (ii) 0, 1 or 2 carbon atoms in said carbocyclylene and heterocyclylene groups are replaced by -C(O)- groups.

The present invention still further provides ex vivo use of the cyclosporine analogue of the present invention, for increasing the efficiency of transduction of an isolated population of human haematopoietic stem and/or progenitor cells by a vector derived from HIV-1, HIV-2, SIV, FIV, BIV, EIAV, CAEV or visna lentivirus.

The present invention also provides a method of transducing a population of human haematopoietic stem and/or progenitor cells comprising the steps of: a) contacting the population of cells with the cyclosporine analogue of the present invention; and b) transducing the population of cells with a vector derived from HIV-1, HIV- 2, FIV, BIV, EIAV, CAEV or visna lentivirus; wherein steps (a) and (b) are carried out ex vivo.

The present invention further provides a population of human haematopoietic stem and/or progenitor cells prepared according to the method of transducing of the present invention, as well as a pharmaceutical composition comprising such a population of haematopoietic stem and/or progenitor cells. The invention further provides this population of haematopoietic stem and/or progenitor cells for use in therapy.

The present invention still further provides the cyclosporine analogue according to the present invention, for use in haematopoietic stem and/or progenitor cell gene therapy.

The present invention also provides the cyclosporine analogue according to the present invention, for use in treatment of a viral infection, preferably wherein the viral infection is human immunodeficiency virus-1 (HIV-1), influenza virus, human cytomegalovirus (hCMV), hepatitis C virus (HCV), dengue virus, a vaccinia virus, feline immunodeficiency virus (FIV) or a corona virus.

For clarity, any reference here to a therapeutic method involving administering a particular substance or composition is to be interpreted as being directed to that substance or composition for use in that method.

### BRIEF DESCRIPTION OF THE DRAWINGS

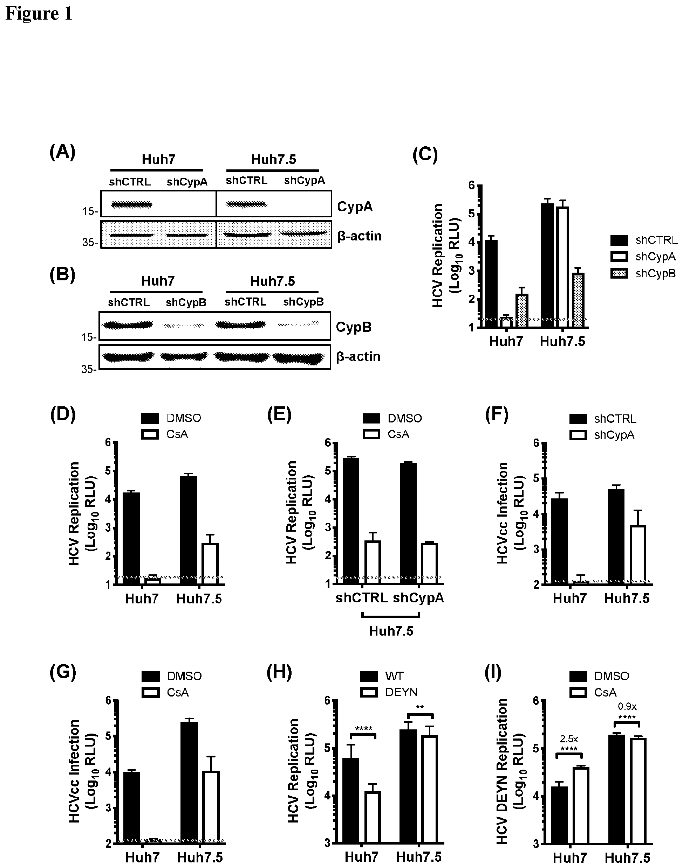

**Figure 1****: CypA is critical for HCV replication in Huh7 cells, but not in Huh7.5 cells. (A)** Western blot detecting CypA **(A)** or CypB **(B)** expression in Huh7 and Huh7.5 cells transduced with Cyp specific shRNA-expressing lentiviruses as shown. Actin was detected as a loading control. **(C)** Evaluation of HCV replicon replication in CypA- and CypB-silenced cells. Luciferase reporter activity was measured at 4 and 48 hours post-electroporation (hpe) and is expressed as relative luciferase units (RLU) at 48 hpe. **(D-E)** Huh7 or Huh7.5 cells (silenced or not for CypA expression) were electroporated with replicon RNA and treated with 1 µM CsA at 4 hours post electroporation (hpe). Luciferase reporter activity was measured at 48 hours post-electroporation. **(F)** HCVcc infection in CypA-silenced Huh7 and Huh7.5 cells. Cells were infected with HCVcc (J6/JFH1-RLuc) and infection was assessed after 72 hours by measuring luciferase activity. **(G)** Huh7 or Huh7.5 cells were infected with HCVcc and treated with 1 µM CsA at 4 hours post-infection (hpi). After 72 hours, infection was measured by luciferase activity. **(H)** Replication of HCV NS5A wild-type (WT) and HCV CsA resistance mutant (NS5A D316E/Y317N; DEYN) in Huh7 and Huh7.5 cells. Cells were electroporated with *in vitro* transcribed replicon RNA as described above, and replication was assessed by luciferase activity at 48 hpe. **(I)** Huh7 or Huh7.5 cells were electroporated with HCV NS5A DEYN replicon RNA. After 4 hours, cells were treated with 1 µM CsA and replication was assessed by luciferase activity at 48 hpe. All graphs show relative luciferase units (RLU) expressed as means ± standard deviation from at least 3 independent experiments each performed in triplicate. Statistical significance was evaluated by t-test using GraphPad Prism (**** *p*-value < 0.0001; ** *p*-value < 0.01).

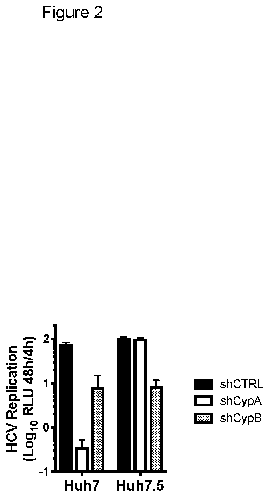

**Figure 2****: CypA depletion abrogates HCV replication in Huh7, but not Huh7.5 cells.** The 48-h values from Figure 1C were normalised to the 4-h RLU to compensate for potential differences in electroporation efficiency between cell lines, and are expressed as fold increase compared to 4-h RLU.

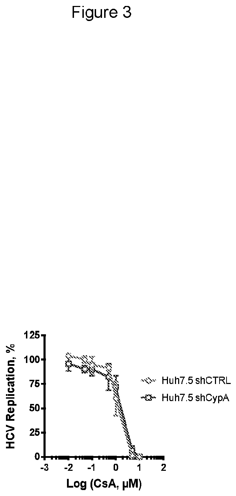

**Figure 3****: CsA is equally potent against HCV replication in CypA-depleted Huh7.5 cells.** Huh7.5 cells (silenced or not for CypA expression) were electroporated with replicon RNA and treated with serially diluted CsA at 4 hours post electroporation (hpe). Luciferase reporter activity was measured at 48 hours post-electroporation. HCV replication is expressed as a percentage relative to the DMSO control.

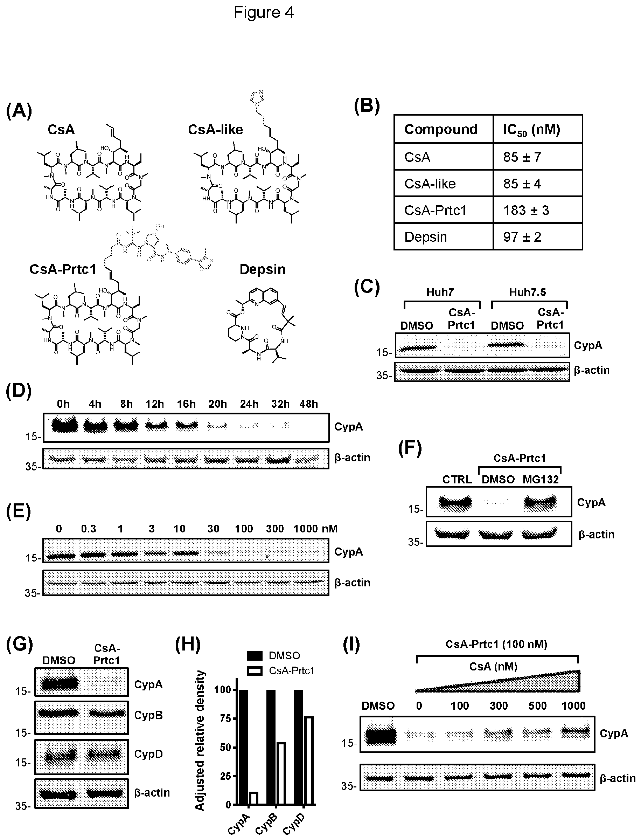

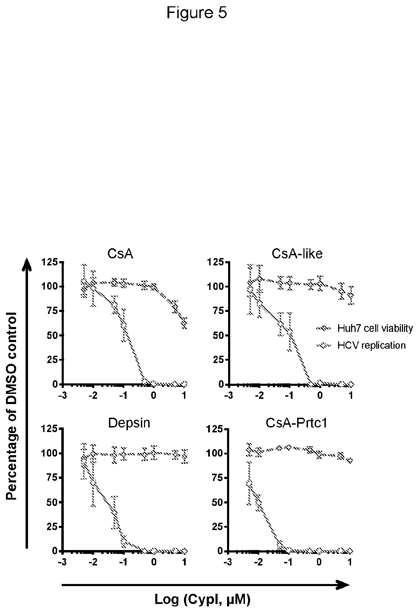

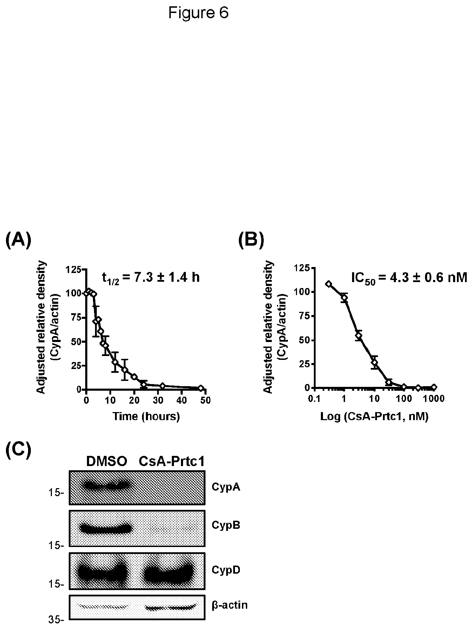

**Figure 4****. Structures and properties of distinct novel CypI. (A)** Structures of CypI used in this study; their effects on viral replication and cell viability are shown in **Figure 5****. (B)** CypI-CypA binding affinity measured by fluorescence polarisation using a fluorescein labelled CsA probe. **(C)** Western blot showing CsA-Prtc1-mediated degradation of CypA in Huh7 and Huh7.5 cells after 48h treatment with 1 µM CsA-Prtc1. **(D)** Analysis of CypA degradation at time points shown after 1 µM CsA-Prtc1 treatment in Huh7 cells detecting CypA expression by Western blot. **(E)** Dose-response of CsA-Prtc1-mediated CypA degradation in Huh7 cells. Cells were treated with the indicated concentrations of CsA-Prtc1 for 48 h, and CypA levels detected by Western blot. **(F)** CsA-Prtc1-mediated degradation of CypA is proteasome-dependent. Cells were treated with CsA-Prtc1 (1 µM) with or without the proteasome inhibitor MG132 (10 µM) for 24 hours. CypA was detected by Western blot. **(G)** CsA-Prtc1 specificity for CypA. Huh7 cells were treated with 1 µM CsA-Prtc1. for 24 h and CypA, CypB or CypD detected by Western blot. **(H)** Quantitation by densitometry of gel in **(G)** showing adjusted relative density normalised to the actin loading control. **(I)** CsA treatment rescues CypA from CsA-Prtc1-mediated degradation. Huh7 cells, treated for 24 h with CsA-Prtc1 (100 nM), in the presence of increasing concentrations of CsA, were lysed and CypA expression detected by Western blot. **(C-I)** One representative Western blot is shown from at least two independent experiments; **(D-E)** quantitation by densitometry analysis (showing the combined data from the independent experiments) is shown in Figure 6A-B.
**Figure 5****: Novel CypI inhibit HCV replication and are not cytotoxic.** Approximately 2 x 10⁶ Huh7 cells were electroporated with 5 µg HCV replicon RNA. CypI were added at 4 hpe. Replication (solid line) was measured by luciferase activity at 48 hpe and is expressed as percentage relative to DMSO treated control. Cell viability (dashed line) was measured by Alamar Blue assay. Graphs show means ± standard deviation from three independent experiments each performed in triplicate.
**Figure 6****: Characterisation of CsA-Prtc1 activity. (A-B)** Quantitation by densitometry analysis showing adjusted relative density normalised to the actin loading control for the time-course and dose-response shown in **Fig.4D-E.** Huh7 cells were treated with CsA-Prtc1 at 1 µM **(A)** or the indicated concentrations **(B).** Lysates were collected at the indicated time points **(A)** or after 48 h of treatment **(B)** and CypA expression was evaluated by Western blot. One representative blot for each is shown in **Fig. 4D-E. (C)** Huh7 cells were treated with CsA-Prtc1 (1 µM). Lysates were collected 48 h later and evaluated for CypA, CypB and CypD expression by Western blot.

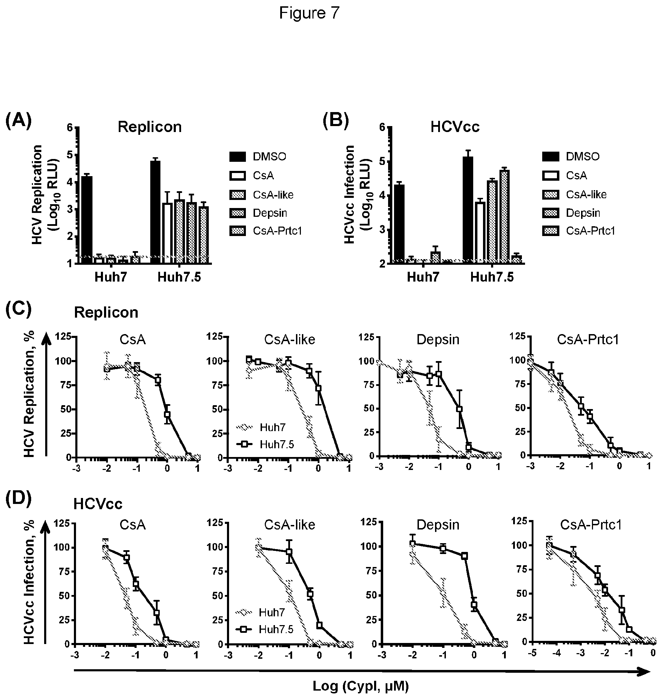

**Figure 7****: CypI are more potent against HCV replication and infection in Huh7 cells than in Huh7.5 cells. (A)** CypI more potently inhibit HCV replication in Huh7 cells than in Huh7.5 cells. Replicon RNA electroporated Huh7 or Huh7.5 cells were treated with 1 µM CypI at 4 hpe and replication was measured by luciferase activity after 48 h. **(B)** CypI more potently inhibit HCVcc infection in Huh7 cells than in Huh7.5 cells. Cells infected with HCVcc were treated with 1 µM CypI at 4 hpi and replication was measured by luciferase activity after 72 h. **(C-D)** Dose-response analyses comparing antiviral activity of CypI in Huh7 and Huh7.5 cells. Cells were electroporated with HCV replicon RNA **(C)** or infected with HCVcc **(D)** and treated with increasing concentrations of CypI 4 later. Replication or infection was measured by luciferase activity after 48 h **(C)** or 72 h **(D),** and is expressed as a percentage relative to the DMSO vehicle-treated control. All graphs show means ± standard deviation from at least three independent experiments each performed in triplicate. In **(A)** and **(B),** bars from left to right are for DMSO, CsA, CsA-like, Depsin and CsA-Prtc1, respectively.

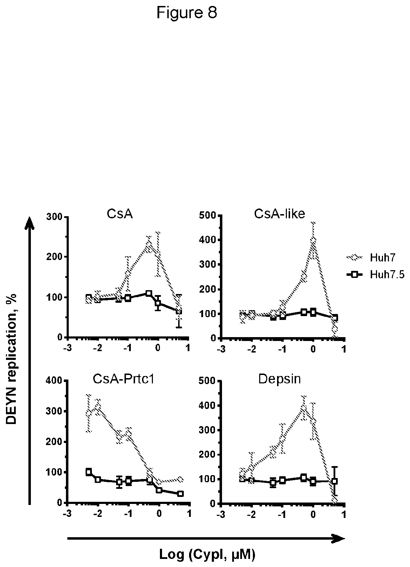

**Figure 8****: DEYN replication is enhanced by CypI treatment in Huh7 cells.** Approximately 2 x 10⁶ Huh7 cells were electroporated with 5 ng HCV NS5A DEYN replicon RNA. CypI were added at 4 hpe. Replication was measured by luciferase activity at 48 hpe and is expressed as percentage relative to DMSO treated control. Graphs show means ± standard deviation from two independent experiments each performed in triplicate.

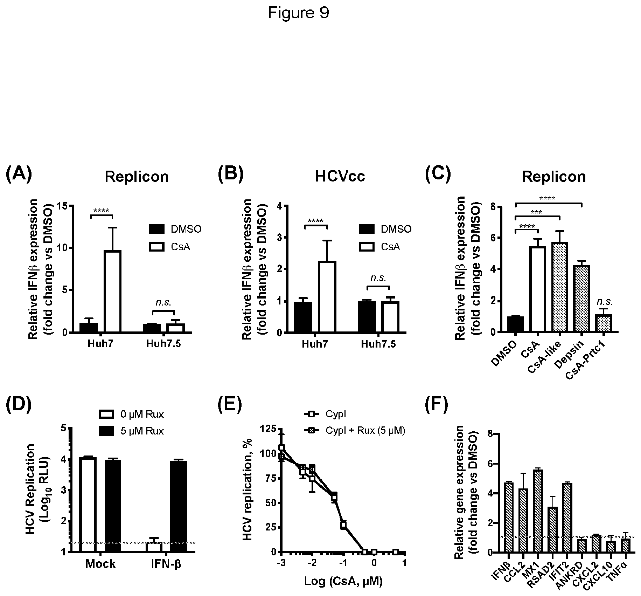

**Figure 9****: CypI induce expression of IFN-β and antiviral genes in Huh7, but not Huh7.5, cells. (A-C)** Cells electroporated with HCV replicon RNA **(A, C)** or infected with HCVcc **(B)** were treated with 5 µM CsA **(A, B)** or CypI **(C)** 4 h later. After 48 h, RNA was extracted and expression of *IFN-β* mRNA was evaluated by qRT-PCR. Data were normalised by GAPDH expression and are expressed as fold change compared to the DMSO vehicle-treated control. **(D-E)** CypI potency does not depend on IFN signalling. HCV replication in Huh7 cells, electroporated as described above, and treated with IFN-β (5 ng/mL) or CypI, in the presence or absence of the Jak/STAT inhibitor ruxolitinib (Rux). Rux treatment rescued HCV replication from IFN-β inhibition **(D)** but not from CypI **(E). (F)** CsA treatment induces expression of a subset of antiviral genes in HCV-replicating Huh7 cells. RNA expression of *IFN-β, CCL2, MX1, RSAD2 IFIT2, ANKRD, CXCL2, CXCL10* and *TNFα* mRNA was evaluated by qRT-PCR at 48 hpe in Huh7 cells electroporated with HCV replicon RNA and treated with CsA (5 µM) at 4 hpe. Data were normalised by GAPDH expression and are expressed as fold change compared to the DMSO vehicle-treated control. All graphs show means ± standard deviation from at least three independent experiments each performed in triplicate. Statistical significance was evaluated by t-test using GraphPad Prism (**** *p*-value < 0.0001; *** *p*-value < 0.001). In **(C),** bars from left to right are for DMSO, CsA, CsA-like, Depsin and CsA-Prtc1, respectively.

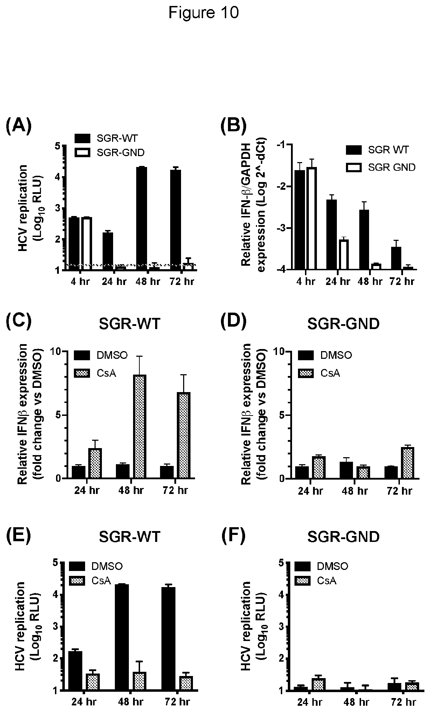

**Figure 10****: Induction of IFN-β expression by CsA depends on HCV replication.** Approximately 2 x 10⁶ Huh7 cells were electroporated with 5 µg wild-type HCV replicon RNA (SGR-WT) or polymerase-defective HCV replicon RNA (SGR-GND). **(A)** Replication was measured by luciferase activity at 4, 24, 48 and 72 hpe and is expressed as RLU. **(B)** RNA was extracted at the indicated time points and expression of IFN-β mRNA was evaluated by qRT-PCR. Data was normalized by GAPDH expression and is expressed on a log scale as 2^-dCt. **(C-F)** Huh7 cells were electroporated with wild-type **(C, E)** or polymerase-defective **(D, F)** HCV replicon RNA as described above. At 4 hpe, 5 µM CsA or DMSO vehicle was added. HCV replication was measured by luciferase reporter activity at the indiciated time points **(E-F),** while RNA was extracted from parallel samples and expression of IFN-β mRNA was evaluated by qRT-PCR **(C-D).** Data was normalised by GAPDH expression and is expressed as fold change compared to the DMSO vehicle-treated control. Graphs show means ± standard error from two independent experiments each done in quadruplicate.

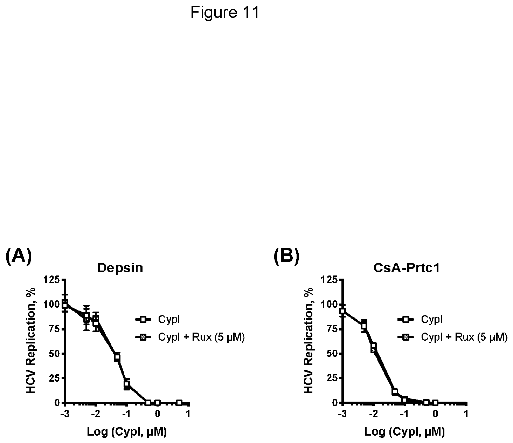

**Figure 11****: Inhibition of IFN-β signaling by ruxolitinib does not affect CypI potency.** Dose-response curves of CypI (A-B) in HCV-replicating cells treated with Rux (as in Figure 4D-E). Graphs show means ± standard deviation from at least three independent experiments each performed in triplicate.

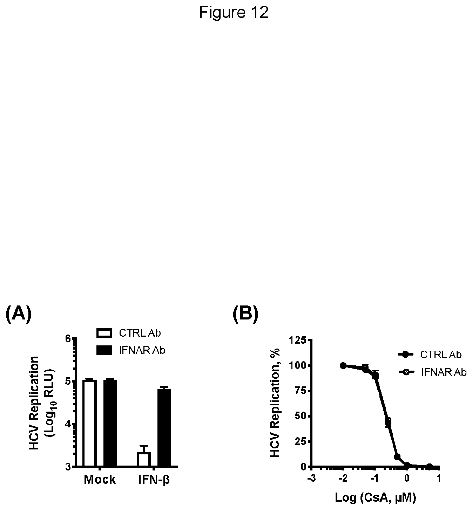

**Figure 12****: Inhibition of IFN-β signaling by IFNAR antibody does not affect CsA potency. (A)** Huh7 cells were treated with 0.025 ng/mL of IFN-beta, in the presence of CTRL antibody or IFNAR antibody (2 ug/mL). **(B)** CsA antiviral activity against HCV in cells treated with CTRL antibody or IFNAR antibody (2 ug/mL). HCV replication is expressed as a percentage relative to the DMSO control. Graphs show two independent experiments.

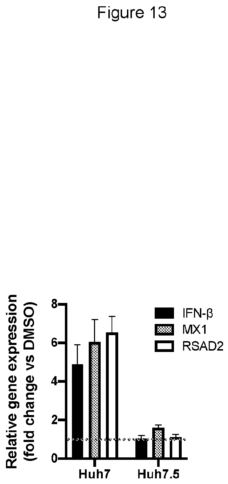

**Figure 13****: CsA induces expression of antiviral genes in Huh7, but not Huh7.5, cells.** Approximately 2 x 10⁶ Huh7 or Huh7.5 cells were electroporated with 5 µg HCV replicon RNA. At 4 hpe, DMSO vehicle or CsA (5 µM) was added. RNA was extracted at 48 hpe and expression of *IFN-β, MX1* or *RSAD2* mRNA was evaluated by qRT-PCR. Data was normalised by GAPDH expression and is expressed as fold change compared to the DMSO vehicle-treated control. Graph shows mean ± standard error from two independent experiments each done in quadruplicate.

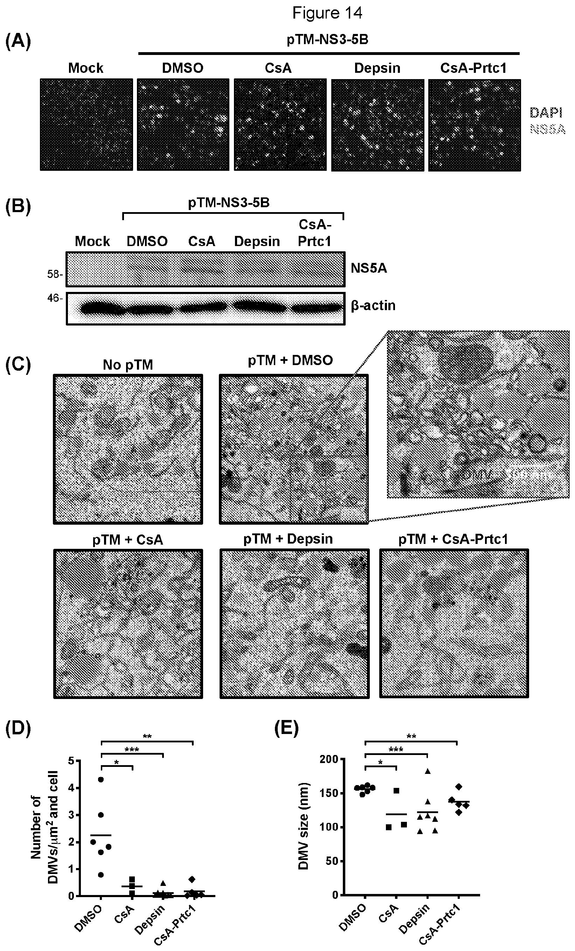

**Figure 14****: Antiviral CypI disrupt formation of the HCV replication organelle. (A-E)** Huh7-Lunet/T7 cells were transfected with pTM_NS3-5B and treated with CypI (5X EC₉₀) at 4 hours post-transfection. Transfection efficiency and NS5A expression were evaluated 24 hours later by immunofluorescence **(A)** or Western blot for NS5A **(B). (C)** Representative electron micrographs showing the effect of CypI treatment on DMV formation. **(D-E)** The number and size of DMVs in 3-7 different cells per condition were quantitated using ImageJ. Statistical significance was evaluated by t-test using GraphPad Prism (* *p*-value < 0.05; ** *p*-value < 0.01; *** *p*-value < 0.001).

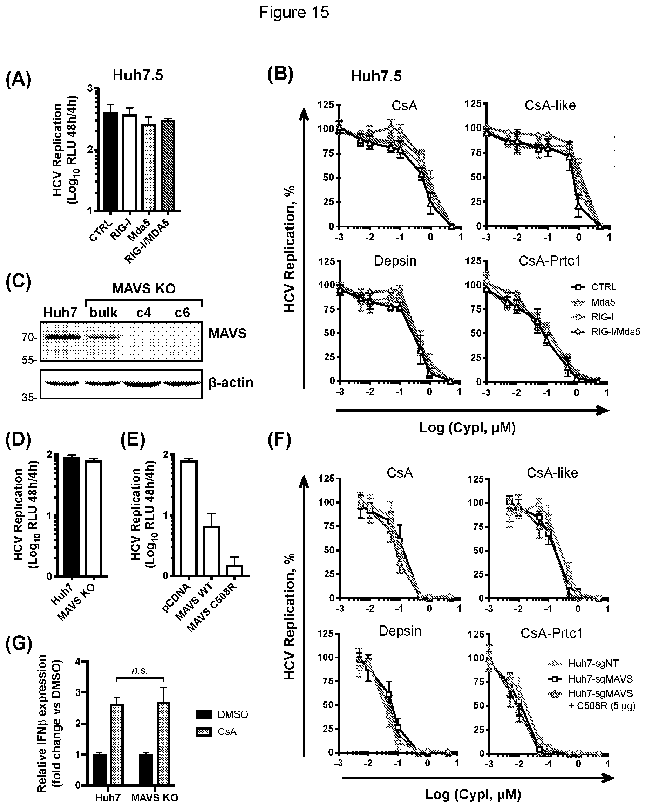

**Figure 15****: The RLR/MAVS pathway does not contribute to the antiviral potency of CypI.** Huh7.5 cells stably expressing RIG-I, Mda5 or both were electroporated with HCV replicon RNA and treated with increasing concentrations of CypI at 4 hpe. Replication was measured by luciferase activity at 48 hpe and is expressed as RLU **(A)** or percentage relative to the DMSO vehicle-treated control **(B).** Expression of RIG, Mda5 or both did not significantly affect HCV replication at 48 hours **(A)** or CypI dose-response curves in Huh7.5 cells **(B). (C)** Western blot detecting MAVS in single cell cloned Huh7 cells following MAVS knockout by CRISPR/Cas9. Huh7-sgMAVS cells were electroporated with 5 µg HCV replicon RNA in the presence or absence of plasmid encoding wild type MAVS (MAVS-WT) or mutant MAVS-C508R (conferring NS3/4A protease resistance). CypI were added at 4 hpe. Replication was measured by luciferase activity at 48 hpe and is expressed as RLU **(D)** or percentage relative to DMSO treated control **(E).** HCV replication was not affected by knockout of MAVS **(D)** but was decreased by transfection of plasmid encoding MAVS-C508R **(E).** The presence or absence of MAVS did not affect the CypI dose response curves (F). **(F)** Huh7 or Huh7 MAVS KO cells were electroporated with HCV replicon RNA as described above, and treated with 5 µM CsA at 4 hpe. At 48 hpe, RNA was extracted and expression of *IFN-β* mRNA was evaluated by qRT-PCR. Data was normalised by GAPDH expression and is expressed as fold change compared to the DMSO vehicle-treated control. **(A-F)** All graphs show means ± standard deviation from at least three independent experiments each performed in triplicate. Statistical significance was evaluated by t-test using GraphPad Prism (n.s., not significant; *p*-value > 0.05).

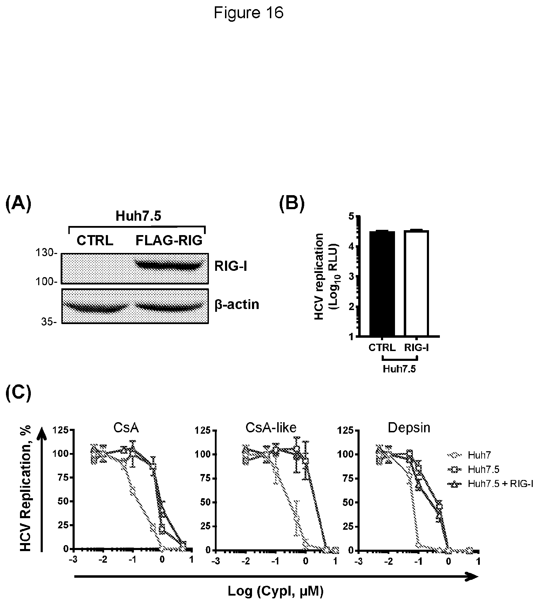

**Figure 16****: Expression of RIG-I in Huh7.5 cells does not affect CypI potency.** Approximately 2 x 10⁶ Huh7.5 were electroporated with 5 µg HCV replicon RNA in the presence or absence of 5 µg FLAG-RIG-I plasmid. **(A)** Western blot showing RIG-I expression in the transfected cells. **(B)** CypI were added at 4 hpe. Replication was measured by luciferase activity at 48 hpe and is expressed as RLU **(B)** or percentage relative to DMSO treated control **(C).** Graphs show means ± standard deviation from two independent experiments each performed in triplicate.

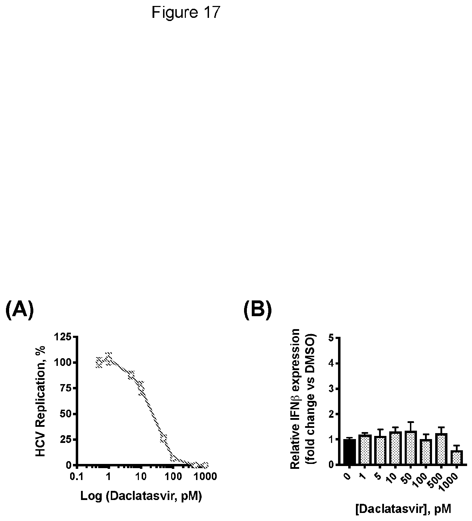

**Figure 17****: Daclatasvir treatment does not induce IFN expression in HCV-replicating Huh7 cells.** Approximately 2 x 10⁶ Huh7.5 were electroporated with 5 µg HCV replicon RNA. At 4 hpe, DMSO vehicle or increasing concentrations of daclatasvir were added. **(A)** Replication was measured by luciferase activity at 48 hpe and is expressed as RLU. **(B)** RNA was extracted at 48 hpe and expression of IFN-β mRNA was evaluated by qRT-PCR. Data was normalised by GAPDH expression and is expressed as fold change compared to the DMSO vehicle-treated control. Graphs show means ± standard error from two independent experiments each done in triplicate **(A)** or quadruplicate **(B).**

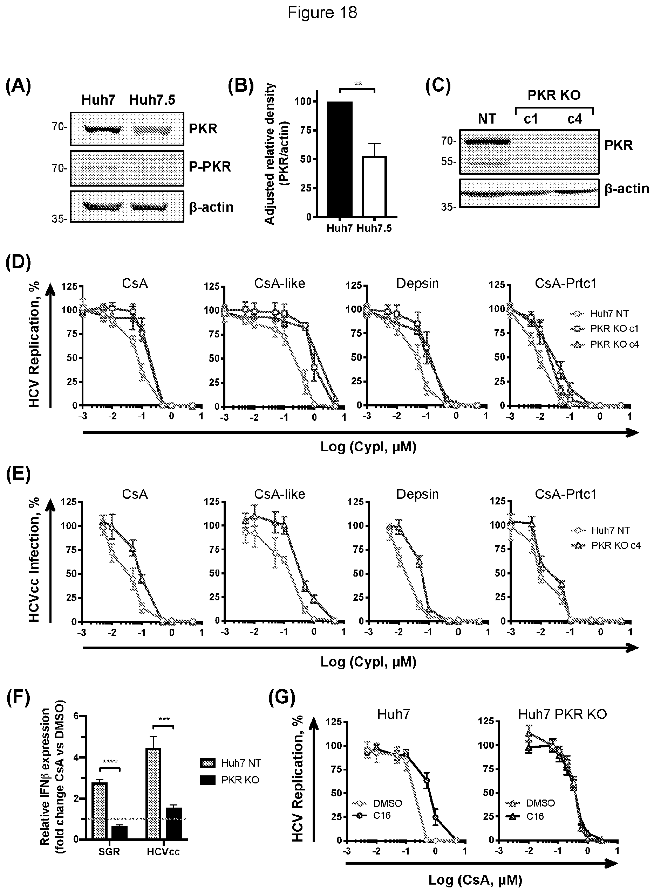

**Figure 18****: PKR modulates the antiviral potency of CypI against HCV.** PKR expression and phosphorylation is reduced in Huh7.5 cells **(A)** Huh7 or Huh7.5 cells electroporated with *in vitro* transcribed HCV replicon RNA were lysed at 48 hpe, and PKR expression and phosphorylation assessed by Western blot. One representative blot out of three independent experiments is shown. **(B)** Quantitation of band density from three independent experiments showing adjusted relative density normalised to the actin loading control. **(C)** Western blot detecting PKR expression in single cell cloned Huh7 cells following PKR knockout by CRISPR/Cas9. **(D)** CypI potency against HCV replication is decreased in the absence of PKR. Non targeted Huh7 (Huh7 (NT)) or Huh7 PKR KO clones 1 (c1) or 4 (c4) were electroporated with *in vitro* transcribed HCV replicon RNA and CypI added at 4 hpe. Replication was measured by luciferase activity at 48 hpe and is expressed as percentage relative to DMSO treated control. **(E)** Huh7 NT or PKR KO cells clone 4 (c4) were infected with HCVcc and treated with increasing concentrations of CypI at 4 hpi. Replication was measured by luciferase activity after 72 h and is expressed as percentage relative to DMSO treated control. **(F)** Huh7 NT or PKR KO cells were electroporated with HCV replicon RNA or infected with HCVcc, and treated with 5 µM CsA at 4 hpe. At 48 hpe, RNA was extracted and expression of *IFN-β* mRNA was evaluated by qRT-PCR. Data was normalised by GAPDH expression and is expressed as fold change compared to the DMSO vehicle-treated control. **(G)** Huh7 or Huh7 PKR KO cells were electroporated as described above, and at 4 hpe were treated with increasing concentrations of CsA in the presence or absence of the PKR inhibitor C16 (1 µM). C16 decreased CypI potency in Huh7 cells, but not in Huh7 PKR KO cells. **(A-F)** All graphs show means ± standard deviation from at least three independent experiments each performed in triplicate. Statistical significance was evaluated by t-test using GraphPad Prism (**** *p*-value < 0.0001; *** *p-*value < 0.001; ** *p*-value < 0.005).

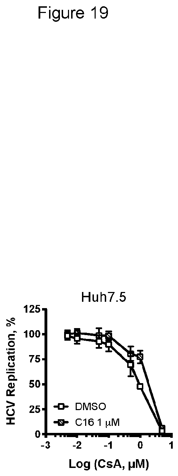

**Figure 19****: PKR inhibitor C16 only minimally affects CsA potency in Huh7.5 cells.** Huh7.5 cells were electroporated as described above, and at 4 hpe were treated with increasing concentrations of CsA in the presence or absence of the PKR inhibitor C16 (1 µM). C16 only minimally decreased CypI potency in Huh7.5 cells. Mean ± standard deviation from two independent experiments each performed in triplicate.

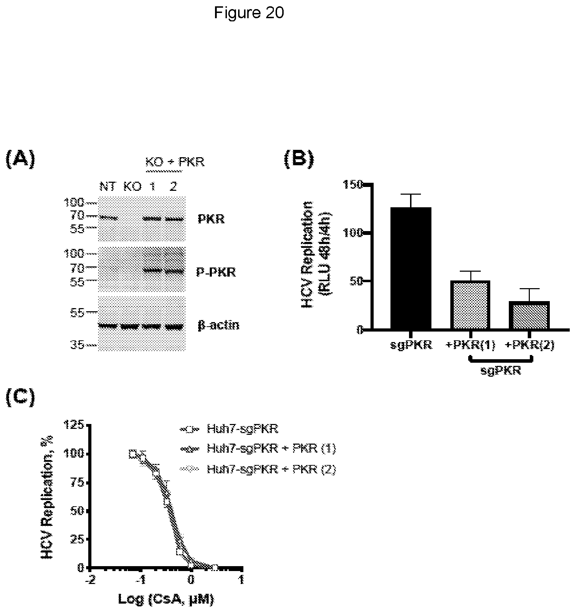

**Figure 20****: PKR overexpression does not affect HCV sensitivity to CsA. (A)** Western blot showing expression of PKR in Huh7 NT, Huh7 PKR KO (c4) or Huh7 PKR KO cells stably overexpressing PKR. **(B)** HCV replication in Huh7 PKR KO cells is inhibited by ectopic expression of PKR. **(C)** Expression of ectopic PKR does not affect HCV sensitivity to CsA.

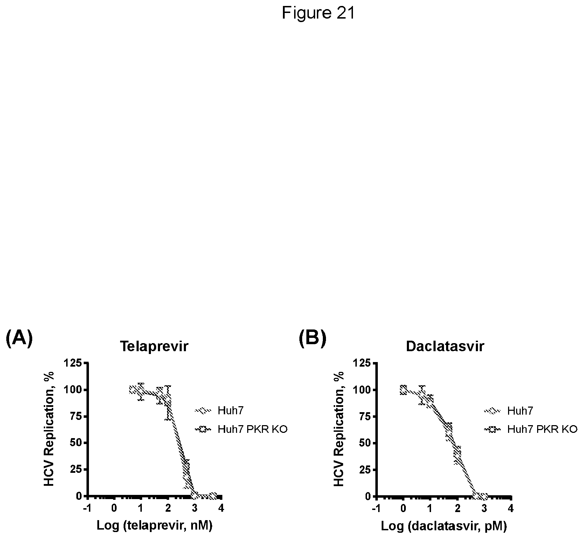

**Figure 21****: PKR does not affect HCV sensitivity to telaprevir or daclatasvir.** Huh7 NT or Huh7 PKR KO c4 were electroporated with *in vitro* transcribed HCV replicon RNA, and the indicated concentrations of telaprevir **(A)** or daclatasvir **(B)** were added at 4 hpe. Replication was measured by luciferase activity at 48 hpe and is expressed as percentage relative to DMSO treated control. All graphs show means ± standard deviation from three independent experiments each performed in triplicate.

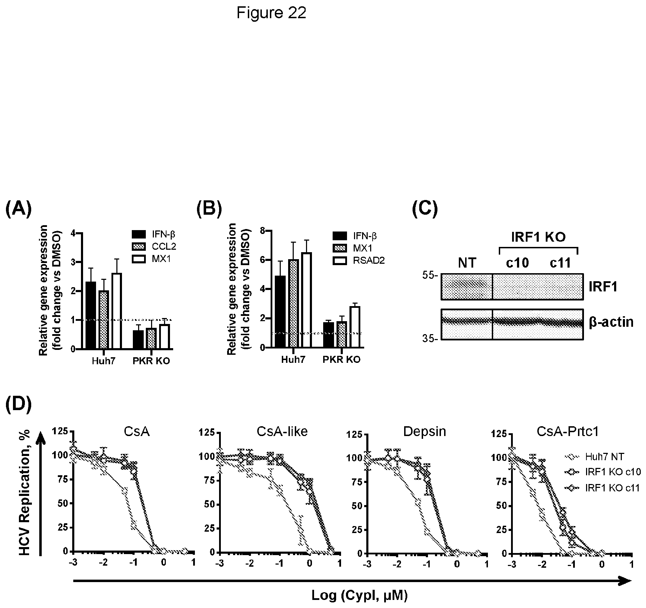

**Figure 22****: PKR induces IRF1-dependent intrinsic antiviral responses in HCV-replicating CypI-treated Huh7 cells. (A-B)** Induction of IRF1 target gene expression in HCV-replicating **(A)** or HCV-infected **(B)** cells depends on PKR. Expression of *IFN-β, CCL2, MX1* or *RSAD2* mRNA was evaluated by qRT-PCR at 48 hpe in Huh7 NT, or PKR KO cells, electroporated with HCV replicon RNA or infected with HCVcc and treated with 5 µM CsA at 4 hpe. Data were normalised by GAPDH expression and are expressed as fold change compared to the DMSO vehicle-treated control. **(C)** Western blot detecting IRF1 in Huh7 cells following IRF1 knockout by CRISPR/Cas9 and single cell cloning. **(D)** CypI potency against HCV replication was decreased in the absence of IRF1. HCV replication in Huh7 NT or IRF1 KO cells, measured by luciferase activity at 48 hpe, after CypI addition at 4 hpe, expressed as percentage relative to DMSO treated control. **(A-D)** All graphs show means ± standard deviation from two or three independent experiments each performed in triplicate.

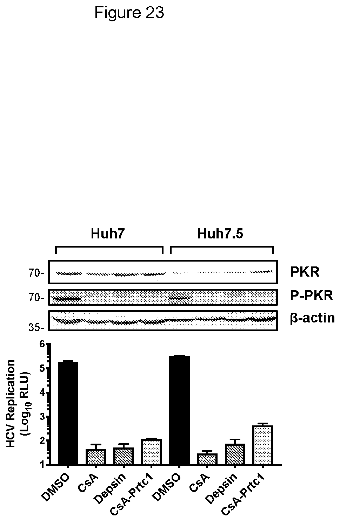

**Figure 23****:** CypI treatment inhibits PKR autophosphorylation at T446. Huh7 or Huh7.5 cells, electroporated with HCV replicon RNA and treated with fully inhibitory concentrations of CypI (5 µM) 4 h later, were extracted and PKR expression and phosphorylation were evaluated by Western blot **(A)** and HCV replication **(B)** at 48 hours. **(A)** Quantitation of PKR band density showing adjusted relative density normalised to the actin loading control.

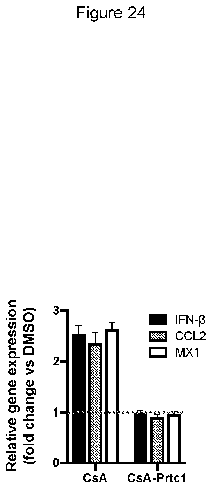

**Figure 24****: CsA, but not CsA-Prtc1, induces expression of antiviral genes.** Approximately 2 x 10⁶ Huh7 cells were electroporated with 5 µg HCV replicon RNA. At 4 hpe, DMSO vehicle or CypI (5 µM) were added. RNA was extracted at 48 hpe and expression of *IFN-β, CCL2* or *MX1* mRNA was evaluated by qRT-PCR. Data was normalised by GAPDH expression and is expressed as fold change compared to the DMSO vehicle-treated control. Graph shows mean ± standard error from three independent experiments each done in quadruplicate.

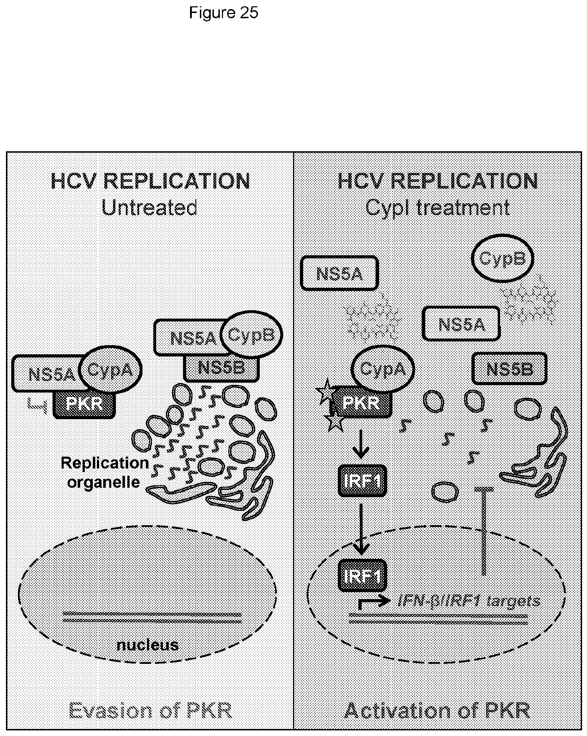

**Figure 25****:** Model where CypB forms a complex with NS5A and NS5B to directly regulate HCV RNA replication, while CypA forms a complex with NS5A and PKR, leading to inhibition of PKR-dependent antiviral responses. CypI, which target both CypA and CypB inhibit HCV replication by targeting CypB, and they disrupt the CypA-NS5A interaction.

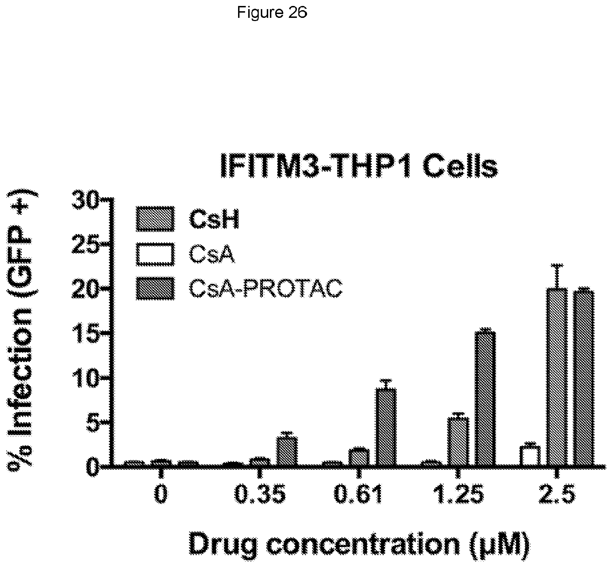

**Figure 26****:** As explained in Example 2, this figure illustrates the effect of CsH, CsA and CsA-PROTAC in enhancing transduction of an HIV vector encoding GFP. HIV-GFP transduction read out by flow cytometry to measure % of GFP positive cells 48 h post transduction. The drugs were added at the time of transduction and remain present throughout the experiment. At each drug concentration, CsH is the leftmost bar, CsA the central bar and CsA-PROTAC the rightmost bar.

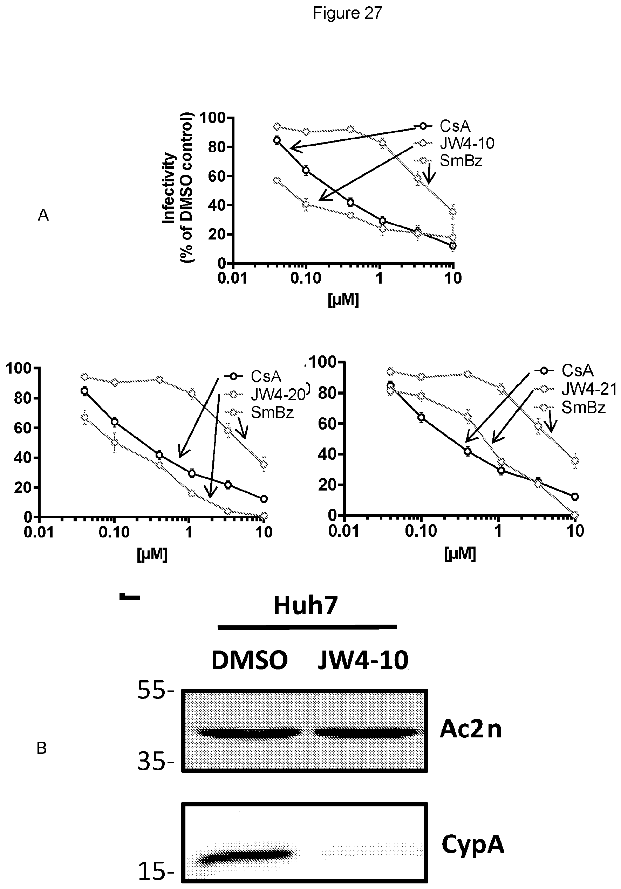

**Figure 27****:** As explained in Example 3, this figure illustrates: in panel A, infection of U87 cells with HIV-1 (GFP), with a titration of inhibitors from 10µm-0.04µM (n=2 ± SEM), the vertical axis in each graph representing infectivity as a % of DMSO control; in panel B, Western blot of CypA in Huh7 cells 48hrs after treatment with and without JW4-10.

### DETAILED DESCRIPTION OF THE INVENTION

### Definitions

As used herein, the term "alkyl" includes both saturated straight chain and branched alkyl groups. Preferably, an alkyl group is a C₁₋₂₀ alkyl group, more preferably a C₁₋₁₅, more preferably still a C₁₋₁₂ alkyl group, more preferably still, a C₁₋₆ alkyl group, and most preferably a C₁₋₄ alkyl group. Particularly preferred alkyl groups include, for example, methyl, ethyl, propyl, isopropyl, butyl, isobutyl, tert-butyl, pentyl and hexyl. The term "alkylene" should be construed accordingly.

As used herein, the term "alkenyl" refers to a group containing one or more carbon-carbon double bonds, which may be branched or unbranched. Preferably the alkenyl group is a C₂₋₂₀ alkenyl group, more preferably a C₂₋₁₅ alkenyl group, more preferably still a C₂₋₁₂ alkenyl group, or preferably a C₂₋₆ alkenyl group, and most preferably a C₂₋₄ alkenyl group. The term "alkenylene" should be construed accordingly.

As used herein, the term "alkynyl" refers to a carbon chain containing one or more triple bonds, which may be branched or unbranched. Preferably the alkynyl group is a C₂₋₂₀ alkynyl group, more preferably a C₂₋₁₅ alkynyl group, more preferably still a C₂₋₁₂ alkynyl group, or preferably a C₂₋₆ alkynyl group and most preferably a C₂₋₄ alkynyl group. The term "alkynylene" should be construed accordingly.

Unless otherwise specified, an alkyl, alkenyl or alkynyl group is typically unsubstituted. However, where such a group is indicated to be unsubstituted or substituted, one or more hydrogen atoms are optionally replaced by halogen atoms or sulfonic acid groups. Preferably, a substituted alkyl, alkenyl or alkynyl group has from 1 to 10 substituents, more preferably 1 to 5 substituents, more preferably still 1, 2 or 3 substituents and most preferably 1 or 2 substituents, for example 1 substituent. Preferably a substituted alkyl, alkenyl or alkynyl group carries not more than 2 sulfonic acid substituents. Halogen atoms are preferred substituents. Preferably, though, an alkyl, alkenyl or alkynyl group is unsubstituted. A haloalkyl group means an alkyl group that is substituted by one or more halogen atoms.

As used herein, halogen atoms are typically F, Cl, Br or I atoms.

As used herein, a C₆₋₁₀ aryl group is a monocyclic or polycyclic 6- to 10-membered aromatic hydrocarbon ring system having from 6 to 10 carbon atoms. Phenyl is preferred. The term "arylene" should be construed accordingly.

As used herein, a 5- to 10- membered heteroaryl group is a monocyclic or polycyclic 5- to 10- membered aromatic ring system, such as a 5- or 6- membered ring, containing at least one heteroatom, for example 1, 2, 3 or 4 heteroatoms, selected from O, S and N. When the ring contains 4 heteroatoms these are preferably all nitrogen atoms. The term "heteroarylene" should be construed accordingly.

Examples of monocyclic heteroaryl groups include thienyl, furyl, pyrrolyl, imidazolyl, thiazolyl, isothiazolyl, pyrazolyl, oxazolyl, isoxazolyl, triazolyl, thiadiazolyl, oxadiazolyl, pyridinyl, pyridazinyl, pyrimidinyl, pyrazinyl, triazinyl and tetrazolyl groups. Examples of polycyclic heteroaryl groups include benzothienyl, benzofuryl, benzimidazolyl, benzothiazolyl, benzisothiazolyl, benzoxazolyl, benzisoxazolyl, benztriazolyl, indolyl, isoindolyl and indazolyl groups. Preferred polycyclic groups include indolyl, isoindolyl, benzimidazolyl, indazolyl, benzofuryl, benzothienyl, benzoxazolyl, benzisoxazolyl, benzothiazolyl and benzisothiazolyl groups, more preferably benzimidazolyl, benzoxazolyl and benzothiazolyl, most preferably benzothiazolyl. However, monocyclic heteroaryl groups are preferred.

Preferably the heteroaryl group is a 5- to 6- membered heteroaryl group. Particularly preferred heteroaryl groups are thienyl, pyrrolyl, imidazolyl, thiazolyl, isothiazolyl, pyrazolyl, oxazolyl, isoxazolyl, triazolyl, pyridinyl, pyridazinyl, pyrimidinyl and pyrazinyl groups. More preferred groups are thienyl, pyridinyl, pyridazinyl, pyrimidinyl, pyrazinyl, pyrrolyl and triazinyl, most preferably pyridinyl.

As used herein, a 5- to 10- membered heterocyclyl group is a non-aromatic, saturated or unsaturated, monocyclic or polycyclic C₅₋₁₀ carbocyclic ring system in which one or more, for example 1, 2, 3 or 4, of the carbon atoms are replaced with a moiety selected from N, O, S, S(O) and S(O)₂. Preferably, the 5- to 10- membered heterocyclyl group is a 5- to 6- membered ring. The term "heterocyclyene" should be construed accordingly.

Examples of heterocyclyl groups include azetidinyl, oxetanyl, thietanyl, pyrrolidinyl, imidazolidinyl, oxazolidinyl, isoxazolidinyl, thiazolidinyl, isothiazolidinyl, tetrahydrofuranyl, tetrahydrothienyl, tetrahydropyranyl, tetrahydrothiopyranyl, dithiolanyl, dioxolanyl, pyrazolidinyl, piperidinyl, piperazinyl, hexahydropyrimidinyl, methylenedioxyphenyl, ethylenedioxyphenyl, thiomorpholinyl, S-oxo-thiomorpholinyl, S,S-dioxo-thiomorpholinyl, morpholinyl, 1,3-dioxolanyl, 1,4-dioxolanyl, trioxolanyl, trithianyl, imidazolinyl, pyranyl, pyrazolinyl, thioxolanyl, thioxothiazolidinyl, 1H-pyrazol-5-(4H)-onyl, 1,3,4-thiadiazol-2(3H)-thionyl, oxopyrrolidinyl, oxothiazolidinyl, oxopyrazolidinyl, succinimido and maleimido groups and moieties. Preferred heterocyclyl groups are pyrrolidinyl, imidazolidinyl, oxazolidinyl, isoxazolidinyl, thiazolidinyl, isothiazolidinyl, tetrahydrofuranyl, tetrahydrothienyl, tetrahydropyranyl, tetrahydrothiopyranyl, dithiolanyl, dioxolanyl, pyrazolidinyl, piperidinyl, piperazinyl, hexahydropyrimidinyl, thiomorpholinyl and morpholinyl groups and moieties. More preferred heterocyclyl groups are tetrahydropyranyl, tetrahydrothiopyranyl, thiomorpholinyl, tetrahydrofuranyl, tetrahydrothienyl, piperidinyl, piperazinyl, morpholinyl and pyrrolidinyl groups. Most preferred groups are heterocyclyl groups are tetrahydropyranyl, tetrahydrothiopyranyl, thiomorpholinyl, tetrahydrofuranyl, tetrahydrothienyl, piperidinyl, morpholinyl and pyrrolidinyl groups.

For the avoidance of doubt, although the above definitions of heteroaryl and heterocyclyl groups refer to an "N" moiety which can be present in the ring, as will be evident to a skilled chemist the N atom will be protonated (or will carry a substituent as defined below) if it is attached to each of the adjacent ring atoms via a single bond.

As used herein, a C₃₋₇ carbocyclyl group is a non-aromatic saturated or unsaturated hydrocarbon ring having from 3 to 7 carbon atoms. Preferably it is a saturated or mono-unsaturated hydrocarbon ring (i.e. a cycloalkyl moiety or a cycloalkenyl moiety) having from 3 to 7 carbon atoms, more preferably having from 5 to 6 carbon atoms. Examples include cyclopropyl, cyclobutyl, cyclopentyl and cyclohexyl and their mono-unsaturated variants. Particularly preferred carbocyclic groups are cyclopentyl and cyclohexyl. The term "carbocyclylene" should be construed accordingly.

Where specified, 0, 1 or 2 carbon atoms in a carbocyclyl or heterocyclyl group may be replaced by -C(O)- groups. As used herein, the "carbon atoms" being replaced are understood to include the hydrogen atoms to which they are attached. When 1 or 2 carbon atoms are replaced, preferably two such carbon atoms are replaced. Preferred such carbocyclyl groups include a benzoquinone group and preferred such heterocyclyl groups include succinimido and maleimido groups.

Unless otherwise specified, an aryl, heteroaryl, carbocyclyl or heterocyclyl group is typically unsubstituted. However, where such a group is indicated to be unsubstituted or substituted, one or more hydrogen atoms are optionally replaced by halogen atoms or C₁₋₆ alkyl, C₁₋₆ haloalkyl, C₁₋₆ alkoxy, -(C₁₋₆ alkyl)ₙC(O)O(C₁₋₆ alkyl) (where n=0 or 1), -(C₁₋₆ alkyl)ₙOC(O)(C₁₋₆ alkyl) (where n=0 or 1), C₁₋₆ alkylthiol, -N(R_{N})₂ (wherein each R_{N} independently represents a hydrogen atom or a C₁₋₆ alkyl group), -CN, -S(O)₂NH₂, nitro or sulfonic acid groups. Preferably, a substituted aryl, heteroaryl, carbocyclyl or heterocyclyl group has from 1 to 4 substituents, more preferably 1 to 2 substituents and most preferably 1 substituent. Preferably a substituted aryl, heteroaryl, carbocyclyl or heterocyclyl group carries not more than 2 nitro substituents and not more than 2 sulfonic acid substituents.

As used herein, a C₁₋₆ alkoxy group is a C₁₋₆ alkyl (e.g. a C₁₋₄ alkyl) group which is attached to an oxygen atom.

As used herein, a C₁₋₆ alkylthiol group is a C₁₋₆ alkyl (e.g. a C₁₋₄ alkyl) group which is attached to a sulfur atom.

In some instances, the compounds of the present invention can be provided in the form of a pharmaceutical salt. Substantially any pharmaceutically acceptable salt can be used. Those skilled in the art of preparing compounds for use in medical applications will be familiar with suitable such salt compounds. For instance, the present compounds may be in the form of a salt with a pharmaceutically acceptable base. Pharmaceutically acceptable bases include, but are by no means limited to, alkali metal (e.g. sodium or potassium) and alkali earth metal (e.g. calcium or magnesium) hydroxides and organic bases such as alkyl amines, aralkyl amines or heterocyclic amines.

### Cyclosporine analogues - formula (III)

An aspect of the invention relates to a cyclosporine analogue that is a compound of formula (III) or a pharmaceutical salt thereof.

The stereochemistry at the ethenyl group that connects the cyclosporine ring system to the group -L_{III}-B can be either *E* or *Z*. Thus, the chemical formula (III) embraces both chemical formula (IIIa) and (IIIb):

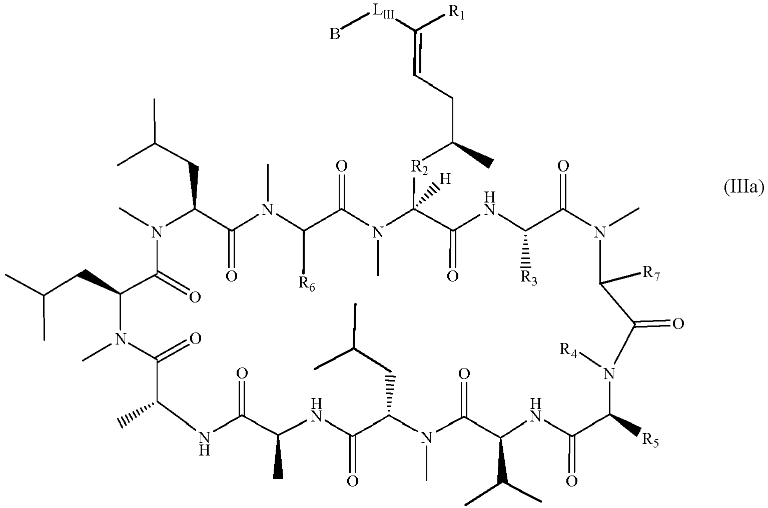

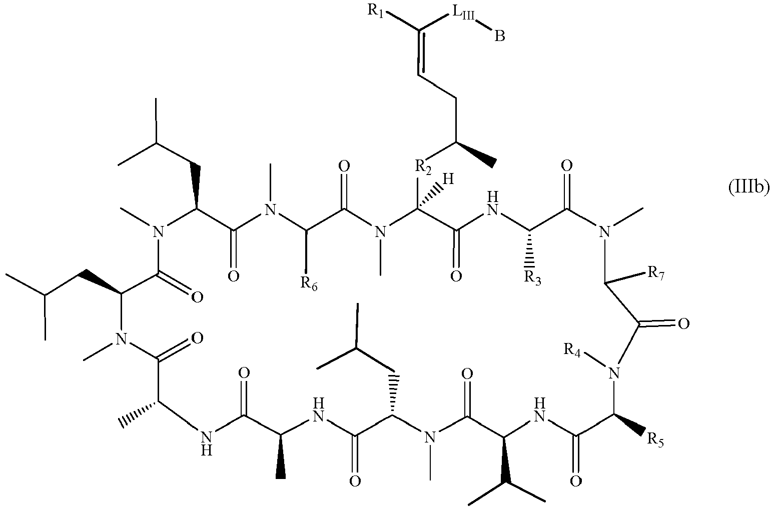

Moieties of formula (IIIa) are currently preferred.

The cyclosporine analogue of the present invention comprises a group of formula -L_{III}-B. L_{III} is a direct bond or a linker moiety.

Where L_{III} is a linker moiety (not a direct bond), then it represents a moiety which is a C₁₋₂₀ alkylene group, a C₂₋₂₀ alkenylene group or a C₂₋₂₀ alkynylene group (preferably a C₁₋₁₅ alkylene group, a C₂₋₁₅ alkenylene group or a C₂₋₁₅ alkynylene group, more preferably a C₁₋₁₅ alkylene group and most preferably a C₁₋₁₂ alkylene group), which is unsubstituted or substituted by one or more substituents selected from halogen atoms and sulfonic acid groups (preferably unsubstituted), and in which
(a) 0, 1, 2 or 3 (preferably 0, 1 or 2, more preferably 0 or 1, most preferably 0) carbon atoms are replaced by groups selected from C₆₋₁₀ arylene, 5- to 10-membered heteroarylene, C₃₋₇ carbocyclylene and 5- to 10-membered heterocyclylene groups, and
(b) up to half (e.g., 0-8, more preferably 0-5, most preferably 0-3) -CH₂- groups are replaced by groups selected from -O-, -S-, -C(O)- and -N(C₁₋₆ alkyl)- groups (preferably groups selected from -O- and -C(O)-), wherein:
   (i) said arylene, heteroarylene, carbocyclylene and heterocyclylene groups are unsubstituted or substituted by one or more substituents selected from halogen atoms and C₁₋₆ alkyl, C₁₋₆ haloalkyl, C₁₋₆ alkoxy, -(C₁₋₆ alkyl)ₙC(O)O(C₁₋₆ alkyl) (where n=0 or 1), -(C₁₋₆ alkyl)ₙOC(O)(C₁₋₆ alkyl) (where n=0 or 1), C₁₋₆ alkylthiol, -N(R_{N})₂ (wherein each R_{N} independently represents a hydrogen atom or a C₁₋₆ alkyl group), -CN, -S(O)₂NH₂, nitro and sulfonic acid groups; and
   (ii) 0, 1 or 2 carbon atoms in said carbocyclylene and heterocyclylene groups are replaced by -C(O)- groups.

For the avoidance of doubt, with respect to (a) in this definition, it is acceptable to replace adjacent -CH₂- groups where chemically meaningful, e.g. to replace -CH₂-CH₂- with -C(O)-O-, -O-C(O)-, etc. Similarly, it is acceptable to replace the -CH₂-of a terminal methyl group (i.e., CH₃ or -CH₂-H), e.g. replace -CH₃ with -OH. Preferably not more than two adjacent -CH₂- groups are replaced (e.g., typically any replacements do not involve replacement of three or more adjacent/contiguous -CH₂- groups).

In one further preferred embodiment, L_{III} is a C₁₋₅ alkylene group, e.g. a C₂₋₃ alkylene group.

### Synthesis

Compounds of the invention may be prepared by standard methods known in the art. Representative examples of synthesis of certain compounds are provided in the working examples.

### Gene therapy applications

The compounds of the present invention can be used in gene therapy, such as for increasing the efficiency of transduction of human haematopoietic stem cells (HSC) and/or progenitor cells by a gene therapy vector. The present invention embraces such uses of the compound, associated pharmaceutical compositions, and methods of treatment. Advantageously, it has been found that the compounds of the invention are easy-to-synthesise, highly potent and selective IFITM3 inhibitors that can be utilised to enhance HIV-vector infection, reduce vector dose required and overcome patient variability. As further discussed elsewhere herein, preferred embodiments also make use of compounds that have reduced binding to CypA, and which therefore overcome a limitation associated with the previously described use of CsA in similar applications.

### Cells

A stem cell is able to differentiate into many cell types. A cell that is able to differentiate into all cell types is known as totipotent. In mammals, only the zygote and early embryonic cells are totipotent. Stem cells are found in most, if not all, multicellular organisms. They are characterised by the ability to renew themselves through mitotic cell division and differentiate into a diverse range of specialised cell types. The two broad types of mammalian stem cells are embryonic stem cells that are isolated from the inner cell mass of blastocysts, and adult stem cells that are found in adult tissues. In a developing embryo, stem cells can differentiate into all of the specialised embryonic tissues. In adult organisms, stem cells and progenitor cells act as a repair system for the body, replenishing specialised cells, but also maintaining the normal turnover of regenerative organs, such as blood, skin or intestinal tissues.

Haematopoietic stem cells (HSCs) are multipotent stem cells that may be found, for example, in peripheral blood, bone marrow and umbilical cord blood. HSCs are capable of self-renewal and differentiation into any blood cell lineage. They are capable of recolonising the entire immune system, and the erythroid and myeloid lineages in all the haematopoietic tissues (such as bone marrow, spleen and thymus). They provide for lifelong production of all lineages of haematopoietic cells.

Haematopoietic progenitor cells have the capacity to differentiate into a specific type of cell. In contrast to stem cells however, they are already more specific: they are pushed to differentiate into their "target" cell. A difference between stem cells and progenitor cells is that stem cells can replicate indefinitely, whereas progenitor cells can only divide a limited number of times. Haematopoietic progenitor cells can be rigorously distinguished from HSCs only by functional in vivo assay (i.e. transplantation and demonstration of whether they can give rise to all blood lineages over prolonged time periods).

Preferably, the haematopoietic stem and progenitor cells of the invention comprise the CD34 cell surface marker (denoted as CD34+).

A population of haematopoietic stem and/or progenitor cells may be obtained from a tissue sample. For example, a population of haematopoietic stem and/or progenitor cells may be obtained from peripheral blood (e.g. adult and foetal peripheral blood), umbilical cord blood, bone marrow, liver or spleen. Preferably, these cells are obtained from peripheral blood or bone marrow. They may be obtained after mobilisation of the cells in vivo by means of growth factor treatment.

Mobilisation may be carried out using, for example, G-CSF, plerixaphor or combinations thereof. Other agents, such as NSAIDs and dipeptidyl peptidase inhibitors, may also be useful as mobilising agents.

With the availability of the stem cell growth factors GM-CSF and G-CSF, most haematopoietic stem cell transplantation procedures are now performed using stem cells collected from the peripheral blood, rather than from the bone marrow. Collecting peripheral blood stem cells provides a bigger graft, does not require that the donor be subjected to general anaesthesia to collect the graft, results in a shorter time to engraftment and may provide for a lower long-term relapse rate. Bone marrow may be collected by standard aspiration methods (either steady-state or after mobilisation), or by using next-generation harvesting tools (e.g. Marrow Miner). In addition, haematopoietic stem and/or progenitor cells may also be derived from induced pluripotent stem cells.

HSCs are typically of low forward scatter and side scatter profile by flow cytometric procedures. Some are metabolically quiescent, as demonstrated by Rhodamine labelling which allows determination of mitochondrial activity. HSCs may comprise certain cell surface markers such as CD34, CD45, CD133, CD90 and CD49f. They may also be defined as cells lacking the expression of the CD38 and CD45RA cell surface markers. However, expression of some of these markers is dependent upon the developmental stage and tissue-specific context of the HSC. Some HSCs called "side population cells" exclude the Hoechst 33342 dye as detected by flow cytometry. Thus, HSCs have descriptive characteristics that allow for their identification and isolation.

CD38 is the most established and useful single negative marker for human HSCs. Human HSCs may also be negative for lineage markers such as CD2, CD3, CD14, CD16, CD19, CD20, CD24, CD36, CD56, CD66b, CD271 and CD45RA. However, these markers may need to be used in combination for HSC enrichment. By "negative marker" it is to be understood that human HSCs lack the expression of these markers.

CD34 and CD133 are the most useful positive markers for HSCs. Some HSCs are also positive for lineage markers such as CD90, CD49f and CD93. However, these markers may need to be used in combination for HSC enrichment. By "positive marker" it is to be understood that human HSCs express these markers.

A differentiated cell is a cell which has become more specialised in comparison to a stem cell or progenitor cell. Differentiation occurs during the development of a multicellular organism as the organism changes from a single zygote to a complex system of tissues and cell types. Differentiation is also a common process in adults: adult stem cells divide and create fully-differentiated daughter cells during tissue repair and normal cell turnover. Differentiation dramatically changes a cell's size, shape, membrane potential, metabolic activity and responsiveness to signals. These changes are largely due to highly-controlled modifications in gene expression. In other words, a differentiated cell is a cell which has specific structures and performs certain functions due to a developmental process which involves the activation and deactivation of specific genes. Here, a differentiated cell includes differentiated cells of the haematopoietic lineage such as monocytes, macrophages, neutrophils, basophils, eosinophils, erythrocytes, megakaryocytes/platelets, dendritic cells, T-cells, B-cells and NK-cells. For example, differentiated cells of the haematopoietic lineage can be distinguished from stem cells and progenitor cells by detection of cell surface molecules which are not expressed or are expressed to a lesser degree on undifferentiated cells. Examples of suitable human lineage markers include CD33, CD13, CD14, CD15 (myeloid), CD19, CD20, CD22, CD79a (B), CD36, CD71, CD235a (erythroid), CD2, CD3, CD4, CD8 (T) and CD56 (NK).

By "isolated population" of cells it is to be understood that the population of cells has been previously removed from the body. An isolated population of cells may be cultured and manipulated ex vivo or in vitro using standard techniques known in the art. An isolated population of cells may later be reintroduced into a subject. Said subject may be the same subject from which the cells were originally isolated or a different subject. Methods and uses carried out on isolated populations of cells are ex vivo or in vitro methods and uses.

A population of cells may be purified selectively for cells that exhibit a specific phenotype or characteristic, and from other cells which do not exhibit that phenotype or characteristic, or exhibit it to a lesser degree. For example, a population of cells that expresses a specific marker (such as CD34) may be purified from a starting population of cells. Alternatively, or in addition, a population of cells that does not express another marker (such as CD38) may be purified.

By "enriching" a population of cells for a certain type of cells it is to be understood that the concentration of that type of cells is increased within the population. The concentration of other types of cells may be concomitantly reduced.

Purification or enrichment may result in the population of cells being substantially pure of other types of cell. Purifying or enriching for a population of cells expressing a specific marker (e.g. CD34 or CD38) may be achieved by using an agent that binds to that marker, preferably substantially specifically to that marker.

An agent that binds to a cellular marker may be an antibody, for example an anti-CD34 or anti-CD38 antibody. The term "antibody" refers to complete antibodies or antibody fragments capable of binding to a selected target, and including Fv, ScFv, F(ab') and F(ab')2, monoclonal and polyclonal antibodies, engineered antibodies including chimeric, CDR-grafted and humanised antibodies, and artificially selected antibodies produced using phage display or alternative techniques. In addition, alternatives to classical antibodies may also be used in the invention, for example "avibodies", "avimers", "anticalins", "nanobodies" and "DARPins".

The agents that bind to specific markers may be labelled so as to be identifiable using any of a number of techniques known in the art. The agent may be inherently labelled, or may be modified by conjugating a label thereto. By "conjugating" it is to be understood that the agent and label are operably linked. This means that the agent and label are linked together in a manner which enables both to carry out their function (e.g. binding to a marker, allowing fluorescent identification, or allowing separation when placed in a magnetic field) substantially unhindered. Suitable methods of conjugation are well known in the art and would be readily identifiable by the skilled person.

A label may allow, for example, the labelled agent and any cell to which it is bound to be purified from its environment (e.g. the agent may be labelled with a magnetic bead, or an affinity tag, such as avidin), detected or both. Detectable markers suitable for use as a label include fluorophores (e.g. green, cherry, cyan and orange fluorescent proteins) and peptide tags (e.g. His tags, Myc tags, FLAG tags and HA tags).

A number of techniques for separating a population of cells expressing a specific marker are known in the art. These include magnetic bead-based separation technologies (e.g. closed-circuit magnetic bead-based separation), flow cytometry, fluorescence-activated cell sorting (FACS), affinity tag purification (e.g. using affinity columns or beads, such biotin columns to separate avidin-labelled agents) and microscopy-based techniques. It may also be possible to perform the separation using a combination of different techniques, such as a magnetic bead-based separation step followed by sorting of the resulting population of cells for one or more additional (positive or negative) markers by flow cytometry. Clinical grade separation may be performed, for example, using the CliniMACS^{®} system (Miltenyi). This is an example of a closed-circuit magnetic bead-based separation technology.

It is also envisaged that dye exclusion properties (e.g. side population or rhodamine labelling) or enzymatic activity (e.g. ALDH activity) may be used to enrich for HSCs.

The cells of the present invention may be formulated for administration to subjects with a pharmaceutically acceptable carrier, diluent or excipient. Suitable carriers and diluents include isotonic saline solutions, for example phosphate-buffered saline, and potentially contain human serum albumin. B Handling of the cell therapy product is preferably performed in compliance with FACT-JACIE International Standards for cellular therapy.

### Vectors

A vector is a tool that allows or facilitates the transfer of an entity from one environment to another. Vectors that are used to transduce haematopoietic stem and/or progenitor cells in the present invention may be viral vectors. The viral vectors may be derived from HIV-1, HIV-2, SIV, FIV, BIV, EIAV, CAEV or visna lentivirus. These viruses are all lentiviruses. By "vector derived from" a certain type of virus, it is to be understood that the vector comprises at least one component part derivable from that type of virus.

A retroviral vector may be derived from or may be derivable from any suitable retrovirus. A large number of different retroviruses have been identified. Examples include murine leukaemia virus (MLV), human T-cell leukaemia virus (HTLV), mouse mammary tumour virus (MMTV), Rous sarcoma virus (RSV), Fujinami sarcoma virus (FuSV), Moloney murine leukaemia virus (Mo-MLV), FBR murine osteosarcoma virus (FBR MSV), Moloney murine sarcoma virus (Mo-MSV), Abelson murine leukaemia virus (A-MLV), avian myelocytomatosis virus-29 (MC29) and avian erythroblastosis virus (AEV). A detailed list of retroviruses may be found in Coffin, J.M. et al. (1997) Retroviruses, Cold Spring Harbour Laboratory Press, 758-63.

Retroviruses may be broadly divided into two categories, "simple" and "complex". Retroviruses may be even further divided into seven groups. Five of these groups represent retroviruses with oncogenic potential. The remaining two groups are the lentiviruses and the spumaviruses. A review of these retroviruses is presented in Coffin, J.M. et al. (1997) Retroviruses, Cold Spring Harbour Laboratory Press, 758-63.

The basic structure of retrovirus and lentivirus genomes share many common features such as a 5' LTR and a 3' LTR. Between or within these are located a packaging signal to enable the genome to be packaged, a primer binding site, integration sites to enable integration into a host cell genome, and gag, pol and env genes encoding the packaging components - these are polypeptides required for the assembly of viral particles. Lentiviruses have additional features, such as rev and RRE sequences in HIV, which enable the efficient export of RNA transcripts of the integrated provirus from the nucleus to the cytoplasm of an infected target cell.

In the provirus, these genes are flanked at both ends by regions called long terminal repeats (LTRs). The LTRs are responsible for proviral integration and transcription. LTRs also serve as enhancer-promoter sequences and can control the expression of the viral genes.

The LTRs themselves are identical sequences that can be divided into three elements: U3, R and U5. U3 is derived from the sequence unique to the 3' end of the RNA. R is derived from a sequence repeated at both ends of the RNA. U5 is derived from the sequence unique to the 5' end of the RNA. The sizes of the three elements can vary considerably among different retroviruses.

In a defective retroviral vector genome gag, pol and env may be absent or not functional.

In a typical retroviral vector, at least part of one or more protein coding regions essential for replication may be removed from the virus. This makes the viral vector replication-defective. Portions of the viral genome may also be replaced by a library encoding candidate modulating moieties operably linked to a regulatory control region and a reporter moiety in the vector genome in order to generate a vector comprising candidate modulating moieties which is capable of transducing a target host cell and/or integrating its genome into a host genome.

Lentivirus vectors are part of the larger group of retroviral vectors. A detailed list of lentiviruses may be found in Coffin, J.M. et al. (1997) Retroviruses, Cold Spring Harbour Laboratory Press, 758-63. In brief, lentiviruses can be divided into primate and non-primate groups. Examples of primate lentiviruses include but are not limited to human immunodeficiency virus (HIV), the causative agent of human acquired immunodeficiency syndrome (AIDS); and simian immunodeficiency virus (SIV). Examples of non-primate lentiviruses include the prototype "slow virus" visna/maedi virus (VMV), as well as the related caprine arthritis-encephalitis virus (CAEV), equine infectious anaemia virus (EIAV), and the more recently described feline immunodeficiency virus (FIV) and bovine immunodeficiency virus (BIV).

The lentivirus family differs from retroviruses in that lentiviruses have the capability to infect both dividing and non-dividing cells (Lewis, P et al. (1992) EMBO J. 11: 3053-8; Lewis, P.F. et al. (1994) J. Virol. 68: 510-6). In contrast, other retroviruses, such as MLV, are unable to infect non-dividing or slowly dividing cells such as those that make up, for example, muscle, brain, lung and liver tissue.

A lentiviral vector, as used herein, is a vector which comprises at least one component part derivable from a lentivirus. Preferably, that component part is involved in the biological mechanisms by which the vector infects cells, expresses genes or is replicated.

The lentiviral vector may be a "primate" vector. The lentiviral vector may be a "non-primate" vector (i.e. derived from a virus which does not primarily infect primates, especially humans). Examples of non-primate lentiviruses may be any member of the family of lentiviridae which does not naturally infect a primate.

As examples of lentivirus-based vectors, HIV-1- and HIV-2-based vectors are described below.

The HIV-1 vector contains cis-acting elements that are also found in simple retroviruses. It has been shown that sequences that extend into the gag open reading frame are important for packaging of HIV-1. Therefore, HIV-1 vectors often contain the relevant portion of gag in which the translational initiation codon has been mutated. In addition, most HIV-1 vectors also contain a portion of the env gene that includes the RRE. Rev binds to RRE, which permits the transport of full-length or singly spliced mRNAs from the nucleus to the cytoplasm. In the absence of Rev and/or RRE, full-length HIV-1 RNAs accumulate in the nucleus. Alternatively, a constitutive transport element from certain simple retroviruses such as Mason-Pfizer monkey virus can be used to relieve the requirement for Rev and RRE. Efficient transcription from the HIV-1 LTR promoter requires the viral protein Tat.

Most HIV-2-based vectors are structurally very similar to HIV-1 vectors. Similar to HIV-1-based vectors, HIV-2 vectors also require RRE for efficient transport of the full-length or singly spliced viral RNAs.

In one system, the vector and helper constructs are from two different viruses, and the reduced nucleotide homology may decrease the probability of recombination. In addition to vectors based on the primate lentiviruses, vectors based on FIV have also been developed as an alternative to vectors derived from the pathogenic HIV-1 genome. The structures of these vectors are also similar to the HIV-1 based vectors.

Preferably the viral vector used in the present invention has a minimal viral genome. By "minimal viral genome" it is to be understood that the viral vector has been manipulated so as to remove the non-essential elements and to retain the essential elements in order to provide the required functionality to infect, transduce and deliver a nucleotide sequence of interest to a target host cell. Further details of this strategy can be found in WO 1998/017815.

Preferably the plasmid vector used to produce the viral genome within a host cell/packaging cell will have sufficient lentiviral genetic information to allow packaging of an RNA genome, in the presence of packaging components, into a viral particle which is capable of infecting a target cell, but is incapable of independent replication to produce infectious viral particles within the final target cell. Preferably the vector lacks a functional gag-pol and/or env gene and/or other genes essential for replication.

However, the plasmid vector used to produce the viral genome within a host cell/packaging cell will also include transcriptional regulatory control sequences operably linked to the lentiviral genome to direct transcription of the genome in a host cell/packaging cell. These regulatory sequences may be the natural sequences associated with the transcribed viral sequence (i.e. the 5' U3 region), or they may be a heterologous promoter, such as another viral promoter (e.g. the CMV promoter).

The vectors may be self-inactivating (SIN) vectors in which the viral enhancer and promoter sequences have been deleted. SIN vectors can be generated and transduce non-dividing cells in vivo with an efficacy similar to that of wild-type vectors. The transcriptional inactivation of the long terminal repeat (LTR) in the SIN provirus should prevent mobilisation by replication-competent virus. This should also enable the regulated expression of genes from internal promoters by eliminating any cis-acting effects of the LTR.

The vectors may be integration-defective. Integration defective lentiviral vectors (IDLVs) can be produced, for example, either by packaging the vector with catalytically inactive integrase (such as an HIV integrase bearing the D64V mutation in the catalytic site; Naldini, L. et al. (1996) Science 272: 263-7; Naldini, L. et al. (1996) Proc. Natl. Acad. Sci. USA 93: 11382-8; Leavitt, A.D. et al. (1996) J. Virol. 70: 721-8) or by modifying or deleting essential at sequences from the vector LTR (Nightingale, S.J. et al. (2006) Mol. Ther. 13: 1121-32), or by a combination of the above.

HIV-derived vectors for use in the present invention are not particularly limited in terms of HIV strain. Numerous examples of sequences of HIV strains may be found at the HIV Sequence Database (http://www.hiv.lanl.gov/content index). For example, a HIV-1-derived vector may be derived from any of the HIV-1 strains NL4-3, IIIB_LAI or HXB2_LAI (X4-tropic), or BAL (R5-tropic), or a chimaera thereof. A HIV-2-derived vector may be derived, for example, from the HIV-2 strain ROD.

As discussed elsewhere herein, cyclosporine analogues according to the invention may, in some embodiments bind to, inhibit and/or degrade CypA. Some viral vectors recruit CypA to assist with efficient infection (e.g., the efficient infection of HSCs) because it shields the capsid from restriction by another antiviral protein called TRIM5. Inhibition of CypA recruitment by the cyclosporine analogues consequently risks reducing the efficiency of transduction to suboptimal levels.

In preferred embodiments of the present invention, therefore, the following alternative approaches can be applied.

First, a viral vector that is not sensitive, or has limited sensitivity, to CypA can be utilised (e.g. a viral vector that does not bind to CypA). Non-limiting examples of such vectors include HIV capsid mutants that are insensitive to CypA and resistant to TRIM5 restriction such as A92E and G94D (see, for instance, Ylinen et al., Journal of Virology 83(4), 2009, p. 2044-2047).

Second, a cyclosporine analogue having reduced or eliminated binding to CypA (compared, for instance, to cyclosporine A, CsA) can be utilised. Such cyclosporine analogues may have, for instance, a higher IC50 than CsA (e.g., at least twice as high, more preferably at least three times as high) for binding to CypA under identical test conditions. A routine competitive binding assay can also be used to establish that a particular cyclosporine analogue has reduced binding to CypA compared with CsA.

### Transduction of haematopoietic stem and/or progenitor cells

In one aspect, the present invention provides the use of a cyclosporine analogue according to the invention for increasing the efficiency of transduction of an isolated population of human haematopoietic stem and/or progenitor cells by a vector derived from HIV-1, HIV-2, SIV, FIV, BIV, EIAV, CAEV or visna lentivirus.

Increasing the efficiency of transduction refers to an increase in the transduction of haematopoietic stem and/or progenitor cells in the presence of an agent (e.g. a cyclosporine analogue according to the invention), in comparison to the transduction achieved in the absence of the agent but under otherwise substantially identical conditions. An increased efficiency of transduction may therefore allow the multiplicity of infection (MOI) and/or the transduction time required to achieve effective transduction to be reduced.

In one embodiment, the percentage of haematopoietic stem and/or progenitor cells transduced by the vector is increased. In another embodiment, the vector copy number per cell is increased. Preferably both are achieved at the same time.

Methods for determining the percentage of cells transduced by a vector are known in the art. Suitable methods include flow cytometry, fluorescence-activated cell sorting (FACS) and fluorescence microscopy. The technique employed is preferably one which is amenable to automation and/or high throughput screening.

For example, a population of cells may be transduced with a vector which harbours a reporter gene. The vector may be constructed such that the reporter gene is expressed when the vector transduces a cell. Suitable reporter genes include genes encoding fluorescent proteins, for example green, yellow, cherry, cyan or orange fluorescent proteins. Once the population of cells has been transduced by the vector, both the number of cells expressing and not-expressing the reporter gene may be quantified using a suitable technique, such as FACS. The percentage of cells transduced by the vector may then be calculated.

Alternatively, quantitative PCR (qPCR) may be used to determine the percentage of cells transduced by a vector that does not harbour a reporter gene. For example, single colonies of CD34+ cells may be picked from a semi-solid culture and qPCR may be performed on each colony separately to determine the percentage of vector-positive colonies among those analysed.

Methods for determining vector copy number are also known in the art. The technique employed is preferably one which is amenable to automation and/or high throughput screening. Suitable techniques include quantitative PCR (qPCR) and Southern blot-based approaches.

The concentration at which a cyclosporine analogue according to the invention can be applied to a population of haematopoietic stem and/or progenitor cells may be adjusted for different vector systems to optimise transduction efficiency. Methods for determining transduction efficiency have been described above.

A cyclosporine analogue according to the invention may be toxic to haematopoietic stem and/or progenitor cells if it is applied at too high a concentration. The toxicity the cyclosporine analogue according to the invention on haematopoietic stem and/or progenitor cells may be determined by quantifying the number of viable cells remaining after exposure to the cyclosporine analogue for a certain time. Methods for quantifying the number of viable cells are known in the art. A skilled person may therefore select a suitable concentration of a cyclosporine analogue according to the invention to maximise increase in transduction efficiency while minimising the effect of toxicity using the approaches described herein.

For example, the concentration of the cyclosporine analogue applied to the population of haematopoietic stem and/or progenitor cells may be about 0.1-50 µM, about 1-50 µM, about 5-50 µM, about 10-50 µM, about 5-40 µM, about 10-40 µM, about 10-25 µM, or about 10-15 µM.

The present invention encompasses the use of a cyclosporine analogue according to the invention. The cyclosporine analogue of the present invention may be those which increase the efficiency of transduction of an isolated population of haematopoietic stem and/or progenitor cells by a vector derived from HIV-1, HIV-2, SIV, FIV, BIV, EIAV, CAEV or visna lentivirus.

Cyclosporine analogues according to the invention are preferably of low toxicity for mammals, in particular human, and preferably are of low toxicity for haematopoietic stem and/or progenitor cells.

### Therapeutic applications: gene therapy and transplantation

The vector used in the present invention preferably comprises a nucleotide of interest (NOI). Preferably the NOI gives rise to a therapeutic effect and therefore has utility in gene therapy. Suitable NOIs include, but are not limited to sequences encoding enzymes, cytokines, chemokines, hormones, antibodies, anti-oxidant molecules, engineered immunoglobulin-like molecules, single chain antibodies, fusion proteins, immune co-stimulatory molecules, immunomodulatory molecules, anti-sense RNA, microRNA, shRNA, siRNA, bozymes, miRNA target sequences, a transdomain negative mutant of a target protein, toxins, conditional toxins, antigens, tumour suppressor proteins, growth factors, transcription factors, membrane proteins, surface receptors, anti-cancer molecules, vasoactive proteins and peptides, anti-viral proteins and hbozymes, and derivatives thereof (such as derivatives with an associated reporter group). The NOIs may also encode pro-drug activating enzymes. An example of a NOI is the beta-globin chain which may be used for gene therapy of thalassemia/sickle cell disease.

NOIs may also include those useful for the treatment of other diseases requiring nonurgent/elective gene correction in the myeloid lineage such as: chronic granulomatous disease (CGD, e.g. the gp91 phox transgene), leukocyte adhesion defects, other phagocyte disorders in patients without ongoing severe infections and inherited bone marrow failure syndromes (e.g. Fanconi anaemia), as well as primary immunodeficiencies (SCIDs). NOIs may also include those useful in the treatment of lysosomal storage disorders and immunodeficiencies.

The present invention also provides a population of haematopoietic stem and/or progenitor cells prepared according to a method of the invention for use in therapy, for example for use in gene therapy. The use may be as part of a haematopoietic stem and/or progenitor cell transplantation procedure.

Haematopoietic stem cell transplantation (HSCT) is the transplantation of blood stem cells that may be derived from the bone marrow (in this case known as bone marrow transplantation) or blood. Stem cell transplantation is a medical procedure in the fields of haematology and oncology, most often performed for people with diseases of the blood or bone marrow, or certain types of cancer. Many recipients of HSCTs are multiple myeloma or leukaemia patients who would not benefit from prolonged treatment with, or are already resistant to, chemotherapy. Candidates for HSCTs include paediatric cases where the patient has an inborn defect such as severe combined immunodeficiency or congenital neutropenia with defective stem cells, and also children or adults with aplastic anaemia who have lost their stem cells after birth. Other conditions treated with stem cell transplants include sickle-cell disease, myelodysplastic syndrome, neuroblastoma, lymphoma, Ewing's Sarcoma, Desmoplastic small round cell tumour and Hodgkin's disease. More recently non-myeloablative, or so-called "mini transplant", procedures have been developed that require smaller doses of preparative chemotherapy and radiation. This has allowed HSCT to be conducted in the elderly and other patients who would otherwise be considered too weak to withstand a conventional treatment regimen.

In one embodiment, a population of haematopoietic stem and/or progenitor cells prepared according to a method of the invention is administered as part of an autologous stem cell transplant procedure.

In another embodiment, a population of haematopoietic stem and/or progenitor cells prepared according to a method of the invention is administered as part of an allogeneic stem cell transplant procedure.

By "autologous stem cell transplant procedure" it is to be understood that the starting population of cells (which are then transduced according to a method of the invention) is obtained from the same subject as that to which the transduced cell population is administered. Autologous transplant procedures are advantageous as they avoid problems associated with immunological incompatibility and are available to subjects irrespective of the availability of a genetically matched donor.

By "allogeneic stem cell transplant procedure" it is to be understood that the starting population of cells (which are then transduced according to a method of the invention) is obtained from a different subject as that to which the transduced cell population is administered. Preferably, the donor will be genetically matched to the subject to which the cells are administered to minimise the risk of immunological incompatibility.

Suitable doses of transduced cell populations are such as to be therapeutically and/or prophylactically effective. The dose to be administered may depend on the subject and condition to be treated, and may be readily determined by a skilled person.

The products, methods and uses of the present invention may be useful in the treatment of the disorders listed in WO 1998/005635. For ease of reference, part of that list is now provided: cancer, inflammation or inflammatory disease, dermatological disorders, fever, cardiovascular effects, haemorrhage, coagulation and acute phase response, cachexia, anorexia, acute infection, HIV infection, shock states, graft-versus-host reactions, autoimmune disease, reperfusion injury, meningitis, migraine and aspirin-dependent anti-thrombosis; tumour growth, invasion and spread, angiogenesis, metastases, malignant, ascites and malignant pleural effusion; cerebral ischaemia, ischaemic heart disease, osteoarthritis, rheumatoid arthritis, osteoporosis, asthma, multiple sclerosis, neurodegeneration, Alzheimer's disease, atherosclerosis, stroke, vasculitis, Crohn's disease and ulcerative colitis; periodontitis, gingivitis; psoriasis, atopic dermatitis, chronic ulcers, epidermolysis bullosa; corneal ulceration, retinopathy and surgical wound healing; rhinitis, allergic conjunctivitis, eczema, anaphylaxis; restenosis, congestive heart failure, endometriosis, atherosclerosis or endosclerosis.

In addition, or in the alternative, the products, methods and uses of the present invention may be useful in the treatment of the disorders listed in WO 1998/007859. For ease of reference, part of that list is now provided: cytokine and cell proliferation/differentiation activity; immunosuppressant or immunostimulant activity (e.g. for treating immune deficiency, including infection with human immune deficiency virus; regulation of lymphocyte growth; treating cancer and many autoimmune diseases, and to prevent transplant rejection or induce tumour immunity); regulation of haematopoiesis, e.g. treatment of myeloid or lymphoid diseases; promoting growth of bone, cartilage, tendon, ligament and nerve tissue, e.g. for healing wounds, treatment of burns, ulcers and periodontal disease and neurodegeneration; inhibition or activation of follicle-stimulating hormone (modulation of fertility); chemotactic/chemokinetic activity (e.g. for mobilising specific cell types to sites of injury or infection); haemostatic and thrombolytic activity (e.g. for treating haemophilia and stroke); anti-inflammatory activity (for treating e.g. septic shock or Crohn's disease); as antimicrobials; modulators of e.g. metabolism or behaviour; as analgesics; treating specific deficiency disorders; in treatment of e.g. psoriasis, in human or veterinary medicine.

In addition, or in the alternative, the products, methods and uses of the present invention may be useful in the treatment of the disorders listed in WO 1998/009985. For ease of reference, part of that list is now provided: macrophage inhibitory and/or T cell inhibitory activity and thus, anti-inflammatory activity; anti-immune activity, i.e. inhibitory effects against a cellular and/or humoral immune response, including a response not associated with inflammation; inhibit the ability of macrophages and T cells to adhere to extracellular matrix components and fibronectin, as well as up-regulated fas receptor expression in T cells; inhibit unwanted immune reaction and inflammation including arthritis, including rheumatoid arthritis, inflammation associated with hypersensitivity, allergic reactions, asthma, systemic lupus erythematosus, collagen diseases and other autoimmune diseases, inflammation associated with atherosclerosis, arteriosclerosis, atherosclerotic heart disease, reperfusion injury, cardiac arrest, myocardial infarction, vascular inflammatory disorders, respiratory distress syndrome or other cardiopulmonary diseases, inflammation associated with peptic ulcer, ulcerative colitis and other diseases of the gastrointestinal tract, hepatic fibrosis, liver cirrhosis or other hepatic diseases, thyroiditis or other glandular diseases, glomerulonephritis or other renal and urologic diseases, otitis or other oto-rhino-laryngological diseases, dermatitis or other dermal diseases, periodontal diseases or other dental diseases, orchitis or epididimo-orchitis, infertility, orchidal trauma or other immune-related testicular diseases, placental dysfunction, placental insufficiency, habitual abortion, eclampsia, pre-eclampsia and other immune and/or inflammatory-related gynaecological diseases, posterior uveitis, intermediate uveitis, anterior uveitis, conjunctivitis, chorioretinitis, uveoretinitis, optic neuritis, intraocular inflammation, e.g. retinitis or cystoid macular oedema, sympathetic ophthalmia, scleritis, retinitis pigmentosa, immune and inflammatory components of degenerative fondus disease, inflammatory components of ocular trauma, ocular inflammation caused by infection, proliferative vitreo-retinopathies, acute ischaemic optic neuropathy, excessive scarring, e.g. following glaucoma filtration operation, immune and/or inflammation reaction against ocular implants and other immune and inflammatory-related ophthalmic diseases, inflammation associated with autoimmune diseases or conditions or disorders where, both in the central nervous system (CNS) or in any other organ, immune and/or inflammation suppression would be beneficial, Parkinson's disease, complication and/or side effects from treatment of Parkinson's disease, AIDS-related dementia complex HIV-related encephalopathy, Devic's disease, Sydenham chorea, Alzheimer's disease and other degenerative diseases, conditions or disorders of the CNS, inflammatory components of stokes, post-polio syndrome, immune and inflammatory components of psychiatric disorders, myelitis, encephalitis, subacute sclerosing panencephalitis, encephalomyelitis, acute neuropathy, subacute neuropathy, chronic neuropathy, Guillaim-Barre syndrome, Sydenham chora, myasthenia gravis, pseudo-tumour cerebri, Down's Syndrome, Huntington's disease, amyotrophic lateral sclerosis, inflammatory components of CNS compression or CNS trauma or infections of the CNS, inflammatory components of muscular atrophies and dystrophies, and immune and inflammatory related diseases, conditions or disorders of the central and peripheral nervous systems, post-traumatic inflammation, septic shock, infectious diseases, inflammatory complications or side effects of surgery, bone marrow transplantation or other transplantation complications and/or side effects, inflammatory and/or immune complications and side effects of gene therapy, e.g. due to infection with a viral carrier, or inflammation associated with AIDS, to suppress or inhibit a humoral and/or cellular immune response, to treat or ameliorate monocyte or leukocyte proliferative diseases, e.g. leukaemia, by reducing the amount of monocytes or lymphocytes, for the prevention and/or treatment of graft rejection in cases of transplantation of natural or artificial cells, tissue and organs such as cornea, bone marrow, organs, lenses, pacemakers, natural or artificial skin tissue.

In addition, or in the alternative, the products, methods and uses of the present invention may be useful in the treatment of β-thalassemia, chronic granulomatous disease, metachromatic leukodystrophy, mucopolysaccharidoses disorders and other lysosomal storage disorders.

Gene therapy may occur through the sustained or transient release of product encoded by the NOI, for example an encoded product set out above. For example, haematopoietic progenitor cells generally provide short term engraftment. Accordingly, gene therapy by administering transduced haematopoietic progenitor cells may provide a non-permanent effect in the subject. For example, the effect may be limited to 1-6 months following administration of the transduced haematopoietic progenitor cells. An advantage of this approach would be better safety and tolerability, due to the self-limited nature of the therapeutic intervention. Such haematopoietic progenitor cell gene therapy may be suited to treatment of acquired disorders, for example cancer, where time-limited expression of a (potentially toxic) anti-cancer nucleotide of interest may be sufficient to eradicate the disease. In contrast, HSCs may be more likely to provide long term engraftment, and therefore may be better suited to providing a longer term effect in the subject or an effect sustained throughout the lifetime of the subject. For example, the effect may be limited to 3 months to 30 years following administration of the transduced HSCs. A longer term or sustained effect may be suited to treatment of inherited genetic disorders, for example SCID, where long term expression of a nucleotide of interest may be desirable.

Following gene therapy, the encoded product may be released systemically in the subject, for example into the circulation. Systemic release may result in a 1.1, 1.2, 1.5, 2, 5, 10, 25, 50, 100, 250, 500 or 1000 fold increase in encoded product activity relative to the activity before gene therapy. Assays for measuring encoded product activity would be apparent to the skilled person.

Alternatively, following gene therapy, the encoded product may be released in a targeted fashion such that it is targeted to a specific group of tissue and/or organ. For example, the encoded product may be targeted to the central nervous system (CNS), heart, face, mouth, eye, bone, liver, spleen and/or lung. Targeted release may result in a 1.1, 1.2, 1.5, 2, 5, 10, 25, 50, 100, 250, 500 or 1000 fold increase in encoded product activity in the targeted tissue and/or organ relative to the activity in the same tissue and/or organ before gene therapy. In addition, or in the alternative, targeted release may result in a 1.1, 1.2, 1.5, 2, 5, 10, 25, 50, 100, 250, 500 or 1000 fold increase in encoded product activity in the targeted tissue and/or organ after gene therapy, relative to the activity in non-targeted tissues and/or organs. Assays for measuring encoded product activity would be apparent to the skilled person.

Thus, the invention provides means whereby pathological phenotypes associated with the indications provided above can be corrected, treated, arrested, palliated and/or prevented. Correction can refer to both partial, total correction and hyper-correction. Correction may be achieved after about 10 days, 20 days, 30 days, 40 days, 50 days, 60 days, 70 days, 80 days, 90 days, 100 days, 125 days, 150 days, 175 days, 200 days, 250 days, 300 days, 1 year, 1.5 years, 2 years, 2.5 year, 3 years, 4 year or 5 years. The effect of correcting, treating, arresting, palliating and/or preventing a phenotype may be transient. Alternatively, the effect of correcting, treating, arresting, palliating and/or preventing a phenotype may be long term, or sustained.

The treatment of mammals, particularly humans, is preferred. However, both human and veterinary treatments may be within the scope of the present invention.

### Kit

In another aspect, the present invention provides a kit comprising the cyclosporine analogue and/or cell populations of the invention. The cyclosporine analogue and/or cell populations of the invention may be provided in suitable containers. The kit may also include instructions for use.

### Treatment of viral infections

Cyclosporine A, CsA, has previously been disclosed as having antiviral activity, including against coronaviruses (see, e.g.: Carbajo-Lozoya et al., Virus Res. 2014 184:44-53; Nasiri et al., J Dermatolog Treat. 2020:1-6; de Wilde et al., Virus Res. 2017 15;228:7-13).

Compounds (i.e., the analogues) of the present invention can also be useful in the treatment of viral infections, for instance via their ability to bind to, inhibit and/or degrade CypA.

The compounds of the invention are useful in the treatment or prevention of a viral infection in a patient. Typically, the patient is a mammal, such as a human or a cat, preferably a human.

Typically, the viral infection is human immunodeficiency virus-1 (HIV-1), influenza virus, human cytomegalovirus (hCMV), hepatitis C virus (HCV), dengue virus, a vaccinia virus (such as Small Pox), feline immunodeficiency virus (FIV) or a corona virus (such as COVID-19 or SARs). Preferably, the viral infection is COVID-19, human immunodeficiency virus-1 (HIV-1), influenza virus, human cytomegalovirus (hCMV) or hepatitis C virus (HCV), more preferably COVID-19 or human immunodeficiency virus-1 (HIV-1) and more preferably still COVID-19. COVID-19 (i.e., coronavirus disease 2019 is the disease caused by severe acute respiratory syndrome coronavirus 2 (SARS-CoV-2).

The compounds of the invention may be administered to humans in various manners such as oral, rectal, vaginal, parenteral, intramuscular, intraperitoneal, intraarterial, intrathecal, intrabronchial, subcutaneous, intradermal, intravenous, nasal, buccal or sublingual routes of administration. The particular mode of administration and dosage regimen will be selected by the attending physician, taking into account a number of factors including the age, weight and condition of the patient.

The compound is typically administered as a pharmaceutical composition, which generally comprises a derivative of the invention and a pharmaceutically acceptable excipient, diluent or carrier. Thus, pharmaceutical compositions that contain the compounds of the invention will normally be formulated with an appropriate pharmaceutically acceptable excipient, carrier or diluent depending upon the particular mode of administration being used. For instance, parenteral formulations are usually injectable fluids that use pharmaceutically and physiologically acceptable fluids such as physiological saline, balanced salt solutions, or the like as a vehicle. Oral formulations, on the other hand, may be solids, e.g. tablets or capsules, or liquid solutions or suspensions.

Compositions may be formulated in unit dosage form, i.e., in the form of discrete portions containing a unit dose, or a multiple or sub-unit of a unit dose.

The amount of the compound of the invention that is given to a patient will depend upon on the activity of the particular compound in question. Further factors include the condition being treated, the nature of the patient under treatment and the severity of the condition under treatment. The timing of administration of the compound should be determined by medical personnel. As a skilled physician will appreciate, and as with any drug, the compound may be toxic at very high doses. For example, the compound may be administered at a dose of from 0.01 to 30 mg/kg body weight, such as from 0.1 to 10 mg/kg, more preferably from 0.1 to 5 mg/kg body weight.

The compounds of the invention may be given alone or in combination with one or more additional anti-viral agents, preferably one or more agents useful for treating human immunodeficiency virus-1 (HIV-1), influenza virus, human cytomegalovirus (hCMV), hepatitis C virus (HCV), dengue virus, vaccinia virus, feline immunodeficiency virus (FIV) or corona virus.

One such anti-viral agent is Remdesivir.

Anti-viral agents useful for treating HIV-1 include non-nucleoside reverse transcriptase inhibitors (NNRTIs), nucleoside analogue reverse transcriptase inhibitor (NRTIs) and nucleotide analog reverse-transcriptase inhibitors (NtRTIs). Preferred NRTIs include Zidovudine, Didanosine, Zalcitabine, Stavudine, Lamivudine, Abacavir, Emtricitabine, Entecavir and Apricitabine, Preferred NNRTIs include Efavirenz, Nevirapine, Delavirdine, Etravirine and Rilpivirine. Preferred NtRTIs include Tenofovir and Adefovir.

Anti-viral agents useful for treating influenza virus include (a) neuraminidase inhibitors such as oseltamivir and zanamivir, and (b) M2 protein inhibitors such as amantadine and rimantadine.

Anti-viral agents useful for treating human cytomegalovirus (hCMV) include human cytomegalovirus antibodies and antiviral agents such as Ganciclovir, Valganciclovir, Foscarnet and cidofovir.

Anti-viral agents useful for treating hepatitis C virus (HCV) include pegylated interferon alpha and ribavirin.

Anti-viral agents useful for treating vaccinia virus include cidofovir.

Anti-viral agents useful for treating feline immunodeficiency virus (FIV) include Lymphocyte T-Cell Immunomodulator.

The active ingredients are typically administered as a combined preparation.

Accordingly, the present invention also provides a combination comprising a c of the invention and one or more said additional anti-viral agents. The combination is typically for use in the treatment or prevention of said viral infection in a patient.

The invention further provides a compound of the invention for use in the treatment or prevention of a viral infection in a patient, by co-administration with one or more said additional anti-viral agents. Co-administration can be simultaneous, concurrent, separate or sequential.

The invention further provides one or more additional said anti-viral agents, for use in the treatment or prevention of a said viral infection in a patient, by co-administration with a compound of the invention. Co-administration can be simultaneous, concurrent, separate or sequential.

The present invention further provides a product comprising a compound of the invention and one or more said additional anti-viral agents, as a combined preparation for simultaneous, concurrent, separate or sequential use in the treatment or prevention of a said viral infection in a patient.

### EXAMPLES

### Example 1 - Antiviral activity of the cyclosporine analogues

### Introduction

Viruses encounter a remarkable array of intracellular antiviral defences that they must suppress or evade in order to replicate. The cyclophilin (Cyp) family of host proteins have emerged as key players at the virus-host interface. Cyclophilin A (CypA) is a cofactor for a variety of established and emerging viruses, including *Flaviviridae* such as hepatitis C virus (HCV) (Yang et al., 2008) and dengue virus (Qing et al., 2009), as well as *Coronaviridae* such as SARS coronavirus (Pfefferle et al., 2011). Like other Cyps, CypA has peptidyl prolyl isomerase activity, which is thought to induce conformational changes in bound target proteins (Wang & Heitman, 2005). Importantly, recruitment of CypA also affects protein complex formation (Liu et al., 1991). The role of CypA as a viral cofactor is best understood for human immunodeficiency virus (HIV-1), where CypA binds to the viral capsid (Luban et al., 1993; Thali et al., 1994) to regulate interactions with downstream cofactors and protect the capsid and encapsidated viral genome from cellular innate immune sensors (Rasaiyaah et al., 2013; Schaller et al., 2011; Kim et al., 2019). However, the mechanisms by which CypA contributes to other viral infections are less well understood.

Cyps have been implicated in the regulation of viral innate immune evasion (Rasaiyaah et al., 2013) and innate immune signalling (Sun et al., 2014; Liu et al., 2017; Obata et al., 2005). In the case of HCV, clinical trials demonstrated that pharmacological inhibition of CypA suppressed HCV replication and led to elevated type 1 interferon (IFN) in patients (Hopkins et al., 2012). Given the links between CypA and HCV innate immune evasion, we sought to understand the potential roles of CypA in viral innate immune evasion using HCV as a model. Both CypA binding and resistance to cyclophilin inhibitors (CypI) map to the HCV NS5A protein (Hanoulle et al., 2009; Yang et al., 2010), which has essential roles in HCV replication and assembly (Ross-Thriepland & Harris, 2015) but crucially also contributes to immune evasion by several key mechanisms. For example, NS5A is necessary for formation of the membranous replication organelle (RO) (Romero-Brey et al., 2012) that cloaks viral RNA replication from cytosolic pattern recognition receptors (Neufeldt et al., 2016), preventing innate immune activation. Notably, CypA plays a role in the formation of the RO (Madan et al., 2014; Chatterji et al., 2015). NS5A also inhibits activation of the key antiviral effector protein kinase R (PKR) (Gale et al., 1997) and subsequent PKR-dependent activation of interferon regulatory factor-1 (IRF1)-driven antiviral responses (Pflugheber et al., 2002).

Here we have used a panel of novel CypI alongside genetics approaches to discover that CypA regulates HCV evasion of PKR and IRF1 antiviral responses, and that diverse CypI overcome this evasion strategy leading to suppression of virus replication. Our findings advance understanding of CypA-HCV interactions and PKR mechanisms, and open perspectives for the development of novel CypA-targeted therapies that harness host intrinsic antiviral responses to combat infection.

### Results

### CypA is critical for HCV replication in Huh7 cells, but not in Huh7.5 cells

To characterise the role of CypA in HCV innate immune evasion, we took advantage of the human hepatoma cell line Huh7 and its derivative Huh7.5. Huh7.5 cells were selected for enhanced ability to support HCV replication (Blight et al., 2002) and spread (Koutsoudakis et al., 2007), and also have defective innate immunity (Sumpter et al., 2005). We silenced CypA and CypB expression in Huh7 and Huh7.5 cells by stably expressing specific shRNAs **(****Figure 1A-B)** and subsequently evaluated HCV replication using the subgenomic replicon (SGR) model. Silencing of CypB expression inhibited HCV replication by ~100-fold in both cell lines **(****Figure 1C****),** consistent with its previously described role in viral RNA replication (Watashi et al., 2005). Intriguingly, silencing of CypA abrogated HCV replication in Huh7 cells but had minimal effect in Huh7.5 cells **(****Figure 1C****).** We observed the same inhibition profile when the data was normalised to the input luciferase signal at 4 hours post-electroporation (hpe) **(****Figure 2****),** confirming that this observation was not due to differences in electroporation efficiency between cell lines. The differential effect of CypA depletion was mirrored by treatment with the classical CypI CsA. CsA abrogated HCV replication in Huh7 cells, but only partially inhibited replication in Huh7.5 cells **(****Figure 1D****).** CsA also inhibited HCV replication in Huh7.5 cells silenced for CypA expression **(****Figure 1E****),** with similar antiviral potency regardless of CypA expression **(****Figure 3****).** This suggests that the observed inhibition in Huh7.5 cells may result from inhibition of CypB, which is also a CsA target (Davis et al., 2010). This observation explains the controversy of whether CypA or CypB are HCV cofactors, by demonstrating that both have roles, and suggests differential cofactor requirements between Huh7 and more permissive Huh7.5 cells.

We validated the differential cyclophilin dependence in Huh7 and Huh7.5 cells using the HCVcc (J6/JFH1-Rluc) infection model. Silencing of CypA expression, or addition of CsA, completely inhibited HCVcc infection in Huh7 cells, but only weakly inhibited infection in Huh7.5 cells **(****Figure 1F-G),** which may reflect a role for CypA in HCV assembly (Nag et al., 2012). To further probe this differential requirement for CypA, we evaluated replication of the CypA-independent NS5A D316E/Y317N (DEYN) mutant (Yang et al., 2010), which was selected by CypI treatment in highly permissive HCV replicating cell lines. HCV DEYN replicated at wild type levels in Huh7.5 cells, but exhibited a 5-fold replication defect in innate immune competent Huh7 cells **(****Figure 1H****).** The replication defect in Huh7 cells was partially rescued by addition of CsA **(****Figure 11****).** This observation mirrors similar observations for HIV-1 mutants selected to replicate in the presence of CsA in that they too become somewhat dependent on CsA for maximal replication (Ylinen et al., 2009). Together, these results confirm that CypA is crucial for HCV replication in innate sensing competent Huh7 cells, but significantly less important for replication in more permissive Huh7.5 cells.

### Structurally distinct CypI are more potent against HCV replication in Huh7 cells than in Huh7.5 cells

CsA binds to the cyclophilin active site and forms a ternary inhibitory complex with phosphatase calcineurin, which inhibits T cell proliferation leading to immunosuppression (Liu et al., 1991). To confirm that the phenotype we observed with CsA treatment **(****Figure 1****)** was due to cyclophilin binding, and not off-target complex formation, and to generate chemical probes suitable for further mechanistic analyses, we synthesized a panel of novel CypI with distinct structures acting by distinct mechanisms. These included novel CsA analogues and synthetic small molecules based on sanglifehrin chemistry termed depsins **(****Figure 4A****).** The synthesis and favourable pharmacokinetic properties of depsin molecules were recently described (Mackman et al., 2018). Importantly, depsin molecules do not cause immunosuppression via the calcineurin pathway (Zenke et al., 2001). We also designed and synthesized a CsA-derived proteolysis targeting chimera (PROTAC; CsA-Prtc1) **(****Figure 4A** **- the compound "CsA-like" is a representative cyclosporine analogue of the presently claimed invention; the other compounds shown are reference compounds),** which is expected to recruit the von Hippel-Lindau E3 ubiquitin ligase to CsA targets, leading to their proteasomal degradation.

In total, we evaluated ~80 novel CypI for their inhibitory effects on HCV replication in Huh7 cells. We selected the most potent antiviral, and least cytotoxic, molecule from each CypI type for further characterisation **(****Figure 5****).** Using a fluorescence polarisation assay, in which fluorescent CsA is competed from purified recombinant CypA with unlabelled CypI (Warne et al., 2016), we first confirmed that the selected molecules bound to CypA with similar nanomolar affinities **(****Figure 4B****).** CsA-Prtc1 treatment led to degradation of CypA in Huh7 and Huh7.5 cells **(****Figure 4C****)** within a matter of hours **(****Figure 6A****)** in a dose-dependent manner **(Figure 5B),** with complete loss of detectable CypA protein expression occurring within 24 hours at concentrations as low as 100 nM. Degradation was proteasome-dependent **(****Figure 4F****)** with selectivity for CypA **(****Figure 4G-H),** with minimal impact on CypB or CypD protein expression after 24 hours in Huh7 cells, although CypB degradation was observed after 48 hours incubation **(****Figure 6C****).** Moreover, CsA treatment protected CypA from CsA-Prtc1-mediated degradation **(Figure 5I),** illustrating CypA recruitment of CsA within cells.

We next compared the effect of our selected CypI on HCV replication and infection in Huh7 and Huh7.5 cells. Like CsA, our three novel and structurally distinct CypI abrogated HCV replication **(****Figure 7A****)** and HCVcc infection **(****Figure 7B****)** in Huh7 cells, but only partially inhibited replication and infection in Huh7.5 cells at the tested dose **(****Figure 7A-B**). The CsA-Prtc1 was extremely potent and completely inhibited HCVcc infection in both cell lines at the single dose tested **(****Figure 7B****).** However, dose-response analyses showed that the CypI were similarly 5- to 10-fold more potent against HCV replication in Huh7 cells compared to Huh7.5 cells **(****Figure 7C****),** with low nanomolar IC₅₀ in Huh7 cells **(Table 1).** The selected CypI were similarly more potent against HCVcc infection of Huh7 cells compared to Huh7.5 cells **(****Figure 7D****, Table 1).** Concordantly, while replication of the NS5A DEYN mutant was resistant to CypI treatment in Huh7.5 cells, CypI treatment dose-dependently enhanced DEYN replication in Huh7 cells **(****Figure 8****),** again illustrating the differential requirements for CypA in the two cell lines during HCV replication.

### Induction of cellular antiviral responses in Huh7 cells, but not in Huh7.5 cells, contributes to the antiviral potency of CypI against HCV

Given that all of the CypI inhibited HCV more potently in innate immune competent Huh7 cells, we hypothesized that the increased potency might be due to induction of effective innate antiviral responses in parental Huh7 that were lacking in the more permissive Huh7.5 cells. To test this, we evaluated expression of IFN-β mRNA by qPCR after treating HCV-replicating or HCVcc-infected Huh7 or Huh7.5 cells with CsA. Consistent with our hypothesis, we observed induction of IFN-β expression in Huh7 cells but not in more permissive Huh7.5 cells **(****Figure 9A-B).** Intriguingly, CsA-like molecules and structurally unrelated depsins, but not CsA-Prtc1 (which degrades CypA rather than simply binding CypA and affecting complex formation), were capable of inducing IFN-β expression in HCV-replicating Huh7 cells **(****Figure 9C****).** One possibility is that activation of IFN-β expression requires the presence of CypA, which is consistent with previous studies suggesting CypA is a necessary co-factor for innate immune sensors, including RIG-I (Liu et al., 2017) and PKR (Daito et al., 2014).

We confirmed that induction of antiviral responses by CypI in Huh7 cells was specifically the result of HCV replication (rather than transfection of RNA into the cytoplasm) by comparison to a replication-defective replicon with a mutation in the polymerase active site (Schaller et al., 2007). Electroporation with either wild-type or replication-defective replicon RNA similarly resulted in translation of luciferase from the input RNA **(****Figure 10A****)** and induction of IFN-β expression at 4 hours post electroporation (hpe) **(****Figure 10B****),** reflecting initial transfection of RNA into the cytoplasm. By 24-48 hpe, however, no luciferase activity or IFN-β mRNA expression above the background level could be detected in cells electroporated with the replication-defective replicon **(****Figure 10A-B).** Furthermore, the induction of IFN-β expression by CsA at 48 and 72 hpe was only observed in cells electroporated with the wild-type replicon **(****Figure 10C-F).**

We next evaluated whether the CypI antiviral potency was dependent on increased IFN-β signalling. Addition of exogenous IFN-β inhibited HCV replication in Huh7 cells **(****Figure 9C****),** confirming that Huh7 cells are capable of responding to IFN. Although pharmacological inhibition of the Jak/STAT pathway by ruxolitinib treatment rescued HCV replication from inhibition by IFN-β **(****Figure 9D****),** ruxolitinib treatment had no effect on viral replication in the absence of exogenous IFN-β. Furthermore, ruxolitinib did not affect the potency of CsA **(****Figure 9E****)** or other CypI **(****Figure 11****)** against HCV replication in Huh7 cells, suggesting that the antiviral effect is independent of IFN signalling. The notion that IFN was not required for maximal CypI activity was also supported by an experiment using anti-human interferon alpha/beta receptor chain 2 antibody (IFNAR) to inhibit IFN activity through receptor blockade. Anti-IFNAR treatment did not affect the potency of CsA against HCV but effectively rescued HCV replication from inhibition by IFN-β in a control experiment. **(****Figure 12A-B**). The lack of a requirement for IFN in the effect of CypI is likely explained by direct induction of antiviral genes with inhibitory activity against HCV, here exemplified by viperin (*RSAD2*) (Helbig et al., 2011; Wang et al., 2012), in Huh7 but not Huh7.5 cells **(****Figure 9F****,** **Figure 13****).** Thus, we propose that in these experiments, CypI induces IFN-independent, cell-intrinsic antiviral immunity. However, *in vivo,* IFN induction would be expected to influence HCV replication and adaptive immune responses and thus the antiviral activity of CypI in patients.

### CypI disrupt formation of the HCV replication organelle

We next sought to identify the mechanisms underlying the observed activation of antiviral genes. CypA has previously been implicated in formation of the HCV RO. Silencing of CypA expression by RNAi (Chatterji et al., 2015) or treatment with a CsA analogue (cyclosporine D) (Madan et al., 2014) inhibited formation of the double membrane vesicles (DMVs) that comprise the HCV RO, thus inhibiting HCV replication. We hypothesised a model in which "uncloaking" of viral RNA, aided by disruption of the RO, and subsequent sensing of exposed cytosolic viral RNA, leads to IFN production in Huh7 cells. To test this model, we first evaluated whether our novel CypI inhibited formation of HCV-induced DMVs. As described previously, we used an NS3-5B expression construct to specifically evaluate DMV formation independently of viral RNA replication (Madan et al., 2014; Romero-Brey et al., 2012). Huh7-Lunet/T7 cells were transfected with the pTM-NS3-5B expression construct and treated with CypI 4 hours later. At 24 hours post-transfection, we evaluated NS5A expression and DMV formation. CypI treatment did not affect expression of NS5A as measured by immunofluorescence **(****Figure 14A****)** and Western blot **(****Figure 14B****).** However, treatment with CsA, depsin or CsA-Prtc1 caused a significant reduction in the number and size of DMVs observed by transmission electron microscopy (TEM) in transfected cells **(****Figure 14C-E),** suggesting incomplete and impaired formation of the RO.

### RIG-I-like receptors and MAVS do not contribute to the antiviral potency of CypI

Given the proposed role of the RO in viral innate immune evasion, RO disruption may plausibly increase exposure of replicating viral RNA to innate immune sensors. We therefore sought to determine which sensors might contribute to detection of viral RNA in the presence of CypI. Huh7 cells are capable of responding to cytosolic RNA and initiating antiviral signalling through RIG-I, MAVS and IRF3 (Sumpter et al., 2005; Binder et al., 2007). However, Huh7.5 cells are less responsive, which may reflect a defect in RIG-I (Sumpter et al., 2005). Therefore, we hypothesized that the active RIG-I pathway in Huh7 cells contributes to the antiviral signalling induced by CypI. We first evaluated the RIG-I-like receptors (RLRs), RIG-I and Mda5. We tested the activity of CypI against HCV replication in Huh7.5 cells stably expressing functional RIG-I, Mda5, or both RIG-I and Mda5. These reconstituted cell lines have been described previously and have restored RNA sensing of HCV (Hiet et al., 2015). However, stable expression of RIG-I or Mda5 (or both) had little, if any, effect on HCV replication at 48 h **(****Figure 15A****)** and, more importantly, did not affect the antiviral potency of the CypI in Huh7.5 cells **(****Figure 15B****).** Similarly, transient transfection of RIG-I into Huh7.5 cells **(****Figure 16A****)** did not affect HCV replication **(****Figure 16B****),** consistent with previous findings (Binder et al., 2007), or CypI potency **(****Figure 16C****).**

Since MAVS is a key adaptor protein downstream of RIG-I and Mda5, we confirmed this observation in a loss-of-function context in Huh7 cells by generating clonal Huh7 MAVS knockout (KO) cell lines by CRISPR/Cas9 **(****Figure 15C****).** We evaluated HCV replication and CypI potency in MAVS KO Huh7 cells compared to control cells generated in the same manner with a non-targeting guide RNA. The loss of MAVS did not affect HCV replication evaluated compared to control cells **(****Figure 15D****).** However, exogenous expression of WT or HCV protease-resistant MAVS mutant C508R (Li et al., 2005) into MAVS KO Huh7 cells decreased viral replication (Figure 6E), likely due to induction of interferon responses (Bender et al., 2015). This is consistent with the importance of MAVS and its cleavage by HCV protease in HCV replication. Crucially, the antiviral potency of CypI was unaffected by the absence of MAVS **(****Figure 15F****),** and transfection of the C508R protease-resistant MAVS into MAVS KO Huh7 cells also had no effect on CypI activity **(****Figure 15F****).** Furthermore, CypI still induced expression of IFN-β mRNA in HCV-replicating MAVS KO Huh7 cells **(****Figure 15G****),** indicating that CypI induction of IFN-β expression is not dependant on the RLR/MAVS pathway. It is worth noting that treatment with daclatasvir, an inhibitor that similarly blocks formation of the HCV RO (Berger et al., 2014) and targets domain I of NS5A (Gao et al., 2010), inhibits HCV replication without inducing IFN-β expression **(****Figure 17A-B).** Therefore, sensing of viral RNA in the presence of CypI is likely not the result of simply "uncloaking" by disruption of the RO but rather through a more complex CypA-dependent mechanism. Notably, NS5A domain II (where CypA binds) is dispensable for RO formation (Romero-Brey et al., 2015) but is required to suppress IFN (Hiet et al., 2015) and control PKR (Gale et al., 1998).

### PKR modulates the antiviral potency of CypI against HCV

Given the documented role of the CypA target NS5A in binding and inhibiting PKR (Gale et al., 1997) and the proposed role of CypA in regulating PKR activity (Daito et al., 2014), we next evaluated a role for PKR in determining CypI potency against HCV. We first compared PKR expression and activation in HCV replicating Huh7 and Huh7.5 cells by Western blot **(****Figure 18A-B).** Importantly, PKR was more abundant in Huh7 cells, and, strikingly PKR T446 autophosphorylation (a marker of PKR activation) was observed in Huh7 but decreased in Huh7.5 cells **(****Figure 18A****).** We hypothesised that PKR may be impaired in sensing HCV RNA in Huh7.5 cells relative to the parental Huh7 cells. To test this hypothesis, we generated Huh7 PKR KO cell lines by CRISPR/Cas9 **(****Figure 18C****)** and evaluated the sensitivity of HCV to CypI inhibition in these cells. The antiviral potency of the CypI against HCV replication and HCVcc infection was markedly decreased **(****Figure 18D-E**). These data are consistent with PKR contributing to CypI potency and confirm a role for PKR in the control of HCV replication. Importantly, CsA treatment failed to induce IFN-β mRNA expression in HCV-replicating PKR KO Huh7 cells **(****Figure 18F****),** further supporting the model in which induction of IFN, and anti-HCV restriction factors, is mediated through PKR in the presence of CypI. To confirm the role of PKR activity, we next tested CypI potency in Huh7 and Huh7.5 cells in the presence of the PKR inhibitor C16, which prevents PKR activation (Jammi et al., 2003). C16 decreased CsA potency in Huh7 cells, but not in Huh7 PKR KO cells **(****Figure 18G****),** and only minimally affected CsA potency against HCV in Huh7.5 cells **(****Figure 19****).**

We also sought to confirm the role of PKR in CypI activity by over-expressing PKR in Huh7 PKR knockout cells. However, PKR over-expression in itself led to PKR activation, as evidenced by its autophosphorylation **(****Figure 20A****).** Concordantly, PKR over-expression had antiviral activity **(****Figure 20B****)** but this did not impact CsA sensitivity **(****Figure 20C****),** presumably because the PKR antiviral effect in this experiment is mediated through activation by phosphorylation and suppression of translation. This is consistent with previous observations of translation shutdown on PKR over-expression (Grolleau et al., 2000; Barber et al., 1993; Chong et al., 1992; Thomis & Samuel, 1992).

We next tested whether the absence of PKR broadly affects the sensitivity of HCV to the antiviral activity of telaprevir (NS3/4A protease inhibitor) and daclatasvir (NS5A inhibitor) in Huh7 and Huh7 PKR KO cells. Unlike Cyp inhibition **(****Figure 18D-E**), the absence of PKR did not affect the inhibitory activity of telaprevir or daclatasvir **(****Figure 21****).** Collectively, these data are consistent with a specific role for PKR in the enhanced antiviral activity of CypI in Huh7 cells.

### CypI treatment induces PKR- and IRF1-dependent cell-intrinsic antiviral responses

The most well characterised function of PKR is inhibition of RNA translation, which requires PKR autophosphorylation to activate its kinase activity leading to eIF2α phosphorylation. CypI have previously been shown to inhibit PKR autophosphorylation, thus preventing eIF2α phosphorylation by PKR (Daito et al., 2014; Bobardt et al., 2014), which was proposed to restore expression of ISGs at the protein level to contribute to the antiviral effect (Daito et al., 2014). Consistently, we observed that our CypI inhibited PKR autophosphorylation in HCV-replicating cells **(Figure 23A)** while only minimally affecting PKR expression based on densitometry analysis **(Figure 23B-C).** However, our observation that CypI treatment induces PKR-dependent expression of IFN-β mRNA in HCV-replicating or HCV-infected cells **(****Figure 9A-C,** **Figure 18F****)** suggests the involvement of additional transcriptional mechanisms.

PKR activates inflammatory transcription factors NF-κB and IRF1 directly and this is thought to be independent of its kinase activity, at least in the case of NF-κB activation (Bonnet et al., 2000; Bonnet et al., 2006). Interestingly, CsA predominantly induced expression of IRF1 target genes, and not canonical NF-κB targets (Yamane et al., 2019) **(****Figure 9F****),** and this induction was PKR-dependent in both HCV-replicating **(****Figure 22A****)** and HCVcc-infected cells **(****Figure 22B****).** Thus, restriction of HCV replication by CypI depends on the PKR-mediated induction of antiviral gene expression, including IRF1 targets. Notably, IRF1 and several IRF1 target genes have been shown to negatively regulate HCV replication (Kanazawa et al., 2004; Yamane et al., 2019), such as RSAD2 (viperin) (Helbig et al., 2011; Wang et al., 2012). Unlike CsA, CsA-Prtc1 treatment did not induce expression of IRF1 target genes **(****Figure 24****),** consistent with our earlier findings evaluating IFN-β expression **(****Figure 9C****).** Importantly, CsA treatment did not induce expression of these genes in the Huh7.5 cells, which we propose are defective for PKR function **(****Figure 13****).**

To confirm the involvement of IRF 1, we generated Huh7 IRF1 KO cell lines by CRISPR/Cas9 **(****Figure 22C****)** and tested the effect of CypI against HCV replication in these cells. Strikingly, the antiviral potency of the CypI against HCV replication was markedly decreased in the IRF1 KO cells **(****Figure 22D****).** Importantly, the effect of IRF1 KO closely matched the phenotype we observed in the PKR KO cells **(****Figure 18D****).** Collectively, these findings demonstrate that PKR signalling through IRF1 determines the potency of CypI against HCV.

### Discussion

CypA is a critical host factor for many viruses, although the mechanisms underlying its remarkably broad usage as a viral host factor remain unclear. One intriguing possibility is that viruses recruit CypA to evade host antiviral responses, evidenced by recent studies implicating CypA in viral innate immune evasion (Rasaiyaah et al., 2013) and in regulation of innate immune signalling (Sun et al., 2014; Liu et al., 2017). Therefore, CypA is an exciting target for broadly-acting antiviral intervention based on disrupting viral evasion and harnessing host intrinsic antiviral responses to combat infection. Given that CypA was linked with HCV immune evasion in patients (Hopkins et al., 2012), we sought to understand the potential mechanisms in the context of HCV infection. We found that CypA is crucial for HCV evasion of PKR-dependent, but not RLR/MAVS-dependent, antiviral responses. Furthermore, our results suggest that a deficiency in PKR-dependent responses, as well as defective RIG-I (Sumpter et al., 2005), may contribute to Huh7.5 cell permissivity for HCV replication, which is consistent with previous observations showing that RIG-I does not play a role (Binder et al., 2007). Indeed, differences in permissivity between Huh7 and Huh7.5 cells are likely governed by a combination of factors, including RIG-I (Sumpter et al., 2005) and CD81 (Koutsoudakis et al., 2007), among others. Overall, our findings: **(a)** clarify the role of Cyps in HCV replication, **(b)** provide mechanistic insight into PKR activity and regulation, and **(c)** contribute to understanding the broad exploitation of CypA by viruses, opening perspectives for broadly acting antiviral therapies based on disrupting CypA-mediated viral evasion.

### Clarifying the roles of Cyps in HCV replication

While Cyps are clearly involved in HCV replication, the respective roles of Cyp family members have been disputed (Watashi et al., 2005; Yang et al., 2008). Although binding of CypB to the HCV RNA-dependent RNA polymerase (NS5B) was suggested to be important for HCV RNA replication (Watashi et al., 2005), the interaction of CypA with the intrinsically unstructured domain II of HCV NS5A (Hanoulle et al., 2009; Foster et al., 2011) was also shown to be required for HCV replication and infection (Yang et al., 2008; Kaul et al., 2009; Chatterji et al., 2009; Liu et al., 2009) Furthermore, CypA and CypB both bind to proline residues within the unstructured domain II of NS5A (Hanoulle et al., 2009; Foster et al., 2011), which also interacts with the HCV polymerase NS5B (Ngure et al., 2016). Here, our data support a direct role for CypB in HCV RNA replication (Watashi et al., 2005), which is consistent with it being equally required in Huh7 and Huh7.5 cells **(****Figure 1C****).** In contrast, the requirement for CypA varies according to cell line and appears to be important for evasion of host antiviral responses in innate sensing competent cells (i.e., Huh7) **(****Figure 1C****).**

We propose a model where CypB forms a complex with NS5A and NS5B to directly regulate HCV RNA replication, while CypA forms a complex with NS5A and PKR, leading to inhibition of PKR-dependent antiviral responses **(****Figure 25****).** CypI, which target both CypA and CypB (Davis et al., 2010), directly inhibit HCV replication in both Huh7 and Huh7.5 cells by targeting CypB, and they disrupt the CypA-NS5A interaction, thus rendering them more efficacious in Huh7 cells because there they relieve NS5A inhibition of PKR and restore PKR-dependent antiviral responses. Notably, a differential requirement for CypA in Huh7-Lunet and Huh7.5 cells has also been observed for replication of genotype 1b (Con1) and genotype 2a (JFH-1) replicons (Kaul et al., 2009), suggesting that these mechanisms are consistent across HCV genotypes.

CypA has been proposed to have a role in HCV assembly (Nag et al., 2012; Anderson et al., 2011), which is likely reflected by our observation that CypA depletion in Huh7.5 cells decreased HCVcc infection **(****Figure 1F****),** but not replication of the HCV replicon **(****Figure 1C****).** This is consistent with previous studies showing a large decrease in HCVcc infection in Huh7.5 cells silenced for CypA expression (Gaska et al., 2019).

### Mechanistic insight into PKR-dependent antiviral responses

NS5A is key for HCV evasion of innate antiviral responses (Madan et al., 2014; Kumthip et al., 2012; Park et al., 2012), including those dependent on PKR (Gale et al., 1997; Gale et al., 1998; Pflugheber et al., 2002). Our data suggest that CypA is required for HCV evasion of PKR-mediated antiviral responses **(****Figure 22A-B)** that involve IRF1 signaling **(****Figure 9F****).** Upon activation by dsRNA binding or cellular stress (Williams, 1999), PKR activates IRF1 (Kirchhoff et al., 1995), and IRF1 was recently shown to drive intrinsic hepatocyte resistance to positive-sense RNA viruses (including HCV and other *Flaviviridae*) (Yamane et al., 2019). Furthermore, IRF1 exerted the most potent inhibition out of 380 ISGs screened against four positive-sense RNA viruses (including HCV, *Flaviviridae* and *Togaviridae*) (Schoggins et al., 2011). Consistent with our observations **(****Figure 18B-C),** the antiviral activity of IRF1 was independent of canonical Jak-STAT IFN signalling (Schoggins et al., 2011), suggesting that IRF1 controls a unique IFN-independent antiviral program that is key for antiviral defense in hepatocytes (Yamane et al., 2019). HCV efficiently controls NF-κB (Kumthip et al., 2012; Park et al., 2012) and MAVS (Li et al., 2005), but appears to be more sensitive to IRF1 restriction (Yamane et al., 2019). We propose that the ability of HCV to inhibit PKR and evade downstream IRF1 responses depends critically on the CypA-NS5A interaction, and that disruption of this interaction by CypI leads to activation of PKR and engages IRF1-dependent antiviral responses.

CypA has been proposed as a regulator of PKR activity in the context of eIF2α phosphorylation (Daito et al., 2014). Our data suggest that CypA is required for regulation of PKR in a broader context, including activation of IRF1, since targeting CypA for proteolytic degradation by PROTAC (e.g. CsA-Prtc1) blocked the activation of IFN-β or IRF1 target gene expression that we observed with CsA **(****Figure 9C****,** **Figure 24****).** In contrast to CsA-Prtc1, CsA disrupts CypA interactions and therefore affects complex formation, but does not cause CypA degradation. Given that CypA regulates protein complexes and that CypA, NS5A and PKR have all been shown to interact (Hanoulle et al., 2009; Daito et al., 2014; Gale et al., 1998), CypA-NS5A-PKR complex formation likely regulates PKR activation in HCV-replicating cells. Our data suggest that perturbation of the complex by CypI disrupts NS5A inhibition of PKR, but not PKR activation of IRF1.

The regulation of PKR is not fully understood (Bou-Nader et al., 2019), and the mechanisms by which PKR activates NF-κB and IRF1 in particular are unclear, although appear to be independent of PKR kinase activity, at least in the case of NF-κB (Bonnet et al., 2000; Bonnet et al., 2006). Our data suggest that activation of IRF1 by PKR does not require canonical PKR activation mechanisms (i.e. autophosphorylation), as CypI inhibit PKR autophosphorylation in HCV-replicating cells **(****Figure 23****)** but still induce PKR-dependent activation of antiviral target genes **(****Figure 22A-B)** regulated by IRF1 and potentially other transcription factors, suggesting distinct mechanisms of PKR activation and downstream signalling.

### A novel antiviral mechanism

Using HCV as a model, we have discovered that CypI restore PKR-dependent antiviral responses to inhibit infection. This represents a novel and potentially broadly acting antiviral mechanism based on inhibition of viral evasion and restoration of host intrinsic antiviral immunity. Many viruses do require CypA (Dawar et al., 2017), including medically important (and currently untreatable) human viruses such as *Flaviviridae* and *Coronaviridae* family members (Qing et al., 2009; de Wilde et al., 2011; Pfefferle et al., 2011). Many viruses also encode PKR antagonists.

Overall, CypA is therefore an attractive antiviral target for a broad array of viruses, including emerging human viruses currently lacking specific antiviral therapies. Here, we contribute to the understanding of CypA-HCV interactions and PKR activation, opening perspectives for the further development of CypA-targeting broadly acting antivirals against untreatable human viruses.

**Table 1: Comparison of CypI IC₅₀ against**

| **HCV replication or infection in different cell lines** | | | | |
|---|---|---|---|---|
| **Replication (SGR)** | **IC₅₀ (µM)** | | | |
| **Cell line** | **CsA** | **CsA-like** | **Depsin** | **CsA-Prtc1** |
| Huh7 | 0.188 ± 0.032 | 0.266 ± 0.037 | 0.043 ± 0.005 | 0.016 ± 0.002 |
| Huh7.5 | 0.955 ± 0.123 | 1.374 ± 0.197 | 0.336 ± 0.053 | 0.055 ± 0.007 |
| Huh7.5-CTRL | 0.373 ± 0.085 | 0.527 ± 0.154 | 0.206 ± 0.043 | 0.070 ± 0.013 |
| Huh7.5-RIG-I | 0.560 ± 0.105 | 0.686 ± 0.181 | 0.243 ± 0.054 | 0.093 ± 0.019 |
| Huh7.5-Mda5 | 0.754 ± 0.160 | 0.950 ± 0.247 | 0.302 ± 0.060 | 0.100 ± 0.018 |
| Huh7.5-RIG-I/Mda5 | 0.820 ± 0.206 | 1.234 ± 0.298 | 0.351 ± 0.085 | 0.101 ± 0.021 |
| Huh7 NT c7 | 0.068 ± 0.008 | 0.184 ± 0.026 | 0.037 ± 0.004 | 0.009 ± 0.001 |
| Huh7 MAVS KO | 0.112 ± 0.019 | 0.160 ± 0.023 | 0.056 ± 0.008 | 0.010 ± 0.001 |
| Huh7 MAVS KO + C508R | 0.075 ± 0.014 | 0.141 ± 0.028 | 0.045 ± 0.006 | 0.008 ± 0.001 |
| Huh7 PKR KO c1 | 0.176 ± 0.034 | 0.971 ± 0.186 | 0.125 ± 0.021 | 0.021± 0.003 |
| Huh7 PKR KO c4 | 0.172 ± 0.040 | 1.249 ± 0.327 | 0.091 ± 0.015 | 0.028 ± 0.004 |
| Huh7 IRF1 KO c10 | 0.183 ± 0.042 | 1.219 ± 0.224 | 0.170 ± 0.039 | 0.027± 0.005 |
| Huh7 IRF1 KO c11 | 0.193 ± 0.047 | 1.593 ± 0.284 | 0.192 ± 0.045 | 0.040 ± 0.006 |
| Huh7 + DMSO | 0.216 ± 0.050 | | | |
| Huh7 + C16 | 0.553 ± 0.098 | | | |
| Huh7.5 + DMSO | 0.879 ± 0.131 | | | |
| Huh7.5 + C16 | 1.750 ± 0.495 | | | |
| | | | | |

| **HCVcc infection** | **IC₅₀ (µM)** | | | |
|---|---|---|---|---|
| **Cell line** | **CsA** | **CsA-like** | **Depsin** | **CsA-Prtc1** |
| Huh7 | 0.043 ± 0.012 | 0.091 ± 0.020 | 0.096 ± 0.016 | 0.003 ± 0.001 |
| Huh7.5 | 0.182 ± 0.037 | 0.316 ± 0.083 | 1.084 ± 0.373 | 0.014 ± 0.003 |
| Huh7 NT c7 | 0.033 ± 0.005 | 0.123 ± 0.023 | 0.018 ± 0.003 | 0.012 ± 0.002 |
| Huh7 PKR KO c4 | 0.095 ± 0.019 | 0.433 ± 0.101 | 0.051 ± 0.011 | 0.023 ± 0.005 |

### Materials and methods

| **Key Resources Table** | | | | |
|---|---|---|---|---|
| **Reagent type (species) or resource** | **Designation** | **Source or reference** | **Identifiers** | **Additional information** |
| gene (*Homo sapiens*) | PPIA (CYPA) | | NCBI Gene:5478 | |
| gene (*Homo sapiens*) | PPIB (CYPB) | | NCBI Gene:5479 | |
| gene (*Homo sapiens*) | PPIF (CYPD) | | NCBI Gene: 10105 | |
| gene (*Homo sapiens*) | EIF2AK2 (PKR) | | NCBI Gene:5610 | |
| gene (*Homo sapiens*) | MAVS | | NCBI Gene:57506 | |
| gene (*Homo sapiens*) | DDX58 (RIG-I) | | NCBI Gene:23586 | |
| gene (*Homo sapiens*) | IFIH1 (MDA5) | | NCBI Gene:64135 | |
| gene (*Homo sapiens*) | IRF 1 | | NCBI Gene:3659 | |
| strain, strain background (Hepatitis C Virus) | HCVcc | Apath LLC | mJ6/JFHRluc2 (APV24) | |
| strain, strain background (*Escherichia coli*) | HB101 | Promega | L2015 | Chemically competent *E. coli* |
| cell line (*Homo sapiens*) | Huh7 | Dr. Yoshiharu Matsuura; originally from Japanese Collection of Research Bioresources Cell Bank | JCRB0403 | |
| cell line (*Homo sapiens*) | Huh7.5 | Apath LLC Blight et al., 2002 | APC 166 | |
| cell line (*Homo sapiens*) | Huh7.5-CTRL | Heit et al., 2002 | | |
| cell line (*Homo sapiens*) | Huh7.5-RIG-I | Heit et al., 2002 | | |
| cell line (*Homo sapiens*) | Huh7.5-Mda5 | Heit et al., 2002 | | |
| cell line (*Homo sapiens*) | Huh7.5-RIG-I/Mda5 | Heit et al., 2002 | | |
| cell line (*Homo sapiens*) | Huh7-Lunet | Friebe et al., 2005 | | |
| cell line (*Homo sapiens*) | HEK293T | ATCC | CRL-3216 | |
| transfected construct (human) | HCV SGR | Schaller et al., 2007 | pFKI389Luc/N S3-3'_dg_JFH | Electroporated into human hepatoma cell line |
| transfected construct (human) | HCV SGR-GND | Schaller et al., 2007 | pFKI389Luc/N S3-3'_dg_JFH_GND | Electroporated into human hepatoma cell line |
| transfected construct (human) | HCV SGR-DEYN | This example | pFKI389Luc/N S3-3'_dg_JFH_DE YN | Electroporated into human hepatoma cell line |
| transfected construct (human) | HCV protein expression construct | Romero-Brey et al., 2012 | pTM-NS3-5B | Transfected into human hepatoma cells |
| transfected construct (human) | p8.91 | Zufferey et al., 1997 | | Lentiviral packaging plasmid |
| transfected construct (human) | pMDG | Naldini et al., 1996 | | VSV-G envelope expressing construct |
| transfected construct (human) | pHIV-SIREN | Schaller et al., 2011 | | Lentiviral transfer plasmid |
| transfected construct (human) | pLentiCRISPRv2 | Prof. Feng Zhang | RRID:Addgene _52961 | Lentiviral transfer plasmid for Cas9-mediated KO |
| transfected construct (human) | pHIV-SIREN-shCypA | This example | | Lentiviral transfer plasmid encoding shRNA for CypA knockdown |
| transfected construct (human) | pHIV-SIREN-shCypB | This example | | Lentiviral transfer plasmid encoding shRNA for CypB knockdown |
| transfected construct (human) | pLentiCRISPRv 2-sgPKR | This example | | Lentiviral transfer plasmid for PKR KO |
| transfected construct (human) | pLentiCRISPRv 2-sgMAVS | This example | | Lentiviral transfer plasmid for MAVS KO |
| transfected construct (human) | pLentiCRISPRv 2-sgIRF1 | This example | | Lentiviral transfer plasmid for IRF 1 KO |
| transfected construct (human) | pSCRPSY-EIF2AK | Feng et al. 2018 | | Lentiviral transfer plasmid for PKR expression |
| transfected construct (human) | pcDNA3.1-MAVS-WT | This example | | Plasmid for expression of WT MAVS |
| transfected construct (human) | pcDNA3.1-MAVS-C508R | This example | | Plasmid for expression of C508R MAVS |
| antibody | Anti-NS5A (mouse monoclonal) | Dr. Joe Grove (UCL) (Lindenbach et al., 2005) | | IF (1:1000) |
| antibody | Anti-β-actin (mouse monoclonal) | Abcam | Cat# AB8226, RRID:AB_306 371 | WB (1: 10000) |
| antibody | Anti-β-actin (rabbit polyclonal) | Abcam | Cat# AB8227 , RRID:AB_230 5186 | WB (1: 1000) |
| antibody | Anti-CypA (rabbit polyclonal) | Enzo | Cat# BML-SA296-0100, RRID:AB_205 1206 | WB (1:5000) |
| antibody | Anti-CypB (rabbit polyclonal) | Abcam | Cat# AB16045, RRID:AB_443 295 | WB (1:1400) |
| antibody | Anti-CypD (mouse monoclonal) | Abcam | Cat# AB110324, RRID:AB_108 64110 | WB (1 µg/mL) |
| antibody | Anti-RIG-I (rabbit monoclonal) | Cell Signaling Technology | Cat# 3743, RRID:AB_226 9233 | WB (1: 1000) |
| antibody | Anti-MAVS (mouse monoclonal) | Santa Cruz Biotechnology | Cat# sc166583, RRID:AB_201 2300 | WB (1:500) |
| antibody | Anti-PKR (rabbit monoclonal) | Abcam | Cat# AB32052, RRID:AB_229 3421 | WB (1: 1000) |
| antibody | Anti-phospho-PKR T446 (rabbit monoclonal) | Abcam | Cat# AB32036, RRID:AB_777 310 | WB (1: 1000) |
| antibody | Anti-IFNAR2 (mouse monoclonal) | Pbl Assay Science | Cat# 21385-1, RRID:AB_354 167 | 2 µg/mL |
| antibody | IgG2A control antibody (mouse) | R&D Systems | Cat# 4460-MG-100, RRID:AB_884 569 | 2 µg/mL |
| commercial assay or kit | Steady-Glo Luciferase Assay System | Promega | Cat# E2510 | |
| commercial assay or kit | MEGAScript T7 Transcription Kit | Ambion, Life Technologies | Cat# AM1334 | |
| commercial assay or kit | Neon Transfection System Kit | Life Technologies | Cat# MPK10025 | |
| commercial assay or kit | Amaxa Nucleofecter System - Kit T | Lonza | Cat # VVCA-1002 | |
| commercial assay or kit | TransIT LT1 Transfection Reagent | Mirus Bio LLC | Cat# MIR 2304 | |
| commercial assay or kit | Fugene-6 Transfection Reagent | Promega | Cat# E2691 | |
| commercial assay or kit | Q5 Site-Directed Mutagenesis | New England Biolabs | Cat# E0554S | |
| commercial assay or kit | QuikChange II Site-Directed Mutagenesis | Agilent | Cat# 200523 | |
| commercial assay or kit | Araldite 502/ Embed 812 kit | Electron Microscopy Sciences | Cat# 13940 | |
| commercial assay or kit | RNeasy Mini Kit | Qiagen | Cat# 74106 | |
| commercial assay or kit | SuperScript III Reverse Transcriptase | Invitrogen | Cat# 18080093 | |
| commercial assay or kit | FastSYBR Green master mix | Applied Biosciences | Cat# 4385610 | |
| commercial assay or kit | AlamarBlue Viability Assay | ThermoScientif ic | Cat# DAL1025 | |
| chemical compound, drug | C16 | Sigma-Aldrich | Cat# I9785 | |
| chemical compound, drug | Ruxolitinib | Cell Guidance Systems | Cat# SM87 | |
| chemical compound, drug | Daclatasvir | Insight Biotechnology | Cat# D101505 | |
| chemical compound, drug | Telaprevir (VX-950) | Generon/Adooq Bioscience | Cat# A10902-2 | |
| chemical compound, drug | CsA-like CypI (JW115) | This example | | |
| chemical compound, drug | Depsin CypI (JW3-38) | This example | | |
| chemical compound, drug | CsA-Prtc1 (JW4-10) | This example | | |

***Cell lines.*** Huh7 and Huh7.5 cells were kindly provided by Dr. Joe Grove. Huh7, Huh7.5 and 293T cells were cultured in DMEM supplemented with 10% FBS, 50 U/mL penicillin and 50 µg/mL streptomycin at 37°C in 5% CO₂. Huh7-Lunet/T7 cells (Friebe et al., 2005) were cultured in the presence of 5 µg/mL Zeocin.

**Inhibitors.** Synthesis of the novel CypI is described below. CypI were resuspended in dimethyl sulfoxide (DMSO, Sigma-Aldrich) as 10 mM stocks. CypI were diluted in DMEM-10% FBS at the indicated concentrations and added to cells at 4 hours post-electroporation unless otherwise indicated. The PKR inhibitor C16 was obtained from Sigma-Aldrich (I9785). Ruxolitinib was obtained from Cell Guidance Systems. Telaprevir (VX-950) was obtained from Generon/Adooq Bioscience (A10902-2). Daclatasvir (BMS-790052) was obtained from Insight Biotechnology (D101505).

***Antibodies.*** Mouse monoclonal anti-NS5A antibody (9E10) was kindly provided by Dr. Joe Grove (UCL) and has previously been described (Lindenbach et al., 2005). Antibodies against β-actin (Abcam; ab8226 or ab8227), CypA (Enzo; BML-SA296-0100), CypB (Abcam; ab16045), CypD (Abcam; ab110324), RIG-I (Cell Signaling Technology; #3743) MAVS (Santa Cruz Biotechnology; sc166583), PKR (Abcam; ab32052) and phospho-PKR T446 (Abcam; ab32036) were also used. Secondary IRDye 680- or 800-labelled antibodies and AlexaFluor-conjugated antibodies were obtained from LI-COR Biosciences or Thermo Scientific, respectively. Anti-human interferon alpha/beta receptor chain 2 antibody (IFNAR2) (Pbl Assay Science, 21385-1) and an IgG2A control antibody (R&D Systems, 4460-MG-100) were used at 2 µg/mL.

***Plasmids.*** The subgenomic reporter replicon pFKI389Luc/NS3-3'_dg_JFH (HCV SGR) and replication deficient mutant with a deletion in the NS5B active site (ΔGDD) have been described previously (Schaller et al., 2007). The plasmid J6/JFHRluc2 (HCVcc) was kindly provided by Dr. Joe Grove (UCL) with permission from Apath LLC. The HCV polyprotein expression construct pTM_NS3-5B has been described previously (Romero-Brey et al., 2012). LentiCRISPRv2 was a gift from Feng Zhang (Addgene plasmid #52961). For knockout of specific genes, synthetic oligos were cloned into LentiCRISPRv2 as described (Sanjana et al., 2014). The lentiviral PKR expression plasmid (pSCRPSY-EIF2AK) (Feng et al., 2018) was a kind gift from Dr. Sam Wilson (University of Glasgow).

***In vitro transcription and electroporation of RNA.*** Plasmid DNA (10 µg) was linearised by digestion with MluI (HCV SGR) or XbaI (HCVcc). Purified linearised DNA (1 µg) was used as a template for *in vitro* transcription according to the T7 MEGAscript Kit instructions (Ambion, Life Technologies). RNA was resuspended in nuclease-free water at a concentration of 1 µg/µL, aliquoted and stored at -80°C. HCV SGR RNA (5 µg) or HCVcc RNA (10 µg) was electroporated into 2 x 10⁶ cells or 4 x 10⁶ target cells, respectively, using either a Neon transfection system (Thermo Scientific) or Amaxa nucleofector (Lonza). In both cases, single-cell suspensions were washed with PBS and resuspended in 100 µL of Buffer R (Neon) or Nucleofector Solution T (Amaxa), respectively. Resuspended cells were mixed with RNA and loaded into a Neon Tip or Amaxa cuvette. Cells were electroporated using the Neon Transfection system (1400 V, 20 ms, 1 pulse) or the Amaxa Nucleofector system (program T-016) and resuspended in DMEM-10% FBS prior to seeding in 96-well plates at a density of ~2 x 10⁴ cells/well. For experiments using IFNAR2 antibody, electroporated cells were plated at ~7.5 x 10³ cells/well.

***Preparation of virus stocks.** In vitro* transcribed RNA (10 µg), generated as described above, was electroporated into 4 x 10⁶ Huh7.5 cells. Electroporated cells were plated into 6-well plates, and were split and expanded as necessary. Supernatants containing HCVcc were collected on days 3 and 7, and filtered through a 0.45 µm syringe filter.

***Lentivirus production and generation of stable cell lines.*** HEK293T cells plated in 10-cm² dishes were transfected with 1 µg packaging plasmid p8. 91 (Zufferey et al., 1997), 1 µg envelope plasmid pMDG encoding VSV-G protein (Naldini et al., 1996) and 1.5 µg of transfer plasmid pHIV-SIREN (Schaller et al., 2011) encoding shRNA or lentiCRISPRv2 (Sanjana et al., 2014) encoding sgRNA or pSCRPSY-EIF2AK2 lentiviral plasmid encoding PKR (Feng et al., 2018) using Fugene-6 transfection reagent (Promega) as described (Fletcher et al., 2015). L entivirus supernatants were collected at 48 h and 72 h posttransfection and clarified by filtration through 0.45 µm syringe filters. Huh7 or Huh7.5 cells were plated in 6-well plates at a density of 2.5 x 10⁵ cells/well prior to being transduced with 1 mL/well of lentivirus supernatant in the presence of 8 µg/mL polybrene. Transduced cells were selected by addition of 2.5 µg/mL puromycin at 72 h post-transduction. Alternatively, to generate CRISPR knockout cells without genome integration of Cas9, the lentiCRISPRv2 plasmid (2.5 µg) was electroporated into Huh7 or Huh7.5 cells (5 x 10⁵ cells) using the Neon electroporator as described above. Electroporated cells were plated in 10-cm² dishes and selected by addition of 2.5 µg/mL puromycin at 24 h post-electroporation. After 72 h of puromycin selection, single cell clones were isolated by limiting dilution in 96-well plates. Loss of target protein expression was confirmed by Western blot.

***Site-directed mutagenesis.*** The HCV NS5A D316E/Y317N mutant (Yang et al., 2010) was generated in the subgenomic replicon using a modified version of the Q5 Site-Directed Mutagenesis Protocol (New England BioLabs). The PCR reaction was assembled according to the protocol, using mutagenic primers. PCR product (4 µL) was used in the subsequent kinase-ligase-Dpn1 reaction, following which 5 µL of ligation product was transformed into chemically competent *E.coli* (strain HB 101). The mutation was confirmed by sequencing using an NS5A forward primer. The MAVS C508R mutation conferring NS3/4A protease resistance (Li et al., 2005) was generated using a modified version of the QuikChange II Site-Directed Mutagenesis (Agilent) protocol. The PCR was assembled using the MAVS-WT plasmid (50 ng) as template with *Pfu* Ultra High Fidelity polymerase (Agilent) and the mutagenic primers. The PCR product was incubated with DpnI restriction enzyme (10 U/µL) at 37 °C for 1 hour and then transformed into *E.coli* HB101. The mutation was confirmed by sequencing using a CMV forward primer.

***Luciferase measurement.*** Firefly luciferase activity was measured using the SteadyGlo reagent according to the manufacturer instructions (Promega). For measurement of Renilla luciferase activity, cells were washed once with PBS and then lysed with 50 µL/well of 1X passive lysis buffer (Promega). Lysates (20 µL) were transferred to 96-well white plates and Renilla activity was measured following addition of 50 µL of 2 µg/mL coelenterazine (NanoLight).

***Transmission electron microscopy.*** Huh7-Lunet/T7 cells were seeded onto glass coverslips at a density of 1 x 10⁵ cells/well. Cells were transfected 24 hours later with the pTM_NS3-5B polyprotein expression construct using the TransIT LT1 transfection reagent (Mirus Bio LLC, Madison, WI). After 4 hours, cells were treated with DMSO or CypI (at 5X EC₉₀, corresponding to 5 µM for CsA, 2 µM for depsin and 1 µM for CsA-Prtc1) until fixation 21 hours later. Cells were fixed for 30 minutes at room temperature with 2.5% glutaraldehyde in 50 mM sodium cacodylate (caco) buffer (pH 7.2) containing 10 mM MgCl₂, 10 mM CaCl₂, 100 mM KCl and 2% sucrose. Following washes with 50 mM caco buffer, cells were incubated with 2% osmium tetroxide in caco buffer for 40 minutes on ice. Samples were then washed with distilled water and incubated in 0.5% uranyl acetate overnight at 4°C. Samples were then washed with distilled water, and dehydrated by sequential incubation with increasing concentrations of ethanol (40%, 50%, 60%, 70%, 80%, 95%, 100%). Dehydrated samples were embedded in araldite-Epon (Araldite 502/Embed 812 kit, Electron Microscopy Sciences) and polymerized for 2 days at 60°C. Embedded cells were then cut into 70-nm thin sections (Leica Ultracut UCT microtome) and mounted onto a mesh grid. Sections were contrasted by incubation with 3% uranyl acetate in 70% methanol for 5 minutes, followed by incubation with 2% lead citrate in distilled water for 2 minutes. Finally, sections were visualised using a JEOL JEM1400 transmission electron microscope (JEOL Ltd., Tokyo, Japan) in the Electron Microscopy Core Facility at Heidelberg University. Images were analysed and double membrane vesicles were counted using ImageJ.

***Immunofluorescence.*** Huh7-Lunet/T7 cells seeded onto glass coverslips at a density of 1 x 10⁵ cells/well were fixed in 4% paraformaldehyde and then washed three times with PBS. Cells were then incubated with NSSA-specific monoclonal antibody (9E10) diluted 1:1000 in PBS containing 1% FBS and 0.5% Triton X-100. After overnight incubation at 4C, cells were washed and secondary donkey anti-mouse AlexaFluor-488 antibody was added. Nuclear DNA was detected by DAPI staining. After incubation at room temperature for 1 hour, coverslips were washed, mounted on slides with FluoromountG and sealed with clear nail polish and imaged using a Nikon Eclipse Ti with 10x objective.

***qRT-PCR.*** Cellular RNA was extracted using the RNeasy Mini kit (Qiagen) according to the manufacturer instructions. Recovered RNA was quantitated by Nanodrop and 500 ng of RNA was used to synthesize cDNA following the Superscript III Reverse Transcriptase protocol (Invitrogen). The resulting cDNA was diluted 1:5 in nuclease-free water prior to quantitative PCR (qPCR) using the FastSYBR Green Master Mix (Applied Biosciences). Reactions contained 5 µL 2X FastSYBR Green master mix, 2 µL diluted cDNA, 1 µL forward primer, 1 µL reverse primer and 1 µL nuclease-free water. Expression of IFN-β, ISGs and glyceraldehyde-3-phosphate dehydrogenase (GAPDH) was determined using specific primers (primer details in **Table S1**). Following normalisation to GAPDH expression, IFN-β or ISG expression was calculated as fold increase relative to DMSO-treated cells.

***Western blot**.* Cells were resuspended in cell lysis buffer (50 mM Tris pH8, 150 mM NaCl, 1 mM EDTA, 10% glycerol, 1% Triton X-100 and 0.05% NP40). Cell lysates were incubated on ice for 30 minutes, followed by centrifugation at 14,000 rpm at 4°C for 15 minutes. Samples were diluted in 4X SDS-PAGE loading buffer (200 mM Tris pH 6.8, 8% SDS, 0.4% bromophenol blue, 40% glycerol and 2% β-mercaptoethanol), heated at 95°C for 5 minutes, and loaded onto 10% or 15% polyacrylamide-SDS gels. Following electrophoresis, proteins were transferred to a nitrocellulose membrane using the Bio-Rad TransBlot Turbo system according to the manufacturer instructions. Membranes were blocked in 5% milk diluted in Tris-buffered saline (TBS) with 0.5% Tween (TBS-T) for 1 hour prior to incubation with primary antibodies diluted in blocking solution overnight at 4°C. Membranes were washed extensively in TBS-T prior to incubation for 1 hour at room temperature with IRDye 800-labelled or IRDye 680-labeled antibodies diluted 1: 10,000 in blocking solution. Membranes were washed extensively in TBS-T followed by washes in TBS (without Tween) and then scanned using an Odyssey Infrared imaging System (LI-COR Biosciences). Alternatively, membranes were incubated with horseradish peroxidase-conjugated mouse-specific secondary antibodies (Sigma-Aldrich, St. Louis, MO) diluted 1: 10,000, prior to detection with the Western Lightning Plus-ECL reagent (Perkin-Elmer, Waltham, MA) and the Intas Science imager. Where indicated, densitometry analyses were performed using ImageJ and expressed as adjusted band density (normalized to actin loading control).

***Cell viability assay.*** Huh7 or Huh7.5 cells were seeded in 96-well plates at a density of 1 x 10⁴ cells/well prior to being treated with serially diluted CypI. After 48 hours, cell viability was assessed using the alamarBlue Cell Viability Assay (ThermoScientific) according to the manufacturer instructions. Absorbance was measured using a microplate reader (Multiskan FC Microplate reader, Thermo Scientific) at 570 nM with a reference measurement at 595 nm.

### Synthesis of novel CypI

### CsA-Prtcl synthesis

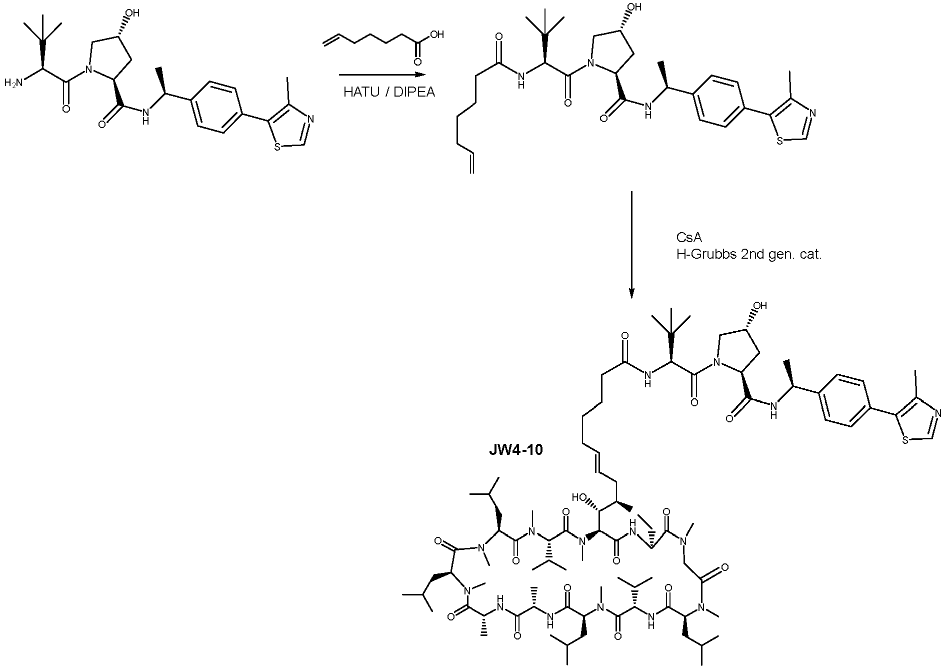

### General Method A (metathesis)

To a solution of Cyclosporin A (72 mg, 0.06 mmol) in DCM (2 mL) was added the olefin (0.072 mmol) and Hoveyda-Grubbs 2^{nd} generation catalyst (6 mg, 0.01 mmol, 17mol%). The reaction was stirred in the microwave at 90°C for 30 minutes and then allowed to cool. Triethylamine was added to the mixture and then stirred overnight with excess P(CH₂OH)₃ to coordinate the ruthenium catalyst. This was then washed away with brine and water before the mixture was passed through a Stratospheres PL Thiol MP SPE cartridge (polymer Lab, Varian Inc) to remove any remaining catalyst. The crude product was purified by chromatography (as detailed) to give the product.

### (2S,4R)-1-((S)-2-(hept-6-enamido)-3,3-dimethylbutanoyl)-4-hydroxy-N-((S)-1-(4-(4-methylthiazol-5-yl)phenyl)ethyl)pyrrolidine-2-carboxamide (JW4-7)

To a solution of E3 ligase ligand 1 (0.072 g, 0.169 mmol) in MeCN were added 6-heptenoic acid (25 µL, 0.186 mmol), HATU (0.071 g, 0.186 mmol) and N,N-diisopropylethylamine (59 µL, 0.338 mmol) and this mixture was stirred at room temperature overnight. The mixture was then purified by column chromatography with 30-50% MeOH in DCM, and then re-purified with 25-35% MeOH in DCM.

The product was isolated as a pale yellow solid (46 mg, 49% yield).

¹H NMR (700MHz, CDCl₃) δ 8.81 (s, C=N, 1H), 3.39 (s, NMe, 3H), 8.20 (d, NH, 1H), 7.62 (d, NH, 1H), 7.29 (d, 2H), 7.19 (d, 2H), 5.59 (m, alkene, 3H), 4.95 (s, OH, 1H), 4.82 (q, 1H), 4.79 (q, 1H), 4.33 (d, 1H), 4.24 (t, 1H), 4.04 (sext, 1H), 2.32 (t, 3H), 2.26 (s, 3H), 2.07 (m, 1H), 2.01 (t, 1H), 1.93 (m, 1H), 1.83 (m, 2H), 1.60 (m, 1H), 1.30 (m, 2H), 1.15 (m, 2H), 0.75 (s, 9H).

¹³C NMR (600 MHz, CDCl₃) δ 177.44, 176.01, 174.97, 156.85, 153.10, 150.01, 136.48, 135.03, 74.14, 63.88, 61.73, 61.57, 60.25, 53.94, 53.06, 43.04, 40.51, 40.01, 38.20, 33.16, 33.04, 31.76, 30.24, 29.35, 27.71, 21.29.

LCMS (ESI, *m*/*z*): [MH]⁺ calcd. for [C₃₀H₄₂N₄O₄S+H]⁺, 555.3005; found 555.3005.

### [Gly-(1S,2R,E)-10-amido (E3 ligase ligand)-1-hydroxy-2-methyloct-4-ene]¹ CsA (CsA-Prtc1, JW4-10)

### Using Method A

The crude product was purified by flash silica chromatography 0-15% MeOH in DCM, then repurified with 5-9% MeOH in DCM to give CsA-Prtc1, JW4-10 as an off-white solid (41 mg, 22% yield).

¹H NMR (600 MHz, CDCl₃) δ 3.48 (s, NMe, 3H), 3.39 (s, NMe, 3H), 3.22 (s, NMe, 3H), 3.11 (s, NMe, 3H), 3.09 (s, NMe, 3H), 2.69 (s, NMe, 3H), 2.68 (s, NMe, 3H).

¹³C NMR (600 MHz, CDCl₃) δ 174.04, 173.79, 173.58, 173.51, 171.66, 171.30, 171.26, 170.53, 170.47, 170.22, 170.17.

HRMS (*m*/*z*): [MH]⁺ calcd. for C₈₉H₁₄₇N₁₅O₁₆S, 1715.0944; found 1715.0952.

### JW115 synthesis

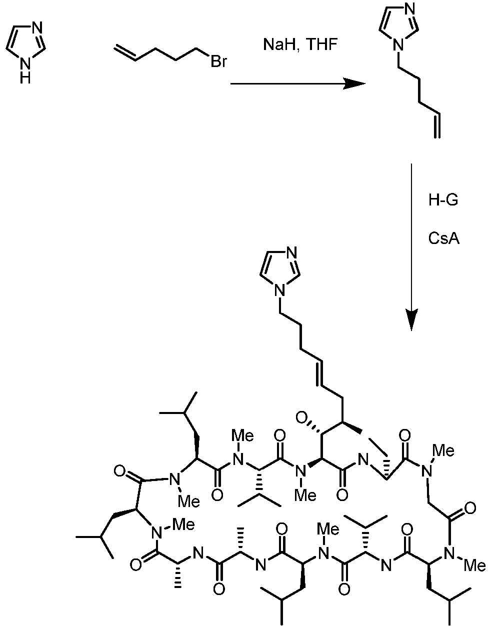

### 1-(pent-4-en-1-yl)-1H-imidazole

To a solution of imidazole (1.702 g, 25 mmol) in THF was added portionwise NaH (60% in mineral oil, 600 mg, 25 mmol). The resulting mixture was refluxed for an hour before cooling to room temperature and the addition of 5-bromo-pent-1-ene (3.25 ml, 27.5 mmol). The mixture was then refluxed for 3 hours, allowed to cool and diluted with diethyl ether. The organic extracts were combined, washed with brine, dried over MgSO₄ and concentrated under reduced pressure. Product was purified with column chromatography 0-20% MeOH in DCM, followed by 9-12% MeOH in DCM.

The product was isolated as a transparent oil (648.5 mg, 68%).

¹H NMR (600 MHz, CDCl₃) δ 7.55 (s, 1H), 7.08 (s, 1H), 6.91 (s, 1H), 5.68-5.82 (m, 1H), 4.97-5.10 (m, 2H), 3.95 (t, *J=* 3.95 Hz, 3H), 2.01-2.11 (m, 2H), 1.85-1.92 (m, 2H).

¹³C NMR (600 MHz, CDCl₃) δ 137.16, 136.77, 129.39, 118.85, 116.24, 46.25, 30.46, 30.08.

HRMS (*m*/*z*): [MH]⁺ calcd. for [C₈H₁₂N+H]⁺,137.1079; found 137.1079.

### [Gly-(1S,2R,E)-8-(1H-imidazole-1-yl)-1-hydroxy-2-methyloct-4-ene]¹ CsA (JW115)

### Using Method A

The crude product was purified by flash silica chromatography 0-10% MeOH in DCM, and re-purified in 5-9% MeOH in DCM to give **JW115** as an off-white powder (11 mg, 10%). ¹H NMR (600 MHz, CDCl₃) δ 3.48 (s, NMe, 3H), 3.39 (s, NMe, 3H), 3.21 (s, NMe, 3H), 3.12 (s, NMe, 3H), 3.11 (s, NMe, 3H), 2.68 (s, NMe, 3H), 2.66 (s, NMe, 3H).

¹³C NMR (600 MHz, CDCl₃) δ 173.92, 173.79, 173.55, 173.48, 171.67, 171.32, 171.23, 170.49, 170.44, 170.19, 170.16.

MS (*m*/*z*): [MH]⁺ calcd. for C₆₇H₁₁₇N₁₃O₁₂, 1296.73; found 1296.85.

### REFERENCES

Anderson, L. J., Lin, K., Compton, T., & Wiedmann, B. (2011). Inhibition of cyclophilins alters lipid trafficking and blocks hepatitis C virus secretion. Virol J, 8, 329. doi:10.1186/1743-422X-8-329
Barber, G. N., Wambach, M., Wong, M. L., Dever, T. E., Hinnebusch, A. G., & Katze, M. G. (1993). Translational regulation by the interferon-induced double-stranded-RNA-activated 68-kDa protein kinase. Proc Natl Acad Sci U S A, 90(10), 4621-4625. doi:10.1073/pnas.90.10.4621
Bender, S., Reuter, A., Eberle, F., Einhorn, E., Binder, M., & Bartenschlager, R. (2015). Activation of type I and III interferon response by mitochondrial and peroxisomal MAVS and inhibition by hepatitis C virus. PLoS Pathog, 11, e1005264. doi:10.1371/journal.ppat.1005264
Berger, C., Romero-Brey, I., Radujkovic, D., Terreux, R., Zayas, M., Paul, D., Harak, C., Hoppe, S., Gao, M., Penin, F., Lohmann, V., & Bartenschlager, R. (2014). Daclatasvir-like inhibitors of NSSA block early biogenesis of hepatitis C virus-induced membranous replication factories, independent of RNA replication. Gastroenterology, 147(5), 1094-105.e25. doi:10.1053/j.gastro.2014.07.019
Binder, M., Kochs, G., Bartenschlager, R., & Lohmann, V. (2007). Hepatitis C virus escape from the interferon regulatory factor 3 pathway by a passive and active evasion strategy. Hepatology, 46(5), 1365-1374. doi:10.1002/hep.21829
Blight, K. J., McKeating, J. A., & Rice, C. M. (2002). Highly permissive cell lines for subgenomic and genomic hepatitis C virus RNA replication. J Virol, 76(24), 13001-13014. doi:10.1128/jvi.76.24.13001-13014.2002
Bobardt, M., Chatterji, U., Lim, P., Gawlik, K., & Gallay, P. (2014). Both cyclophilin inhibitors and direct-acting antivirals prevent PKR activation in HCV-infected cells. Open Virol J, 8, 1-8. doi:10.2174/1874357901408010001
Bonnet, M. C., Daurat, C., Ottone, C., & Meurs, E. F. (2006). The N-terminus of PKR is responsible for the activation of the NF-kappaB signaling pathway by interacting with the IKK complex. Cell Signal, 18(11), 1865-1875. doi:10.1016/j.cellsig.2006.02.010
Bonnet, M. C., Weil, R., Dam, E., Hovanessian, A. G., & Meurs, E. F. (2000). PKR stimulates NF-kappaB irrespective of its kinase function by interacting with the IkappaB kinase complex. Mol Cell Biol, 20(13), 4532-4542. doi:10.1128/mcb.20.13.4532-4542.2000
Bou-Nader, C., Gordon, J. M., Henderson, F. E., & Zhang, J. (2019). The search for a PKR code-differential regulation of protein kinase R activity by diverse RNA and protein regulators. RNA, 25(5), 539-556. doi:10.1261/rna.070169.118
Chatterji, U., Bobardt, M., Selvarajah, S., Yang, F., Tang, H., Sakamoto, N., Vuagniaux, G., Parkinson, T., & Gallay, P. (2009). The isomerase active site of cyclophilin A is critical for hepatitis C virus replication. J Biol Chem, 284(25), 16998-17005. doi:10.1074/jbc.M109.007625
Chatterji, U., Bobardt, M., Tai, A., Wood, M., & Gallay, P. A. (2015). Cyclophilin and NS5A inhibitors, but not other anti-hepatitis C virus (HCV) agents, preclude HCV-mediated formation of double-membrane-vesicle viral factories. Antimicrob Agents Chemother, 59(5), 2496-2507. doi:10.1128/AAC.04958-14
Chong, K. L., Feng, L., Schappert, K., Meurs, E., Donahue, T. F., Friesen, J. D., Hovanessian, A. G., & Williams, B. R. (1992). Human p68 kinase exhibits growth suppression in yeast and homology to the translational regulator GCN2. EMBO J, 11(4), 1553-1562. Retrieved from https://www.ncbi.nlm.nih.gov/pubmed/1348691
Daito, T., Watashi, K., Sluder, A., Ohashi, H., Nakajima, S., Borroto-Esoda, K., Fujita, T., & Wakita, T. (2014). Cyclophilin inhibitors reduce phosphorylation of RNA-dependent protein kinase to restore expression of IFN-stimulated genes in HCV-infected cells. Gastroenterology, 147(2), 463-472. doi:10.1053/j.gastro.2014.04.035
Davis, T. L., Walker, J. R., Campagna-Slater, V., Finerty, P. J., Paramanathan, R., Bernstein, G., MacKenzie, F., Tempel, W., Ouyang, H., Lee, W. H., Eisenmesser, E. Z., & Dhe-Paganon, S. (2010). Structural and biochemical characterization of the human cyclophilin family of peptidyl-prolyl isomerases. PLoS Biol, 8(7), e1000439. doi:10.1371/journal.pbio.1000439
Dawar, F. U., Tu, J., Khattak, M. N., Mei, J., & Lin, L. (2017). Cyclophilin A: A key factor in virus replication and potential target for anti-viral therapy. Curr Issues Mol Biol, 21, 1-20. doi:10.21775/cimb.021.001
de Wilde, A. H., Zevenhoven-Dobbe, J. C., van der Meer, Y., Thiel, V., Narayanan, K., Makino, S., Snijder, E. J., & van Hemert, M. J. (2011). Cyclosporin A inhibits the replication of diverse coronaviruses. J Gen Virol, 92(Pt 11), 2542-2548. doi:10.1099/vir.0.034983-0
Feng, J., Wickenhagen, A., Turnbull, M. L., Rezelj, V. V., Kreher, F., Tilston-Lunel, N. L., Slack, G. S., Brennan, B., Koudriakova, E., Shaw, A. E., Rihn, S. J., Rice, C. M., Bieniasz, P. D., Elliott, R. M., Shi, X., & Wilson, S. J. (2018). Interferon-stimulated gene (ISG)-expression screening reveals the specific antibunyaviral activity of ISG20. J Virol, 92(13). doi:10.1128/JVI.02140-17
Fletcher, A. J., Christensen, D. E., Nelson, C., Tan, C. P., Schaller, T., Lehner, P. J., Sundquist, W. I., & Towers, G. J. (2015). TRIM5α requires Ube2W to anchor Lys63-linked ubiquitin chains and restrict reverse transcription. EMBO J, 34(15), 2078-2095. doi:10.15252/embj.201490361
Foster, T. L., Gallay, P., Stonehouse, N. J., & Harris, M. (2011). Cyclophilin A interacts with domain II of hepatitis C virus NS5A and stimulates RNA binding in an isomerase-dependent manner. J Virol, 85(14), 7460-7464. doi:10.1128/JVI.00393-11
Friebe, P., Boudet, J., Simorre, J. P., & Bartenschlager, R. (2005). Kissing-loop interaction in the 3' end of the hepatitis C virus genome essential for RNA replication. J Virol, 79(1), 380-392. doi:10.1128/JVI.79.1.380-392.2005
Gale, M., Blakely, C. M., Kwieciszewski, B., Tan, S. L., Dossett, M., Tang, N. M., Korth, M. J., Polyak, S. J., Gretch, D. R., & Katze, M. G. (1998). Control of PKR protein kinase by hepatitis C virus nonstructural 5A protein: molecular mechanisms of kinase regulation. Mol Cell Biol, 18(9), 5208-5218. doi:10.1128/mcb.18.9.5208
Gale, M. J., Korth, M. J., Tang, N. M., Tan, S. L., Hopkins, D. A., Dever, T. E., Polyak, S. J., Gretch, D. R., & Katze, M. G. (1997). Evidence that hepatitis C virus resistance to interferon is mediated through repression of the PKR protein kinase by the nonstructural 5A protein. Virology, 230(2), 217-227. doi:10.1006/viro.1997.8493
Gao, M., Nettles, R. E., Belema, M., Snyder, L. B., Nguyen, V. N., Fridell, R. A., Serrano-Wu, M. H., Langley, D. R., Sun, J. H., O'Boyle, D. R., Lemm, J. A., Wang, C., Knipe, J. O., Chien, C., Colonno, R. J., Grasela, D. M., Meanwell, N. A., & Hamann, L. G. (2010). Chemical genetics strategy identifies an HCV NS5A inhibitor with a potent clinical effect. Nature, 465(7294), 96-100. doi:10.1038/nature08960
Gaska, J. M., Balev, M., Ding, Q., Heller, B., & Ploss, A. (2019). Differences across cyclophilin A orthologs contribute to the host range restriction of hepatitis C virus. Elife, 8. doi:10.7554/eLife.44436
Grolleau, A., Kaplan, M. J., Hanash, S. M., Beretta, L., & Richardson, B. (2000). Impaired translational response and increased protein kinase PKR expression in T cells from lupus patients. J Clin Invest, 106(12), 1561-1568. doi:10.1172/JCI9352
Hanoulle, X., Badillo, A., Wieruszeski, J. M., Verdegem, D., Landrieu, I., Bartenschlager, R., Penin, F., & Lippens, G. (2009). Hepatitis C virus NS5A protein is a substrate for the peptidyl-prolyl cis/trans isomerase activity of cyclophilins A and B. J Biol Chem, 284(20), 13589-13601. doi:10.1074/jbc.M809244200
Helbig, K. J., Eyre, N. S., Yip, E., Narayana, S., Li, K., Fiches, G., McCartney, E. M., Jangra, R. K., Lemon, S. M., & Beard, M. R. (2011). The antiviral protein viperin inhibits hepatitis C virus replication via interaction with nonstructural protein 5A. Hepatology, 54(5), 1506-1517. doi: 10.1002/hep.24542
Hiet, M. S., Bauhofer, O., Zayas, M., Roth, H., Tanaka, Y., Schirmacher, P., Willemsen, J., Grünvogel, O., Bender, S., Binder, M., Lohmann, V., Lotteau, V., Ruggieri, A., & Bartenschlager, R. (2015). Control of temporal activation of hepatitis C virus-induced interferon response by domain 2 of nonstructural protein 5A. J Hepatol, 63(4), 829-837. doi:10.1016/j.jhep.2015.04.015
Hopkins, S., DiMassimo, B., Rusnak, P., Heuman, D., Lalezari, J., Sluder, A., Scorneaux, B., Mosier, S., Kowalczyk, P., Ribeill, Y., Baugh, J., & Gallay, P. (2012). The cyclophilin inhibitor SCY-635 suppresses viral replication and induces endogenous interferons in patients with chronic HCV genotype 1 infection. J Hepatol, 57(1), 47-54. doi:10.1016/j.jhep.2012.02.024
Jammi, N. V., Whitby, L. R., & Beal, P. A. (2003). Small molecule inhibitors of the RNA-dependent protein kinase. Biochem Biophys Res Commun, 308(1), 50-57.
Kanazawa, N., Kurosaki, M., Sakamoto, N., Enomoto, N., Itsui, Y., Yamashiro, T., Tanabe, Y., Maekawa, S., Nakagawa, M., Chen, C. H., Kakinuma, S., Oshima, S., Nakamura, T., Kato, T., Wakita, T., & Watanabe, M. (2004). Regulation of hepatitis C virus replication by interferon regulatory factor 1. J Virol, 78(18), 9713-9720. doi:10.1128/JVI.78.18.9713-9720.2004
Kaul, A., Stauffer, S., Berger, C., Pertel, T., Schmitt, J., Kallis, S., Zayas, M., Lopez, M. Z., Lohmann, V., Luban, J., & Bartenschlager, R. (2009). Essential role of cyclophilin A for hepatitis C virus replication and virus production and possible link to polyprotein cleavage kinetics. PLoS Pathog, 5(8), e1000546. doi:10.1371/journal.ppat. 1000546
Kim, K., Dauphin, A., Komurlu, S., McCauley, S. M., Yurkovetskiy, L., Carbone, C., Diehl, W. E., Stramio-De-Castilla, C., Campbell, E. M., & Luban, J. (2019). Cyclophilin A protects HIV-1 from restriction by human TRIM5α. Nat. Microbiol. 4:2044-2051. doi: 10.1038/s41564-019-0592-5.
Kirchhoff, S., Koromilas, A. E., Schaper, F., Grashoff, M., Sonenberg, N., & Hauser, H. (1995). IRF-1 induced cell growth inhibition and interferon induction requires the activity of the protein kinase PKR. Oncogene, 11(3), 439-445. Retrieved from https://www.ncbi.nlm.nih.gov/entrez/query.fcgi?cmd=Retrieve&db=PubMed&dopt= Citation&list_uids=7543195
Koutsoudakis, G., Herrmann, E., Kallis, S., Bartenschlager, R., & Pietschmann, T. (2007). The level of CD81 cell surface expression is a key determinant for productive entry of hepatitis C virus into host cells. J Virol, 81(2), 588-598. doi:10.1128/JVI.01534-06
Kumthip, K., Chusri, P., Jilg, N., Zhao, L., Fusco, D. N., Zhao, H., Goto, K., Cheng, D., Schaefer, E. A., Zhang, L., Pantip, C., Thongsawat, S., O'Brien, A., Peng, L. F., Maneekarn, N., Chung, R. T., & Lin, W. (2012). Hepatitis C virus NS5A disrupts STAT1 phosphorylation and suppresses type I interferon signaling. J Virol, 86(16), 8581-8591. doi:10.1128/JVI.00533-12
Li, X. D., Sun, L., Seth, R. B., Pineda, G., & Chen, Z. J. (2005). Hepatitis C virus protease NS3/4A cleaves mitochondrial antiviral signaling protein off the mitochondria to evade innate immunity. Proc Natl Acad Sci U S A, 102(49), 17717-17722. doi:10.1073/pnas.0508531102
Lindenbach, B. D., Evans, M. J., Syder, A. J., Wölk, B., Tellinghuisen, T. L., Liu, C. C., Maruyama, T., Hynes, R. O., Burton, D. R., McKeating, J. A., & Rice, C. M. (2005). Complete replication of hepatitis C virus in cell culture. Science, 309(5734), 623-626. doi:10.1126/science.1114016
Liu, J., Farmer, J. D., Lane, W. S., Friedman, J., Weissman, I., & Schreiber, S. L. (1991). Calcineurin is a common target of cyclophilin-cyclosporin A and FKBP-FK506 complexes. Cell, 66(4), 807-815. Retrieved from https://www.ncbi.nlm.nih.gov/entrez/query.fcgi?cmd=Retrieve&db=PubMed&dopt= Citation&list_uids=1715244
Liu, W., Li, J., Zheng, W., Shang, Y., Zhao, Z., Wang, S., Bi, Y., Zhang, S., Xu, C., Duan, Z., Zhang, L., Wang, Y. L., Jiang, Z., Liu, W., & Sun, L. (2017). Cyclophilin A-regulated ubiquitination is critical for RIG-I-mediated antiviral immune responses. Elife, 6. doi:10.7554/eLife.24425
Liu, Z., Yang, F., Robotham, J. M., & Tang, H. (2009). Critical role of cyclophilin A and its prolyl-peptidyl isomerase activity in the structure and function of the hepatitis C virus replication complex. J Virol, 83(13), 6554-6565. doi:10.1128/JVI.02550-08
Luban, J., Bossolt, K. L., Franke, E. K., Kalpana, G. V., & Goff, S. P. (1993). Human immunodeficiency virus type 1 Gag protein binds to cyclophilins A and B. Cell, 73(6), 1067-1078. Retrieved from https://www.ncbi.nlm.nih.gov/entrez/query.fcgi?cmd=Retrieve&db=PubMed&dopt= Citation&list_uids=8513493
Mackman, R. L., Steadman, V. A., Dean, D. K., Jansa, P., Poullennec, K. G., Appleby, T., Austin, C., Blakemore, C. A, Cai, R., Cannizzaro, C., Chin, G., Chiva, J. C., Dunbar, N. A., Fliri, H., Highton, A. J., Hui, H., Ji, M., Jin, H., Karki, K., Keats, A. J., Lazarides, L., Lee, Y. J., Liclican, A., Mish, M., Murray, B., Pettit, S. B., Pyun, P., Sangi, M., Santos, R., Sanvoisin, J., Schmitz, U., Schrier, A., Siegel, D., Sperandio, D., Stepan, G., Tian, Y., Watt, G. M., Yang, H., & Schultz, B. E. (2018). Discovery of a potent and orally bioavailable cyclophilin inhibitor derived from the sanglifehrin macrocycle. J Med Chem, 61(21), 9473-9499. doi:10.1021/acs.jmedchem.8b00802
Madan, V., Paul, D., Lohmann, V., & Bartenschlager, R. (2014). Inhibition of HCV replication by cyclophilin antagonists is linked to replication fitness and occurs by inhibition of membranous web formation. Gastroenterology, 146(5), 1361-72.e1. doi:10.1053/j.gastro.2014.01.055
Nag, A., Robotham, J. M., & Tang, H. (2012). Suppression of viral RNA binding and the assembly of infectious hepatitis C virus particles in vitro by cyclophilin inhibitors. J Virol, 86(23), 12616-12624. doi:10.1128/JVI.01351-12
Naldini, L., Blömer, U., Gallay, P., Ory, D., Mulligan, R., Gage, F. H., Verma, I. M., & Trono, D. (1996). In vivo gene delivery and stable transduction of nondividing cells by a lentiviral vector. Science, 272(5259), 263-267. Retrieved from https://www.ncbi.nlm.nih.gov/entrez/query.fcgi?cmd=Retrieve&db=PubMed&dopt= Citation&list_uids=8602510
Neufeldt, C. J., Joyce, M. A., Van Buuren, N., Levin, A., Kirkegaard, K., Gale, M., Tyrrell, D. L., & Wozniak, R. W. (2016). The hepatitis C virus-induced membranous web and associated nuclear transport machinery limit access of pattern recognition receptors to viral replication sites. PLoS Pathog, 12(2), e1005428. doi:10.1371/journal.ppat.1005428
Ngure, M., Issur, M., Shkriabai, N., Liu, H. W., Cosa, G., Kvaratskhelia, M., & Götte, M. (2016). Interactions of the Disordered Domain II of Hepatitis C Virus NS5A with Cyclophilin A, NS5B, and Viral RNA Show Extensive Overlap. ACS Infect Dis, 2(11), 839-851. doi:10.1021/acsinfecdis.6b00143
Obata, Y., Yamamoto, K., Miyazaki, M., Shimotohno, K., Kohno, S., & Matsuyama, T. (2005). Role of cyclophilin B in activation of interferon regulatory factor-3. J Biol Chem, 280(18), 18355-18360. doi:10.1074/jbc.M501684200
Park, J., Kang, W., Ryu, S. W., Kim, W. I., Chang, D. Y., Lee, D. H., Park, D. Y., Choi, Y. H., Choi, K., Shin, E. C., & Choi, C. (2012). Hepatitis C virus infection enhances TNFα-induced cell death via suppression of NF-κB. Hepatology, 56(3), 831-840. doi:10.1002/hep.25726
Pfefferle, S., Schöpf, J., Kögl, M., Friedel, C. C., Müller, M. A., Carbajo-Lozoya, J., Stellberger, T., von Dall'Armi, E., Herzog, P., Kallies, S., Niemeyer, D., Ditt, V., Kuri, T., Züst, R., Pumpor, K., Hilgenfeld, R., Schwarz, F., Zimmer, R., Steffen, I., Weber, F., Thiel, V., Herrler, G., Thiel, H. J., Schwegmann-Wessels, C., Pöhlmann, S., Haas, J., Drosten, C., & von Brunn, A. (2011). The SARS-coronavirus-host interactome: identification of cyclophilins as target for pan-coronavirus inhibitors. PLoS Pathog, 7(10), e1002331. doi:10.1371/journal.ppat.1002331
Pflugheber, J., Fredericksen, B., Sumpter, R., Wang, C., Ware, F., Sodora, D. L., & Gale, M. (2002). Regulation of PKR and IRF-1 during hepatitis C virus RNA replication. Proc Natl Acad Sci U S A, 99(7), 4650-4655. doi:10.1073/pnas.062055699
Qing, M., Yang, F., Zhang, B., Zou, G., Robida, J. M., Yuan, Z., Tang, H., & Shi, P. Y. (2009). Cyclosporine inhibits flavivirus replication through blocking the interaction between host cyclophilins and viral NS5 protein. Antimicrob Agents Chemother, 53(8), 3226-3235. doi:10.1128/AAC.00189-09
Rasaiyaah, J., Tan, C. P., Fletcher, A. J., Price, A. J., Blondeau, C., Hilditch, L., Jacques, D. A., Selwood, D. L., James, L. C., Noursadeghi, M., & Towers, G. J. (2013). HIV-1 evades innate immune recognition through specific cofactor recruitment. Nature, 503(7476), 402-405. doi:10.1038/nature12769
Romero-Brey, I., Berger, C., Kallis, S., Kolovou, A., Paul, D., Lohmann, V., & Bartenschlager, R. (2015). NS5A domain 1 and polyprotein cleavage kinetics are critical for induction of double-membrane vesicles associated with hepatitis C virus replication. MBio, 6(4), e00759. doi:10.1128/mBio.00759-15
Romero-Brey, I., Merz, A., Chiramel, A., Lee, J. Y., Chlanda, P., Haselman, U., Santarella-Mellwig, R., Habermann, A., Hoppe, S., Kallis, S., Walther, P., Antony, C., Krijnse-Locker, J., & Bartenschlager, R. (2012). Three-dimensional architecture and biogenesis of membrane structures associated with hepatitis C virus replication. PLoS Pathog, 8(12), e1003056. doi:10.1371/journal.ppat.1003056
Ross-Thriepland, D., & Harris, M. (2015). Hepatitis C virus NS5A: enigmatic but still promiscuous 10 years on. J Gen Virol, 96(Pt 4), 727-738. doi:10.1099/jgv.0.000009
Sanjana, N. E., Shalem, O., & Zhang, F. (2014). Improved vectors and genome-wide libraries for CRISPR screening. Nat Methods, 11(8), 783-784. doi:10.1038/nmeth.3047
Schaller, T., Appel, N., Koutsoudakis, G., Kallis, S., Lohmann, V., Pietschmann, T., & Bartenschlager, R. (2007). Analysis of hepatitis C virus superinfection exclusion by using novel fluorochrome gene-tagged viral genomes. J Virol, 81(9), 4591-4603. doi:10.1128/JVI.02144-06
Schaller, T., Ocwieja, K. E., Rasaiyaah, J., Price, A. J., Brady, T. L., Roth, S. L., Hué, S., Fletcher, A. J., Lee, K., KewalRamani, V. N., Noursadeghi, M., Jenner, R. G., James, L. C., Bushman, F. D., & Towers, G. J. (2011). HIV-1 capsid-cyclophilin interactions determine nuclear import pathway, integration targeting and replication efficiency. PLoS Pathog, 7(12), e1002439. doi:10.1371/journal.ppat.1002439
Schoggins, J. W., Wilson, S. J., Panis, M., Murphy, M. Y., Jones, C. T., Bieniasz, P., & Rice, C. M. (2011). A diverse range of gene products are effectors of the type I interferon antiviral response. Nature, 472(7344), 481-485. doi:10.1038/nature09907
Sumpter, R., Loo, Y. M., Foy, E., Li, K., Yoneyama, M., Fujita, T., Lemon, S. M., & Gale, M. (2005). Regulating intracellular antiviral defense and permissiveness to hepatitis C virus RNA replication through a cellular RNA helicase, RIG-I. J Virol, 79(5), 2689-2699. doi:10.1128/JVI.79.5.2689-2699.2005
Sun, S., Guo, M., Zhang, J. B., Ha, A., Yokoyama, K. K., & Chiu, R. H. (2014). Cyclophilin A (CypA) interacts with NF-κB subunit, p65/RelA, and contributes to NF-κB activation signaling. PLoS One, 9(8), e96211. doi:10.1371/journal .pone.0096211
Thali, M., Bukovsky, A., Kondo, E., Rosenwirth, B., Walsh, C. T., Sodroski, J., & Göttlinger, H. G. (1994). Functional association of cyclophilin A with HIV-1 virions. Nature, 372(6504), 363-365. doi:10.1038/372363a0
Thomis, D. C., & Samuel, C. E. (1992). Mechanism of interferon action: autoregulation of RNA-dependent P1/eIF-2 alpha protein kinase (PKR) expression in transfected mammalian cells. Proc Natl Acad Sci U S A, 89(22), 10837-10841. doi:10.1073/pnas.89.22.10837
Wang, P., & Heitman, J. (2005). The cyclophilins. Genome Biol, 6(7), 226. doi:10.1186/gb-2005-6-7-226
Wang, S., Wu, X., Pan, T., Song, W., Wang, Y., Zhang, F., & Yuan, Z. (2012). Viperin inhibits hepatitis C virus replication by interfering with binding of NS5A to host protein hVAP-33. J Gen Virol, 93(Pt 1), 83-92. doi:10.1099/vir.0.033860-0
Warne, J., Pryce, G., Hill, J. M., Shi, X., Lennerås, F., Puentes, F., Kip, M., Hilditch, L., Walker, P., Simone, M. I., Chan, A. W., Towers, G. J., Coker, A. R., Duchen, M. R., Szabadkai, G., Baker, D., & Selwood, D. L. (2016). Selective inhibition of the mitochondrial permeability transition pore protects against neurodegeneration in experimental multiple sclerosis. J Biol Chem, 291(9), 4356-4373. doi:10.1074/jbc.M115.700385
Watashi, K., Ishii, N., Hijikata, M., Inoue, D., Murata, T., Miyanari, Y., & Shimotohno, K. (2005). Cyclophilin B is a functional regulator of hepatitis C virus RNA polymerase. Mol Cell, 19(1), 111-122. doi:10.1016/j.molcel.2005.05.014
Williams, B. R. (1999). PKR; a sentinel kinase for cellular stress. Oncogene, 18(45), 6112-6120. doi:10.1038/sj.onc.1203127
Yamane, D., Feng, H., Rivera-Serrano, E. E., Selitsky, S. R., Hirai-Yuki, A., Das, A., McKnight, K. L., Misumi, I., Hensley, L., Lovell, W., González-López, O., Suzuki, R., Matsuda, M., Nakanishi, H., Ohto-Nakanishi, T., Hishiki, T., Wauthier, E., Oikawa, T., Morita, K., Reid, L. M., Sethupathy, P., Kohara, M., Whitmire, J. K., & Lemon, S. M. (2019). Basal expression of interferon regulatory factor 1 drives intrinsic hepatocyte resistance to multiple RNA viruses. Nat Microbiol. doi:10.1038/s41564-019-0425-6
Yang, F., Robotham, J. M., Grise, H., Frausto, S., Madan, V., Zayas, M., Bartenschlager, R., Robinson, M., Greenstein, A. E., Nag, A., Logan, T. M., Bienkiewicz, E., & Tang, H. (2010). A major determinant of cyclophilin dependence and cyclosporine susceptibility of hepatitis C virus identified by a genetic approach. PLoS Pathog, 6(9), e1001118. doi:10.1371/journal.ppat.1001118
Yang, F., Robotham, J. M., Nelson, H. B., Irsigler, A., Kenworthy, R., & Tang, H. (2008). Cyclophilin A is an essential cofactor for hepatitis C virus infection and the principal mediator of cyclosporine resistance in vitro. J Virol, 82(11), 5269-5278. doi:10.1128/JVI.02614-07
Ylinen, L. M., Schaller, T., Price, A., Fletcher, A. J., Noursadeghi, M., James, L. C., & Towers, G. J. (2009). Cyclophilin A levels dictate infection efficiency of human immunodeficiency virus type 1 capsid escape mutants A92E and G94D. J Virol, 83(4), 2044-2047. doi:10.1128/JVI.01876-08
Zenke, G., Strittmatter, U., Fuchs, S., Quesniaux, V. F., Brinkmann, V., Schuler, W., Zurini, M., Enz, A., Billich, A., Sanglier, J. J., & Fehr, T. (2001). Sanglifehrin A, a novel cyclophilin-binding compound showing immunosuppressive activity with a new mechanism of action. J Immunol, 166(12), 7165-7171. doi:10.4049/jimmunol.166.12.7165
Zufferey, R., Nagy, D., Mandel, R. J., Naldini, L., & Trono, D. (1997). Multiply attenuated lentiviral vector achieves efficient gene delivery in vivo. Nat Biotechnol, 15(9), 871-875. doi:10.1038/nbt0997-871

### Reference Example 2 - Antiviral activity of the cyclosporine analogues

CsA-PROTAC ("JW4-10") as used in this example is the same compound as CsA-Prtc1 of Example 1.

We have previously shown that naturally occurring cyclosporines CsA and CsH are inhibitors of the antiviral protein IFITM3. This protein is highly expressed in haematopoetic stem cells and blocks transduction of VSGg-pseudotyped lentiviral gene therapy vectors. We have previously shown that CsA and CsH inhibit IFITM3 to enhance HSC transduction and that CsH is more potent than CsA. To improve CsA potency and specificity, CsA-PROTAC was generated by coupling CsA to a ligand for the von Hippel-Lindau (VHL) E3 ligase.

To determine its potency against IFITM3, we established a cell culture model for IFITM3 restriction of VSV-G pseudotyped HIV vectors encoding GFP. To do so, we transduced the THP1 monocytic cell line with a lentiviral vector encoding IFITM3 and a puromycin resistance gene. 48 hours post transduction we treated cells with puromycin to select for cells expressing IFITM3 at high levels (THP1-IFITM3). To determine the potency of CsA-PROTAC compared to CsA and CsH, THP1-IFITM3 cells were treated with each drug over a range of concentrations (0.35 - 2.5 uM). Drugs were added at the time of transduction with a VSVg-pseudotyped HIV-1 vector encoding a GFP reporter gene. Cells were incubated with the vector and drug combinations for 48 hours, at which point cells were fixed to determine the percentage of infected cells, measured as the percentage of GFP-positive cells by flow cytometry. The results are shown in Figure 26. In the absence of the drugs, IFITM3 strongly restricts the vector, evidenced by very low levels of transduction in the no drug control (0 uM).

Figure 26 shows that, in the absence of the drugs, IFITM3 strongly restricts the vector, evidenced by very low levels of transduction in the no drug control (0 uM). Figure 26 further shows that, in cell lines that make IFITM3, the compound CsA-PROTAC enhanced transduction more potently than CsH (and CsA). In particular, THP1 monocytic cell lines expressing IFITM3 from a lentiviral vector have restricted transduction by HIV vectors encoding GFP, as seen in no drug controls (0uM). CsA-PROTAC treatment (at concentrations shown) enhanced transduction of an HIV vector encoding GFP more potently than CsA, and more potently than the current "best molecule" CsH. CsA-PROTAC was more potent at lower concentrations.

### Reference Example 3 - Further study on antiviral activity of the cyclosporine analogues

We have applied PROTAC to CsA to act as a bridge linking CypA to the proteasomal degradation pathway of the following general formula:

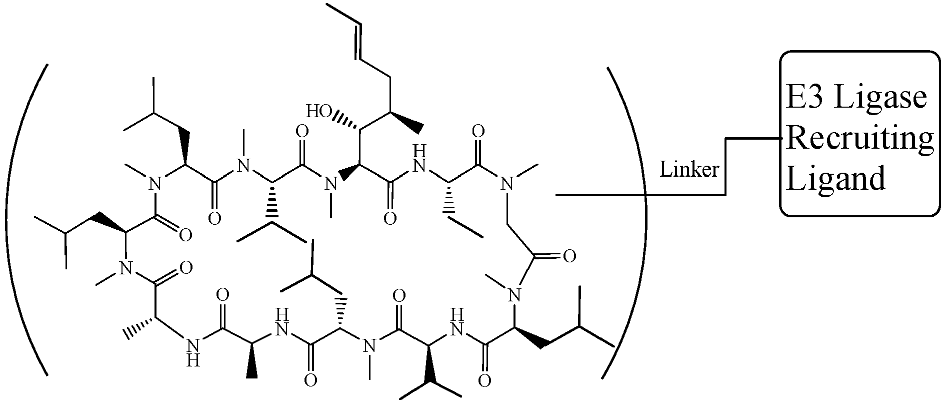

It was hoped to inhibit infection via the degradation of CypA as opposed to simply blocking this CA/cofactor interaction. The analogues JW4-10 and JW4-20 have increased potency to CsA, while JW4-21 has decreased potency (Figure 27A and Table below). Also used as a control in the experiments (see Figure 27A) was SmBz ([Sarcosine-3(4-methylbenzoate)]-CsA). SmBz is disclosed in Malouitre et al. Biochem J. 2009 Dec 14;425(1):137-48. The anti HIV activity of SmBz is described in Rasaiyaah et al. Nature, 2013. 503(7476): p. 402-5.

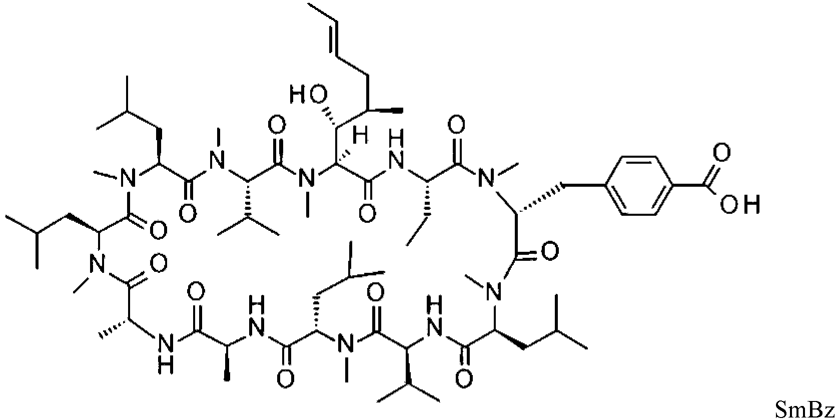

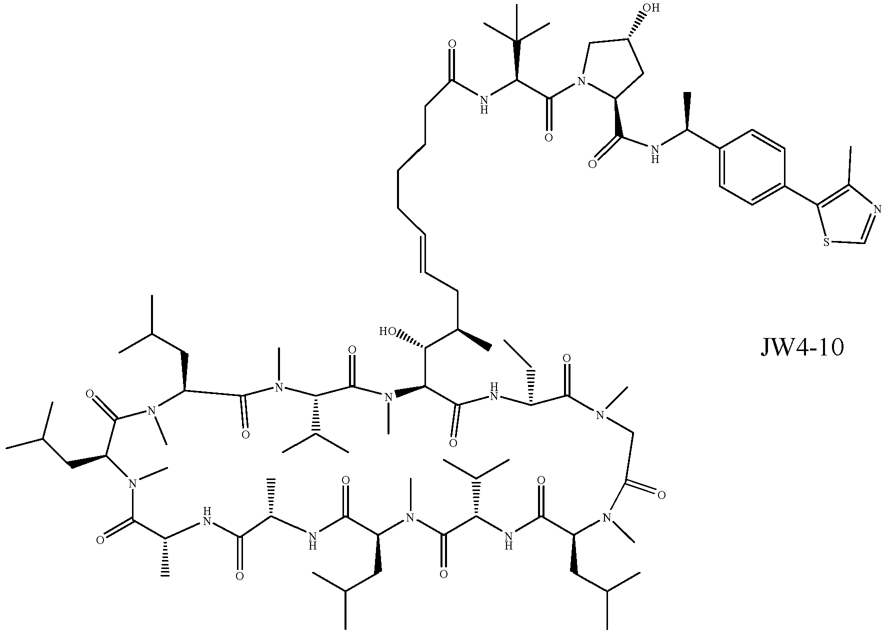

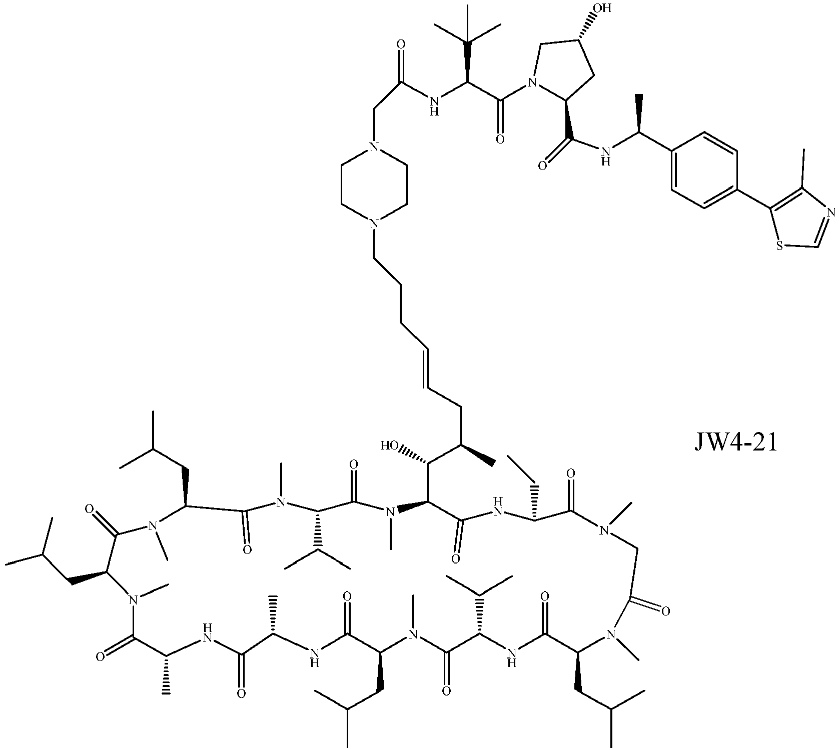

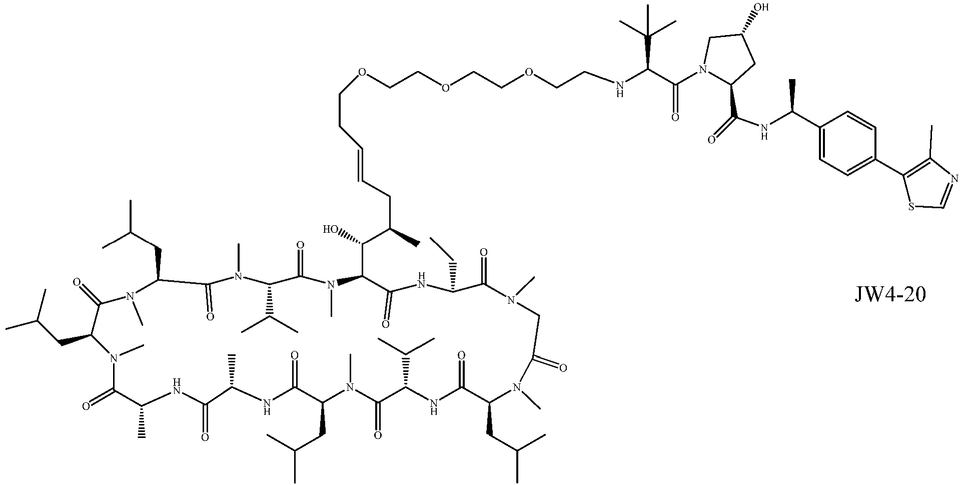

| **CypI** | **IC50 µm** |
|---|---|
| CsA | 0.32 ± 0.06 |
| JW4-10 | 0.09 ± 0.13 |
| JW4-20 | 0.12 ± 0.05 |
| JW4-21 | 0.70 ± 0.08 |

Samples collected 48hrs after PROTAC treatment were subjected to western blotting (Figure 27B), confirming that JW4-10 resulted in the degradation of CypA.

## Claims

1. A cyclosporine analogue that is a compound of formula (III) or a pharmaceutical salt thereof 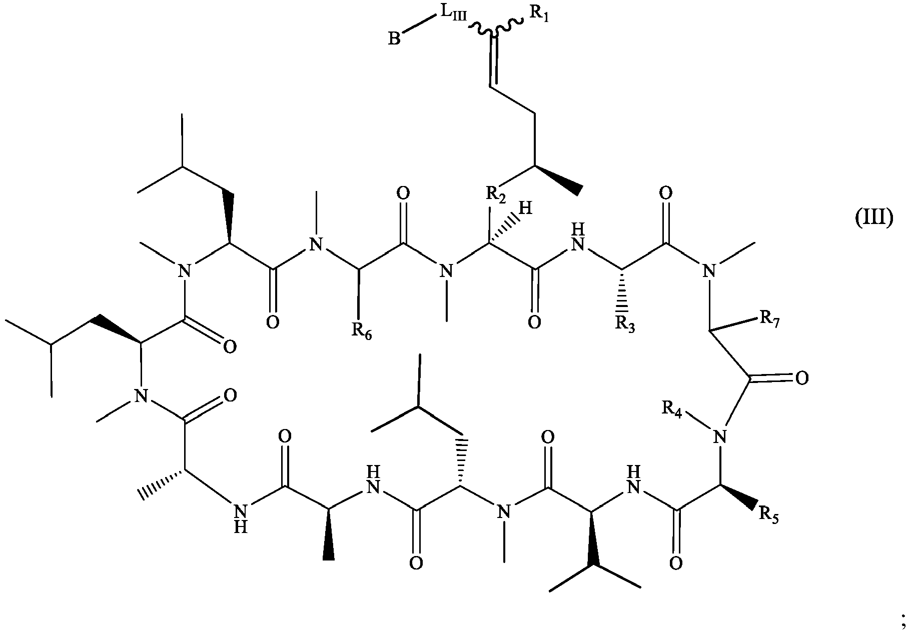 wherein:
L_{III} is a linker moiety or a direct bond;
B is 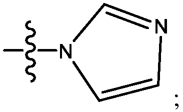
R₁ represents hydrogen;
R₂ represents 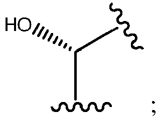
R₃ represents ethyl;
R₄ represents methyl;
R₅ represents -CH₂CH(CH₃)₂;
R₆ represents 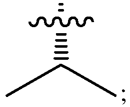
R₇ represents a hydrogen atom; and
the linker moiety is a C₁₋₂₀ alkylene group, a C₂₋₂₀ alkenylene group or a C₂₋₂₀ alkynylene group, which is unsubstituted or substituted by one or more substituents selected from halogen atoms and sulfonic acid groups, and in which
(a) 0, 1, 2 or 3 carbon atoms are replaced by groups selected from C₆₋₁₀ arylene, 5- to 10-membered heteroarylene, C₃₋₇ carbocyclylene and 5- to 10-membered heterocyclylene groups, and
(b) up to half of the -CH₂- groups are replaced by groups selected from -O-, -S-, -C(O)- and -N(C₁₋₆ alkyl)- groups, wherein:
(i) said arylene, heteroarylene, carbocyclylene and heterocyclylene groups are unsubstituted or substituted by one or more substituents selected from: halogen atoms; and C₁₋₆ alkyl; C₁₋₆ haloalkyl; C₁₋₆ alkoxy; -(C₁₋₆ alkyl)ₙC(O)O(C₁₋₆ alkyl) where n=0 or 1; -(C₁₋₆ alkyl)ₙOC(O)(C₁₋₆ alkyl) where n=0 or 1; C₁₋₆ alkylthiol, - N(R_{N})₂ wherein each R_{N} independently represents a hydrogen atom or a C₁₋₆ alkyl group; -CN; -S(O)₂NH₂; nitro; and sulfonic acid groups; and
(ii) 0, 1 or 2 carbon atoms in said carbocyclylene and heterocyclylene groups are replaced by -C(O)- groups.

2. The cyclosporine conjugate according to claim 1, wherein L_{III} is a linker moiety that is a C₁₋₂₀ alkylene group, a C₂₋₂₀ alkenylene group or a C₂₋₂₀ alkynylene group, which is unsubstituted or substituted by one or more substituents selected from halogen atoms and sulfonic acid groups, and in which
(a) 0, 1, 2 or 3 carbon atoms are replaced by groups selected from C₆₋₁₀ arylene, 5- to 10-membered heteroarylene, C₃₋₇ carbocyclylene and 5- to 10-membered heterocyclylene groups, and
(b) up to half of the -CH₂- groups are replaced by groups selected from -O-, -S-, -C(O)- and -N(C₁₋₆ alkyl)- groups, wherein:
(i) said arylene, heteroarylene, carbocyclylene and heterocyclylene groups are unsubstituted or substituted by one or more substituents selected from: halogen atoms; and C₁₋₆ alkyl; C₁₋₆ haloalkyl; C₁₋₆ alkoxy; -(C₁₋₆ alkyl)ₙC(O)O(C₁₋₆ alkyl) where n=0 or 1; -(C₁₋₆ alkyl)ₙOC(O)(C₁₋₆ alkyl) where n=0 or 1; C₁₋₆ alkylthiol, - N(R_{N})₂ wherein each R_{N} independently represents a hydrogen atom or a C₁₋₆ alkyl group; -CN; -S(O)₂NH₂; nitro; and sulfonic acid groups; and
(ii) 0, 1 or 2 carbon atoms in said carbocyclylene and heterocyclylene groups are replaced by -C(O)- groups.

3. The cyclosporine conjugate according to claim 1 or 2, wherein L_{III} is a linker moiety that is a C₁₋₁₅ alkylene group, a C₂₋₁₅ alkenylene group or a C₂₋₁₅ alkynylene group, and in which
(a) 0, 1 or 2 carbon atoms are replaced by groups selected from C₆₋₁₀ arylene, 5- to 10-membered heteroarylene, C₃₋₇ carbocyclylene and 5- to 10-membered heterocyclylene groups, and
(b) up to half of the -CH₂- groups are replaced by groups selected from -O- and -C(O)-, wherein:
(i) said arylene, heteroarylene, carbocyclylene and heterocyclylene groups are unsubstituted or substituted by one or more substituents selected from: halogen atoms; and C₁₋₆ alkyl; C₁₋₆ haloalkyl; C₁₋₆ alkoxy; -(C₁₋₆ alkyl)ₙC(O)O(C₁₋₆ alkyl) where n=0 or 1; -(C₁₋₆ alkyl)ₙOC(O)(C₁₋₆ alkyl) where n=0 or 1; C₁₋₆ alkylthiol, - N(R_{N})₂ wherein each R_{N} independently represents a hydrogen atom or a C₁₋₆ alkyl group; -CN; -S(O)₂NH₂; nitro; and sulfonic acid groups; and
(ii) 0, 1 or 2 carbon atoms in said carbocyclylene and heterocyclylene groups are replaced by -C(O)- groups.

4. Use ex vivo of the cyclosporine analogue according to any one of claims 1 to 3, for increasing the efficiency of transduction of an isolated population of human haematopoietic stem and/or progenitor cells by a vector derived from HIV-1, HIV-2, SIV, FIV, BIV, EIAV, CAEV or visna lentivirus.

5. Use according to claim 4, wherein the percentage of haematopoietic stem and/or progenitor cells transduced by the vector is increased and/or the vector copy number per cell is increased.

6. A method of transducing a population of human haematopoietic stem and/or progenitor cells comprising the steps of:
a) contacting the population of cells with the cyclosporine analogue according to any one of claims 1 to 3; and
b) transducing the population of cells with a vector derived from HIV-1, HIV- 2, FIV, BIV, EIAV, CAEV or visna lentivirus;
wherein steps (a) and (b) are carried out ex vivo.

7. The method according to claim 6, the method having at least one of the following further features (i) to (iii):
(i) the percentage of haematopoietic stem and/or progenitor cells transduced by the vector is increased and/or the vector copy number per cell is increased;
(ii) the population of haematopoietic stem and/or progenitor cells is or has been obtained from mobilised peripheral blood, bone marrow or umbilical cord blood; and
(iii) the method includes a further step of enriching the population for haematopoietic stem and/or progenitor cells.

8. A population of human haematopoietic stem and/or progenitor cells prepared according to the method of claim 6 or 7.

9. A pharmaceutical composition comprising the population of haematopoietic stem and/or progenitor cells according to claim 8.

10. The population of haematopoietic stem and/or progenitor cells according to claim 8 for use in therapy.

11. The population for use according to claim 10, wherein the population is administered as part of an autologous stem cell transplant procedure or an allogeneic stem cell transplant procedure.

12. The cyclosporine analogue according to any one of claims 1 to 3, for use in haematopoietic stem and/or progenitor cell gene therapy.

13. The cyclosporine analogue according to any one of claims 1 to 3, for use in treatment of a viral infection.

14. The cyclosporine analogue for use according to claim 13, wherein the viral infection is human immunodeficiency virus-1 (HIV-1), influenza virus, human cytomegalovirus (hCMV), hepatitis C virus (HCV), dengue virus, a vaccinia virus, feline immunodeficiency virus (FIV) or a corona virus.

15. The cyclosporine analogue for use according to claim 14, wherein the viral infection is COVID-19.

## Patentansprüche

1. Cyclosporin-Analogon, das eine Verbindung der Formel (III) oder ein pharmazeutisches Salz davon ist 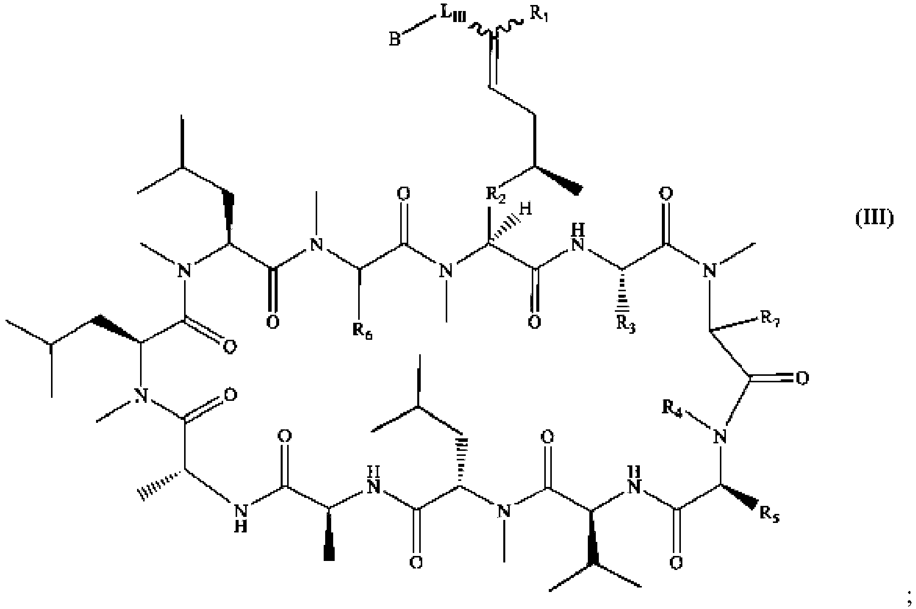 wobei:
L_{III} eine Einheit oder eine direkte Bindung ist;
B 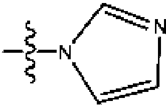 ist;
R₁ Wasserstoff darstellt;
R₂ 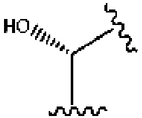 darstellt;
R₃ Ethyl darstellt;
R₄ Methyl darstellt;
R₅ -CH₂CH(CH₃)₂ darstellt;
R₆ 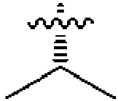 darstellt;
R₇ ein Wasserstoffatom darstellt; und
die Linker-Einheit eine C₁₋₂₀-Alkylengruppe, eine C₂₋₂₀-Alkenylengruppe oder eine C₂₋₂₀-Alkinylengruppe ist, die unsubstituiert ist oder durch einen oder mehrere Substituenten substituiert ist, die aus Halogenatomen und Sulfonsäuregruppen ausgewählt sind, und bei der
(a) 0, 1, 2 oder 3 Kohlenstoffatome durch Gruppen ersetzt sind, die aus C₆₋₁₀-Arylen-, 5- bis 10-gliedrigen Heteroarylen-, C₃₋₇-Carbocyclylen- und 5- bis 10-gliedrigen Heterocyclylen-Gruppen ausgewählt sind, und
(b) bis zur Hälfte der -CH₂-Gruppen durch Gruppen ersetzt sind, die aus -O-, -S-, - C(O)- und -N(C₁₋₆-Alkyl)-Gruppen ausgewählt sind, wobei:
(i) die Arylen-, Heteroarylen-, Carbocyclylen- und Heterocyclylen-Gruppen unsubstituiert sind oder durch einen oder mehrere Substituenten substituiert sind, ausgewählt aus: Halogenatomen; und C₁₋₆-Alkyl; C₁₋₆-Halogenalkyl; C₁₋₆-Alkoxy; -(C₁₋₆-Alkyl)ₙC(O)O(C₁₋₆-Alkyl), wobei n = 0 oder 1; -(C₁₋₆-Alkyl)ₙOC(O)(C₁₋₆-Alkyl), wobei n = 0 oder 1; C₁₋₆-Alkylthiol, -N(RN)₂ wobei jedes R_{N} unabhängig voneinander ein Wasserstoffatom oder eine C₁₋₆-Alkylgruppe; -CN; -S(O)₂NH₂; Nitro; und Sulfonsäuregruppen darstellt; und
(ii) 0, 1 oder 2 Kohlenstoffatome in den Carbocyclylen- und Heterocyclylengruppen durch -C(O)-Gruppen ersetzt sind.

2. Cyclosporin-Konjugat nach Anspruch 1, wobei L_{III} eine Linker-Einheit ist, die eine C₁₋₂₀-Alkylengruppe, eine C₂₋₂₀-Alkenylengruppe oder eine C₂₋₂₀-Alkinylengruppe ist, die unsubstituiert ist oder durch einen oder mehrere Substituenten substituiert ist, die aus Halogenatomen und Sulfonsäuregruppen ausgewählt sind, und bei der
(a) 0, 1, 2 oder 3 Kohlenstoffatome durch Gruppen ersetzt sind, die aus C₆₋₁₀-Arylen-, 5- bis 10-gliedrigen Heteroarylen-, C₃₋₇-Carbocyclylen- und 5- bis 10-gliedrigen Heterocyclylen-Gruppen ausgewählt sind, und
(b) bis zur Hälfte der -CH₂-Gruppen durch Gruppen ersetzt sind, die aus -O-, -S-, - C(O)- und -N(C₁₋₆-Alkyl)-Gruppen ausgewählt sind, wobei:
(i) die Arylen-, Heteroarylen-, Carbocyclylen- und Heterocyclylen-Gruppen unsubstituiert sind oder durch einen oder mehrere Substituenten substituiert sind, ausgewählt aus: Halogenatomen; und C₁₋₆-Alkyl; C₁₋₆-Halogenalkyl; C₁₋₆-Alkoxy; -(C₁₋₆-Alkyl)ₙC(O)O(C₁₋₆-Alkyl), wobei n = 0 oder 1; -(C₁₋₆-Alkyl)ₙOC(O)(C₁₋₆-Alkyl), wobei n = 0 oder 1; C₁₋₆-Alkylthiol; -N(RN)₂ wobei jedes R_{N} unabhängig voneinander ein Wasserstoffatom oder eine C₁₋₆-Alkylgruppe; -CN; -S(O)₂NH₂; Nitro; und Sulfonsäuregruppen darstellt; und
(ii) 0, 1 oder 2 Kohlenstoffatome in den Carbocyclylen- und Heterocyclylengruppen durch -C(O)-Gruppen ersetzt sind.

3. Cyclosporin-Konjugat nach Anspruch 1 oder 2, wobei L_{III} eine Linker-Einheit ist, die eine C₁₋₁₅-Alkylengruppe, eine C₂₋₁₅-Alkenylengruppe oder eine C₂₋₁₅-Alkinylengruppe ist, und bei der
(a) 0, 1 oder 2 Kohlenstoffatome durch Gruppen ersetzt sind, die aus C₆₋₁₀-Arylen-, 5-bis 10-gliedrigen Heteroarylen-, C₃₋₇-Carbocyclylen- und 5- bis 10-gliedrigen Heterocyclylen-Gruppen ausgewählt sind, und
(b) bis zur Hälfte der -CH₂-Gruppen durch Gruppen ersetzt sind, die aus -O- und - C(O)- ausgewählt sind, wobei:
(i) die Arylen-, Heteroarylen-, Carbocyclylen- und Heterocyclylen-Gruppen unsubstituiert sind oder durch einen oder mehrere Substituenten substituiert sind, ausgewählt aus: Halogenatomen; und C₁₋₆-Alkyl; C₁₋₆-Halogenalkyl; C₁₋₆-Alkoxy; -(C₁₋₆-Alkyl)ₙC(O)O(C₁₋₆-Alkyl), wobei n = 0 oder 1; -(C₁₋₆-Alkyl)ₙOC(O)(C₁₋₆-Alkyl), wobei n = 0 oder 1; C₁₋₆-Alkylthiol; -N(RN)₂ wobei jedes R_{N} unabhängig voneinander ein Wasserstoffatom oder eine C₁₋₆-Alkylgruppe; -CN; -S(O)₂NH₂; Nitro; und Sulfonsäuregruppen darstellt; und
(ii) 0, 1 oder 2 Kohlenstoffatome in den Carbocyclylen- und Heterocyclylengruppen durch -C(O)-Gruppen ersetzt sind.

4. Ex-vivo-Verwendung des Cyclosporin-Analogons nach Anspruch 1 bis 3 zur Steigerung der Transduktionseffizienz einer isolierten Population menschlicher hämatopoetischer Stamm- und/oder Vorläuferzellen durch einen Vektor, der von HIV-1, HIV-2, SIV, FIV, BIV, EIAV, CAEV oder dem Visna-Lentivirus abgeleitet ist.

5. Verwendung nach Anspruch 4, wobei der prozentuale Anteil der durch den Vektor transduzierten hämatopoetischen Stamm- und/oder Vorläuferzellen erhöht und/oder die Vektorkopienzahl pro Zelle erhöht wird.

6. Verfahren zur Transduktion einer Population menschlicher hämatopoetischer Stamm- und/oder Vorläuferzellen, das die folgenden Schritte umfasst:
a) Inkontaktbringen der Zellpopulation mit dem Cyclosporin-Analogon nach einem der Ansprüche 1 bis 3; und
b) Transduzieren der Zellpopulation mit einem Vektor, der von HIV-1, HIV-2, FIV, BIV, EIAV, CAEV oder dem Visna-Lentivirus abgeleitet ist;
wobei die Schritte (a) und (b) ex vivo durchgeführt werden.

7. Verfahren nach Anspruch 6, wobei das Verfahren mindestens eines der folgenden weiteren Merkmale (i) bis (iii) aufweist:
(i) der prozentuale Anteil der durch den Vektor transduzierten hämatopoetischen Stamm- und/oder Vorläuferzellen wird erhöht und/oder die Vektorkopienzahl pro Zelle wird erhöht;
(ii) die Population hämatopoetischer Stamm- und/oder Vorläuferzellen wird oder wurde aus mobilisiertem peripherem Blut, Knochenmark oder Nabelschnurblut gewonnen; und
(iii) das Verfahren enthält einen weiteren Schritt zur Anreicherung der Population an hämatopoetischen Stamm- und/oder Vorläuferzellen.

8. Population menschlicher hämatopoetischer Stamm- und/oder Vorläuferzellen, die gemäß dem Verfahren nach Anspruch 6 oder 7 hergestellt sind.

9. Arzneimittelzusammensetzung, die die Population hämatopoetischer Stamm- und/oder Vorläuferzellen nach Anspruch 8 umfasst.

10. Population hämatopoetischer Stamm- und/oder Vorläuferzellen nach Anspruch 8 zur Verwendung in der Therapie.

11. Population zur Verwendung nach Anspruch 10, wobei die Population im Rahmen eines autologen Stammzelltransplantationsverfahrens oder eines allogenen Stammzelltransplantationsverfahrens verabreicht wird.

12. Cyclosporin-Analogon nach Anspruch 1 bis 3 zur Verwendung in der Gentherapie mit hämatopoetischen Stamm- und/oder Vorläuferzellen.

13. Cyclosporin-Analogon nach Anspruch 1 bis 3 zur Verwendung bei der Behandlung einer Virusinfektion.

14. Cyclosporin-Analogon zur Verwendung nach Anspruch 13, wobei die Virusinfektion das humane Immundefizienz-Virus-1 (HIV-1), das Influenzavirus, das humane Cytomegalievirus (hCMV), das Hepatitis-C-Virus (HCV), das Dengue-Virus, ein Vacciniavirus, das feline Immundefizienz-Virus (FIV) oder ein Coronavirus ist.

15. Cyclosporin-Analogon zur Verwendung nach Anspruch 14, wobei die Virusinfektion COVID-19 ist.

## Revendications

1. Analogue de cyclosporine qui est un composé de formule (III) ou sel pharmaceutiquement acceptable de celui-ci 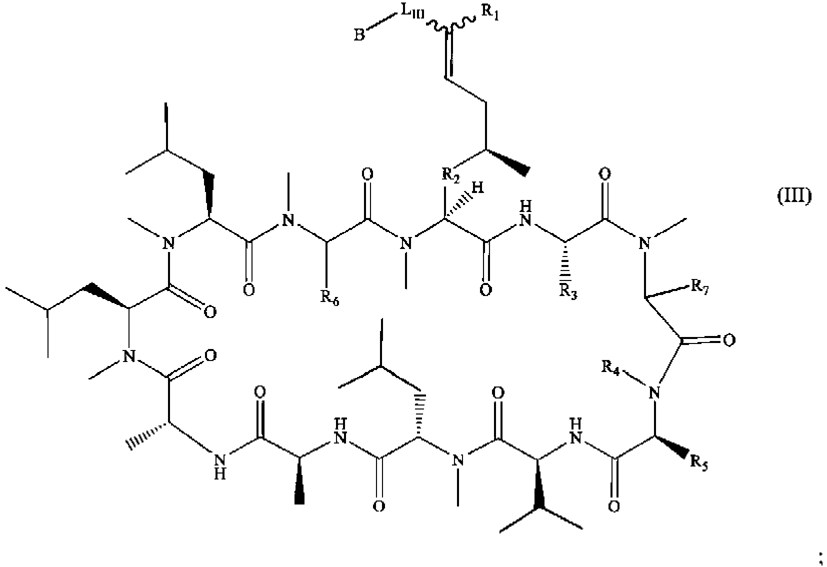 où :
L_{III} est une fraction de lieur ou une liaison directe ;
B est 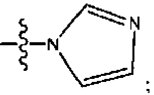
R₁ représente hydrogène ;
R₂ représente 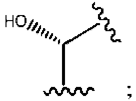
R₃ représente éthyle ;
R₄ représente méthyle ;
R₅ représente -CH₂CH(CH₃)₂ ;
R₆ représente 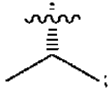
R₇ représente un atome d'hydrogène ; et
la fraction de lieur est un groupe alkylène en C₁₋₂₀, un groupe alcénylène en C₂₋₂₀ ou un groupe alcynylène en C₂₋₂₀, qui est non substitué ou substitué par un ou plusieurs substituants sélectionnés parmi des atomes d'halogène et des groupes acide sulfonique, et dans lequel
(a) 0, 1, 2 ou 3 atomes de carbone sont remplacés par des groupes sélectionnés parmi des groupes arylène en C₆₋₁₀, hétéroarylène à 5 à 10 chaînons, carbocyclylène en C₃₋₇ et hétérocyclylène à 5 à 10 chaînons, et
(b) jusqu'à la moitié des groupes -CH₂- sont remplacés par des groupes sélectionnés parmi -O-, -S-, -C(O)- et des groupes -N(alkyle en C₁₋₆)-, où :
(i) lesdits groupes arylène, hétéroarylène, carbocyclylène et hétérocyclylène sont non substitués ou substitués par un ou plusieurs substituants sélectionnés parmi : des atomes d'halogène ; et alkyle en C₁₋₆ ; halogénoalkyle en C₁₋₆ ; alcoxy en C₁₋₆ ; - (alkyl en C₁₋₆)ₙC(O)O(alkyle en C₁₋₆) où n=0 ou 1 ; -(alkyl en C₁₋₆)ₙOC(O)(alkyle en C₁₋₆) où n=0 ou 1 ; alkylthiol en C₁₋₆, -N(R_{N})₂ où chaque R_{N} représente indépendamment un atome d'hydrogène ou un groupe alkyle en C₁₋₆ ; -CN ; - S(O)₂NH₂ ; nitro ; et des groupes acide sulfonique ; et
(ii) 0, 1 ou 2 atomes de carbone dans lesdits groupes carbocyclylène et hétérocyclylène sont remplacés par des groupes -C(O)-.

2. Conjugué de cyclosporine selon la revendication 1, dans lequel L_{III} est une fraction de lieur qui est un groupe alkylène en C₁₋₂₀, un groupe alcénylène en C₂₋₂₀ ou un groupe alcynylène en C₂₋₂₀, qui est non substitué ou substitué par un ou plusieurs substituants sélectionnés parmi des atomes d'halogène et des groupes acide sulfonique, et dans lequel
(a) 0, 1, 2 ou 3 atomes de carbone sont remplacés par des groupes sélectionnés parmi des groupes arylène en C₆₋₁₀, hétéroarylène à 5 à 10 chaînons, carbocyclylène en C_{3- 7} et hétérocyclylène à 5 à 10 chaînons, et
(b) jusqu'à la moitié des groupes -CH₂- sont remplacés par des groupes sélectionnés parmi -O-, -S-, -C(O)- et des groupes -N(alkyle en C₁₋₆)-, où :
(i) lesdits groupes arylène, hétéroarylène, carbocyclylène et hétérocyclylène sont non substitués ou substitués par un ou plusieurs substituants sélectionnés parmi : des atomes d'halogène ; et alkyle en C₁₋₆ ; halogénoalkyle en C₁₋₆ ; alcoxy en C₁₋₆ ; - (alkyl en C₁₋₆)ₙC(O)O(alkyle en C₁₋₆) où n=0 ou 1 ; -(alkyl en C₁₋₆)ₙOC(O)(alkyle en C₁₋₆) où n=0 ou 1 ; alkylthiol en C₁₋₆, -N(R_{N})₂ où chaque R_{N} représente indépendamment un atome d'hydrogène ou un groupe alkyle en C₁₋₆ ; -CN ; - S(O)₂NH₂ ; nitro ; et des groupes acide sulfonique ; et
(ii) 0, 1 ou 2 atomes de carbone dans lesdits groupes carbocyclylène et hétérocyclylène sont remplacés par des groupes -C(O)-.

3. Conjugué de cyclosporine selon la revendication 1 ou 2, dans lequel L_{III} est une fraction de lieur qui est un groupe alkylène en C₁₋₁₅, un groupe alcénylène en C₂₋₁₅ ou un groupe alcynylène en C₂₋₁₅, et dans lequel
(a) 0, 1 ou 2 atomes de carbone sont remplacés par des groupes sélectionnés parmi des groupes arylène en C₆₋₁₀, hétéroarylène à 5 à 10 chaînons, carbocyclylène en C₃₋₇ et hétérocyclylène à 5 à 10 chaînons, et
(b) jusqu'à la moitié des groupes -CH₂- sont remplacés par des groupes sélectionnés parmi -O- et -C(O)-, où :
(i) lesdits groupes arylène, hétéroarylène, carbocyclylène et hétérocyclylène sont non substitués ou substitués par un ou plusieurs substituants sélectionnés parmi : des atomes d'halogène ; et alkyle en C₁₋₆ ; halogénoalkyle en C₁₋₆ ; alcoxy en C₁₋₆ ; - (alkyl en C₁₋₆)ₙC(O)O(alkyle en C₁₋₆) où n=0 ou 1 ; -(alkyl en C₁₋₆)ₙOC(O)(alkyle en C₁₋₆) où n=0 ou 1 ; alkylthiol en C₁₋₆, -N(R_{N})₂ où chaque R_{N} représente indépendamment un atome d'hydrogène ou un groupe alkyle en C₁₋₆ ; -CN ; - S(O)₂NH₂ ; nitro ; et des groupes acide sulfonique ; et
(ii) 0, 1 ou 2 atomes de carbone dans lesdits groupes carbocyclylène et hétérocyclylène sont remplacés par des groupes -C(O)-.

4. Utilisation ex vivo de l'analogue de cyclosporine selon l'une quelconque des revendications 1 à 3, pour augmenter l'efficacité de transduction d'une population isolée de cellules souches et/ou progénitrices hématopoïétiques humaines par un vecteur dérivé de VIH-1, VIH-2, VIS, VIF, VIB, VAIE, VAEC ou lentivirus visna.

5. Utilisation selon la revendication 4, dans laquelle le pourcentage de cellules souches et/ou progénitrices hématopoïétiques traduites par le vecteur est augmenté et/ou le nombre de copies de vecteur par cellule est augmenté.

6. Procédé de transduction d'une population de cellules souches et/ou progénitrices hématopoïétiques humaines comprenant les étapes consistant à :
a) mettre en contact la population de cellules avec l'analogue de cyclosporine selon l'une quelconque des revendications 1 à 3 ; et
b) transduire la population de cellules avec un vecteur dérivé de VIH-1, VIH-2, VIS, VIF, VIB, VAIE, VAEC ou lentivirus visna ;
dans lequel les étapes (a) et (b) sont effectuées ex vivo.

7. Procédé selon la revendication 6, le procédé ayant au moins l'une des autres caractéristiques (i) à (iii) suivantes :
(i) le pourcentage de cellules souches et/ou progénitrices hématopoïétiques transduites par le vecteur est augmenté et/ou le nombre de copies de vecteur par cellule est augmenté ;
(ii) la population de cellules souches et/ou progénitrices hématopoïétiques est ou a été obtenue à partir de sang périphérique mobilisé, de moelle osseuse mobilisée, ou de sang du cordon ombilical mobilisé ; et
(iii) le procédé comporte une autre étape consistant à enrichir la population de cellules souches et/ou progénitrices hématopoïétiques.

8. Population de cellules souches et/ou progénitrices hématopoïétiques humaines préparée selon le procédé de la revendication 6 ou 7.

9. Composition pharmaceutique comprenant la population de cellules souches et/ou progénitrices hématopoïétiques selon la revendication 8.

10. Population de cellules souches et/ou progénitrices hématopoïétiques selon la revendication 8 pour une utilisation en thérapie.

11. Population pour une utilisation selon la revendication 10, dans laquelle la population est administrée dans le cadre d'une procédure d'autogreffe de cellules souches ou d'une procédure d'allogreffe de cellules souches.

12. Analogue de cyclosporine selon l'une quelconque des revendications 1 à 3, pour une utilisation dans une thérapie génique à cellules souches et/ou progénitrices hématopoïétiques.

13. Analogue de cyclosporine selon l'une quelconque des revendications 1 à 3, pour une utilisation dans le traitement d'une infection virale.

14. Analogue de cyclosporine pour une utilisation selon la revendication 13, dans laquelle l'infection virale est le virus de l'immunodéficience humaine 1 (VIH-1), le virus influenza, le cytomégalovirus humain (CMVh), le virus de l'hépatite C (VHC), le virus de la dengue, un virus de la vaccine, le virus de l'immunodéficience féline (VIF) ou un coronavirus.

15. Analogue de cyclosporine pour une utilisation selon la revendication 14, dans laquelle l'infection virale est la COVID-19.
